# EUROPEAN PATENT SPECIFICATION

(11) **EP 4 400 595 B1**
(45) Date of publication and mention of the grant of the patent: **04.11.2026**
(21) Application number: 24178773.8
(22) Date of filing: 27.05.2021
(51) Int. Cl.: C07C 67/60, C07C 67/08, C07C 69/80, C12P 7/62

(54) **PROCESS FOR PRODUCING HIGH-GRADE FATTY ACID POLYOL ESTERS, PARTICULARLY FATTY ACID GLYCEROL ESTERS**
VERFAHREN ZUR HERSTELLUNG VON HOCHWERTIGEN FETTSÄUREPOLYOLESTERN, INSBESONDERE FETTSÄUREGLYCEROLESTERN
PROCÉDÉ DE PRODUCTION D'ESTERS DE POLYOLS D'ACIDES GRAS DE QUALITÉ ÉLEVÉE, EN PARTICULIER D'ESTERS DE GLYCÉROL D'ACIDES GRAS

(30) Priority: 21.05.2021 WO PCT/EP2021/063582
(43) Date of publication of application: 17.07.2024
(62) Document number(s) of the earlier application(s) in accordance with Art. 76 EPC: 21728905.7 / 4 313 931
(73) Proprietor: IOI Oleo GmbH, 58453 Witten (DE)
(72) Inventor: LOCHMANN, Dirk, 58453 Witten (DE); REYER, Sebastian, 58453 Witten (DE); STEHR, Michael, 58453 Witten (DE)
(74) Representative: Von Rohr Patentanwälte Partnerschaft mbB

(56) References cited:
- US-A1- 2007 148 746
- FABIANA ANDREIA SCHÄFER DE MARTINI SOARES ET AL: "Chemical interesterification of blends of palm stearin, coconut oil, and canola oil: physicochemical properties", JOURNAL OF AGRICULTURAL AND FOOD CHEMISTRY, AMERICAN CHEMICAL SOCIETY, US, vol. 60, no. 6, 15 February 2012 (2012-02-15), pages 1461 - 1469, XP002716822, ISSN: 0021-8561, [retrieved on 20120109], DOI: 10.1021/JF204111T
- SELMI B. ET AL: "Lipase-catalyzed synthesis of tricaprylin in a medium solely composed of substrates. Water production and elimination", ENZYME AND MICROBIAL TECHNOLOGY, vol. 20, no. 5, 1 April 1997 (1997-04-01), US, pages 322 - 325, XP055886034, ISSN: 0141-0229, DOI: 10.1016/S0141-0229(96)00132-9
- DA ROCHA ATAIDE TEREZINHA ET AL: "Chemical synthesis of tricaproin, trienantin and tricaprylin : Synthesis of medium-chain triacylglycerols", INTERNATIONAL JOURNAL OF FOOD SCIENCE AND TECHNOLOGY, vol. 42, no. 12, 18 June 2007 (2007-06-18), GB, pages 1504 - 1508, XP055787620, ISSN: 0950-5423, DOI: 10.1111/j.1365-2621.2006.01376.x

## Description

The present invention relates to the technical field of fatty acid polyol esters, particularly fatty acid glycerol esters, and related metabolisms and medical therapy concepts of related diseases.

Especially, the present invention relates to a process for producing fatty acid glycerol esters (i.e. glycerides of fatty acids), especially with a high purity degree.

In the human energy metabolism, glucose is the short-term available energy carrier, which is metabolized into energy in the mitochondria by releasing water and carbon dioxide. The glycogen stores of the liver are already emptied during the sleep period during the night. However, especially the human central nervous system (CNS) and the heart require a permanent energy supply.

The physiological alternative to glucose, which is mainly available to the central nervous system, are the so-called ketone bodies (synonymously also called keto bodies).

The term ketone body is especially a collective term for three compounds, which are formed mainly in catabolic metabolic states (such as hunger, reduction diets or low-carbohydrate diets) and may lead to ketosis. The term ketone bodies includes especially the three compounds acetoacetate (synonymously also referred to as acetacetate) and acetone as well as 3-hydroxybutyric acid (hereinafter also synonymously referred to as beta-hydroxybutyric acid or BHB or 3-BHB) or its salt (i.e. 3-hydroxybutyrate or beta-hydroxybutyrate), the latter being the most important of the three aforementioned compounds. 3-Hydroxybutyric acid or its salt occurs physiologically as the (R)-enantiomer, i.e. as (R)-3-hydroxybutyric acid (synonymously also called (3R)-3-hydroxybutyric acid to emphasize the centre of chirality in the 3-position) or its salt.

These ketone bodies are also provided physiologically in large amounts from lipids stored in the body by lipolysis during fasting or starvation and replace the energy source glucose almost completely.

The ketone bodies are formed in the liver from acetyl coenzyme A (= acetyl-CoA), which originates from beta-oxidation; they represent a transportable form of the acetyl coenzyme A in the human body. However, in order to utilize the ketone bodies, the brain and muscles must first adapt by expressing enzymes that are required to convert ketone bodies back into acetyl coenzyme A. Especially in times of hunger, the ketone bodies contribute a considerable amount to energy production. For example, after some time the brain is able to get by with only a third of the daily amount of glucose.

Physiologically, the ketone bodies are synthesized from two molecules of activated acetic acid in the form of acetyl coenzyme A, the normal intermediate product of fatty acid degradation, which is extended using a further acetyl coenzyme A unit and the enzyme HMG-CoA-synthase to the intermediate product 3-hydroxy-3-methyl-glutaryl-CoA (HMG-CoA), wherein finally the HMG-CoA-lyase cleaves off the acetoacetate. These three steps take place exclusively in the mitochondria of the liver (i.e. Lynen cycle), wherein 3-hydroxybutyrate is finally formed in the cytosol by the D-beta-hydroxybutyrate dehydrogenase. HMG-CoA is also an end product of the degradation of the amino acid leucine, while acetoacetate is formed during the degradation of the amino acids phenylalanine and tyrosine.

Spontaneous decarboxylation turns acetoacetate into acetone; it can occasionally be perceived in the breath of diabetics and dieters. It cannot be further used by the body. However, the proportion of acetone in the ketone bodies is small.

Acetoacetate is thus reductively converted into the physiologically relevant form of 3-hydroxybutyric acid or 3-hydroxybutyrate, but can also decompose into the physiologically unusable acetone with the release of carbon dioxide, which is detectable and olfactory perceptible in severe ketosis, a ketoacidosis (e. g. in diabetes mellitus type 1 patients without insulin substitution), in the urine and in the exhaled air.

3-Hydroxybutyric acid is currently used and marketed in the weight training sector as a sodium, magnesium or calcium salt.

However, 3-hydroxybutyric acid is not known or only in very small quantities to humans in evolutionary terms, since plants do not produce 3-hydroxybutyric acid and 3-hydroxybutyric acid in the animal organism only occurs in dead emaciated animals in ketosis, so that 3-hydroxybutyric acid causes nausea when administered orally. 3-Hydroxybutyric acid in the form of free acid and its salts also taste very bitter and can cause severe vomiting and nausea.

Moreover, patients, especially newborns, but also adults cannot permanently tolerate large amounts of salts of 3-hydroxybutyric acid, as these compounds can have a kidney-damaging effect.

In addition, the plasma half-life of 3-hydroxybutyric acid and its salts is so short that even if several grams are taken, the ketosis lasts only for about three to four hours, i.e. patients cannot benefit continuously from a therapy with 3-hydroxybutyric acid or its salts, especially at night. In case of metabolic diseases this can lead to life-threatening situations.

Therefore, in the case of the therapy of such metabolic diseases, fatty acid polyol esters, particularly fatty acid glycerol esters, and in particular so-called medium-chain triglycerides (i.e. so-called MCTs or MCT fats/oils) are currently used for ketogenic therapy, i.e. particularly the metabolic conversion of caproic, caprylic and capric acid (i.e. saturated linear C₆-, Cs- and C₁₀-fatty acids) from the corresponding triglycerides is intended.

Medium-chain triglycerides (synonymously also called MCTs, MCT fats, MCT oils or the like) are triglycerides with two or three fatty acids having a linear saturated aliphatic tail of five to twelve carbon atoms, especially six to twelve carbon atoms, i.e. medium-chain fatty acids (MCFAs). Rich food sources for commercial extraction of MCTs include palm kernel oil and coconut oil.

Thus, according to this definition, medium-chain triglycerides (MCTs) are triglycerides containing medium-chain fatty acids. Medium-chain fatty acids particularly and inter alia include caproic (C 6:0), caprylic (C 8:0), capric (C 10:0) and lauric (C 12:0) acids. These acids are saturated fatty acids, which are mainly found in tropical vegetable fats such as coconut oil (approximately 60 %) and palm kernel oil (approximately 55 %) and in butter. To a small extent, they are also found in milk fat (approximately 10 %). Pure MCT oil does not occur in nature but only in mixtures with other triglycerides. MCTs have a slightly lower calorific value or a lower food energy (i.e. 3475 kJ / 100 g) if compared to fat (3852 kJ / 100 g) and also a lower smoke-point if compared to conventional long-chain fats (i.e. LCTs = long-chain triglycerides).

MCT fats are industrially obtained by hydrolysis of coconut oil and palm kernel oil, fractionation of the medium-chain fatty acids and subsequent esterification with glycerol. A pure MCT oil is colourless to yellowish, neutral in odour and taste and of very low viscosity. It is declared as a vegetable oil (i.e. neutral oil or oleum neutrale).

The composition of MCT fats according to the German Pharmacopoeia, 10th Edition, 1991 (i.e. Deutsches Arzneibuch 10 or DAB 10) and corresponding European Pharmacopoeia (Ph. Eur., particularly Ph. Eur. 10.0, Monograph No. 0868 "Triglycerides, medium-chain [Triglycerida saturata media]") is as follows: 50 to 80 % of caprylic acid C₈, 25 to 45 % of capric acid C₁₀, at most 3 % of lauric acid C₁₂ and at most 2 % of caproic acid C₆ (cf. also: Hagers Handbuch der Pharmazeutischen Praxis [Hager's Handbook of Pharmaceutical Practice], 5^{th} Edition, ISBN 978-3-642-63389-8, pages 1059 seq.).

MCTs find a wide range of uses and dosage forms. MCT fats have been produced industrially since 1955 and are used in a variety of applications due to their specific properties. With the GRAS application (generally recognized as safe petition) accepted by the U.S. Food and Drug Administration (FDA) in 1994, MCTs gained interest not least in the food sector. Due to their physical characteristics, the industrially obtained MCT fats also find application in the production of cosmetics (e.g. in ointments, creams, bath oils etc.) and pharmaceuticals (e.g. in tablets, coated tablets etc.), where they may serve particularly as carriers or excipients, solubilizers, release agents, surface-treatment agents, transport agents, lubricants, hydrophobic agents, film-forming and protective agents and viscosity regulators. Furthermore, due to their metabolic peculiarities, they are also used in the form of dietetic foods (e.g. MCT oil and MCT margarine) and products for artificial nutrition (e.g. enteral and parenteral). These products are categorized as dietetic foods for special medical purposes (e.g. balanced diets) according to the German Dietary Products Ordinance (i.e. so-called German "Diätverordnung").

Also, the physiological properties and metabolism of MCTs is of high interest: Due to their shorter fatty acid chain length, MCT fats are relatively soluble in aqueous environments and can therefore be metabolized without bile acids. Their structure also does not require cleavage by pancreatic lipase (i.e. enzyme of the pancreas). They are transported directly in the blood to the liver, bypassing the lymphatic system, where they are preferentially oxidized if compared to conventional fats and the formation of ketone bodies is thus increased. The transport of medium-chain fatty acids (MCFAs) to the mitochondria (i.e. the site of fatty acid oxidation) occurs independently of carnitine. The tolerable daily intake varies individually and is about 50 to about 100 g or more of MCT fats. In order to avoid side-effects (such as diarrhoea, cramps, and headaches) it is common to start with about 20 g of MCT fat per day. This amount can be gradually increased by 5 to 10 g per day. When following a diet with MCT fats, it is essential to ensure the supply of fat-soluble vitamins and essential fatty acids (i.e. omega-3 and omega-6). Fat-soluble vitamins are sufficiently absorbed when MCT fats are used.

Due to their metabolic characteristics, MCT fats are a valuable component of nutritional therapy in the dietetics of various clinical indications.

Medium-chain triglycerides were first used in clinical nutrition in the 1950s for the dietary treatment of malabsorption syndrome, as they are easily soluble in an aqueous environment and are absorbed by the body more quickly than conventional long-chain fats. In cases of lymphangiectasia (i.e. dilatation of lymphatic vessels), Whipple's disease (i.e. a rare infectious disease) and chylothorax (i.e. accumulation of lymphatic fluid in pleural cavity), MCT fats can help relieve congested lymphatic vessels. In premature infants, medium-chain fats are used to achieve weight gain due to the not yet fully developed digestive system.

Since MCT fats can be metabolised independently of pancreatic enzymes and only need to be broken down by gastric lipase, their use is indicated in cases of existing exocrine pancreatic insufficiency with marked steatorrhea (i.e. fatty stools or presence of fat in feces) if enzyme supplementation does not produce the desired effect. Exocrine pancreatic insufficiency occurs, for example, in chronic pancreatitis and cystic fibrosis. Furthermore, their use is also useful in combination with pancreatic supplements. Another classical field of application for MCT fats is short bowel syndrome, in which, depending on the extent and the section of intestine removed, there are digestive disorders, especially of edible fats. If the large intestine is preserved, medium-chain fatty acids can be sufficiently absorbed and represent a suitable alternative to conventional fats. In rare inborn errors of metabolism, such as the defect of β-oxidation of long-chain (LCHAD) and very long-chain fatty acids (VLCAD), medium-chain fatty acids are an essential source of energy. MCT fats are also used in artificial nutrition (i.e. enteral and parenteral) for various diseases, mainly of the gastrointestinal tract.

Since MCT fats have a higher ketogenicity than conventional fats, they are also used in the ketogenic diet (KD), especially as a partial replacement of LCT fats. The ketogenic diet finds application e.g. in pharmacoresistant epilepsy and in some congenital rare metabolic diseases (e.g. pyruvate dehydrogenase deficiency, GLUT 1 defect). The nutrient ratio of the classic form of ketogenic diet is usually 3 to 4 parts fat and one part carbohydrate and protein. Ketogenic diet with MCT fats can be considered a promising alternative to the classical form of diet in children and adults since the higher ketogenicity of MCT fats can shift the ratio of fat to carbohydrate and protein in favour of carbohydrate. Ketogenic diet is also used in the treatment of patients with brain tumors, Parkinson's disease, migraine, Alzheimer's disease and NAFLD (i.e. non-alcoholic fatty liver disease/ hepatitis). Due to the metabolic properties of MCT fats and their lower energy content compared to conventional fats, MCT fats are also used in the field of weight management and metabolic syndrome.

The use of MCT fats in the nutritional therapy of various diseases is to be distinguished from their use in sports nutrition, where they are known as a fast energy supplier and are mainly used in endurance sports.

Medium-chain fatty acids are contraindicated in cases where there is a risk of ketoacidosis, as well as in cases of oxidation disorders of medium-chain fatty acids (MCAD deficiency or MCADD or medium-chain acyl-CoA dehydrogenase deficiency). Due to their low smoke-point, medium-chain fats have only limited heat-resistance, so that manufacturer's information and instructions in this regard should be followed to avoid increased smoke.

Specifically, one particular inventive fatty acid polyol ester, especially fatty acid glycerol ester, of special interest is tricaprylin (which is synonymously also called trioctanoylglyceride, tricaprylyl glycerol, glycerol trioctanoin, tricapryloyl glycerol, octanoic acid-1,2,3-propanetriyl-ester, glycerin tricaprylate, tricaprylyl glycerin, glycerol tricaprylate, glycerol trioctanoate, glyceryl tricaprylate, caprylic triglyceride, tricaprilin, tricaprylyl glycerol etc.) Tricaprylin is the triglyceride of caprylic acid which is usually manufactured by esterification of caprylic acid with glycerin. Tricaprylin occurs as a clear, colourless to pale-yellow liquid. It forms crystals from acetone/ethanol (95 %). Tricaprylin is odourless.

Tricaprylin is used e.g. in pharmaceutical preparations, for example as a neutral carrier or excipient, as an absorption promoter and as a solubilizer for active drugs. It is also used as an oily phase to prepare water-in-oil-in-water multiple emulsions for incorporating water-soluble drugs and also for obtaining stable microcapsules. Tricaprylin also acts as a vehicle for topical creams and lotions as well as cosmetic preparations. It is also used as a penetration-enhancing lipid base with excellent emollient and skin-smoothing properties. Owing to its non-greasy components and low viscosity, it has very good spreadability. In spite of being skin-permeable, tricaprylin does not obstruct natural skin respiration and hence it is used in baby oils, massage oils and face masks. It is an excellent dispersant and acts as a solubilizer, wetting agent and binder in colour cosmetics. Being readily miscible with natural oils and surfactants, tricaprylin is also used as fat component in two-phase foam baths. It is furthermore used in sunscreen creams and oils because of its compatibility with organic and inorganic filter agents. It is also used as a fixative for perfumes and fragrances.

However, as already indicated hereinabove, fatty acid polyol esters, particularly fatty acid glycerol esters, and in particular so-called medium-chain triglycerides (MCTs) and, above all, also tricaprylin have also been used as active ingredients or active compounds in pharmaceutical compositions.

Specifically, a tricaprylin-containing composition was previously marketed under the tradename Axona as a medical food for the clinical dietary management of the impairment of metabolic processes associated with mild to moderate states of Alzheimer's disease. The marketed formulation comprises tricaprylin as fractionated palm kernel oil. During digestion, the caprylic triglyceride is broken down into ketones, which provide an alternative energy source for the brain. Its use is based on the fact that the ability of the brain to use its normal energy source (i.e. glucose) is impaired in Alzheimer's disease.

Consequently, in the prior art there exists a high demand for and an increasing interest in fatty acid polyol esters, particularly fatty acid glycerol esters, such as medium-chain triglycerides (MCTs), especially tricaprylin but also others. Above all, in pharmaceutical and food industry there exists an increasing demand for high-grade fatty acid polyol esters, particularly high-grade fatty acid glycerol esters, with high degrees of active substance and low degrees of impurities, especially toxic impurities.

However, conventional production methods for manufacturing fatty acid polyol esters, particularly fatty acid glycerol esters, such as medium-chain triglycerides (MCTs), especially tricaprylin but also others, including the subsequent multistage refinement treatment (above multistage distillation at elevated temperatures), lead to relatively high degrees of impurities and by-products particularly due to high process temperatures needed and the used starting materials and reagents, particularly halogen-containing substances (e.g. originating from hydrochloric acid HCl or metal-based catalysts used in the esterification process). Especially, when triglycerides with low acid and hydroxyl values and high esterification degrees are required, the mandatory drastic esterification conditions, especially high temperatures over 180 °C and up to 230 °C, the necessary subsequent multistage refinement treatment (above multistage distillation at elevated temperatures) and the use of metal-based catalysts lead to the formation of high contents of toxic by-products and impurities, especially toxic halide-based by-products and impurities.

Above all, conventional production methods for manufacturing fatty acid polyol esters, particularly fatty acid glycerol esters, including the subsequent multistage refinement treatment (above multistage distillation at elevated temperatures), usually produce also relatively high levels of impurities and by-products, especially toxic impurities and by-products, such as, for example, genotoxic glycidol esters of fatty acids (synonymously also called glycidyl esters of fatty acids or simply GEs of fatty acids or only GEs) and nephrotoxic fatty acid esters of monochloropropanediols (synonymously also called MCPD fatty acid esters or simply MCPD esters), especially fatty acid esters of 3-monochloropropanediol (3-MCPD fatty acid esters or 3-MCPD esters) and fatty acid esters of 2-monochloropropanediol (2-MCPD fatty acid esters or 2-MCPD esters).

The following exemplary reaction scheme illustrates the formation of the three aforenamed toxic by-products, i.e. 3-MCPD fatty acid ester, 2-MCPD fatty acid ester and glycidol fatty acid ester for the triglyceride tricaprylin selected as a non-limiting example, which formation occurs when high temperatures (e.g. temperatures of 200 °C and more) are applied to the fatty acid triglyceride (e.g. tricaprylin) in the presence of chlorine-containing substances originating from the production process. The substance 3-chloro-1,2-propanediol originates from glycerine when reacting to 3-MCPD in the presence of chlorine-containing substances or chlorides, respectively.

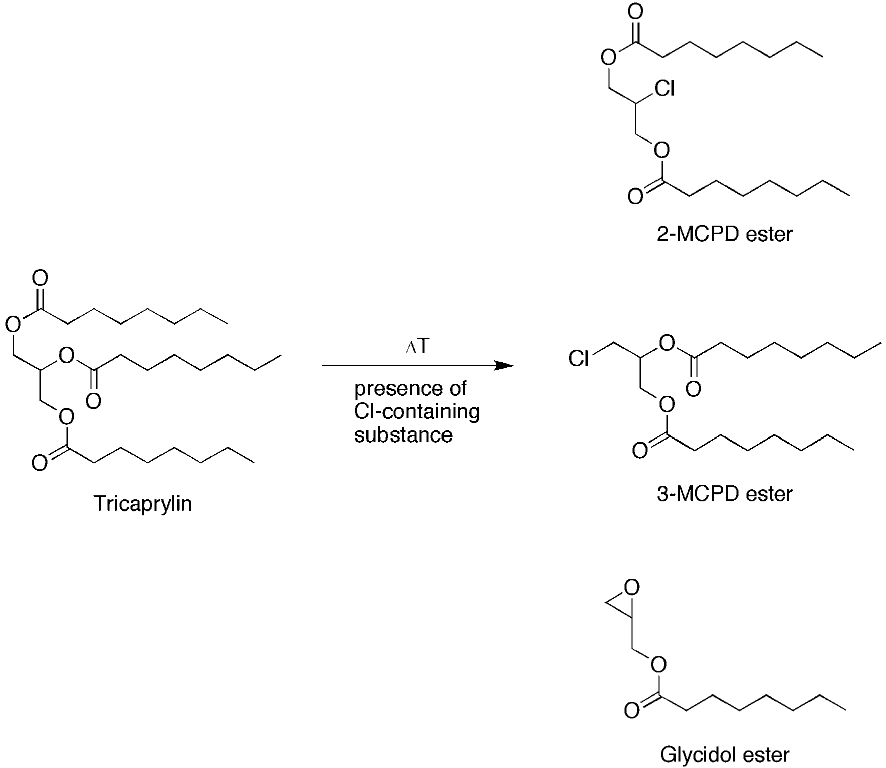

Above all with respect to pharmaceutical and nutritional applications, controlling the impurity profile is crucial and compulsory, especially for known toxic by-products such as the three aforenamed toxic by-products, i.e. 3-MCPD fatty acid ester, 2-MCPD fatty acid ester and glycidol fatty acid ester.

Although it has been well known for decades that, e.g. due to the harsh process conditions occurring during the conventional esterification/condensation methods of fats and oils, these undesired and toxic by-products are formed, no attempts have been made to avoid such harsh conditions by improving or modifying the production processes. For, it is known that mild esterification conditions using biobased catalysts and lower esterification temperatures reduce the formation of toxic by-products, such as e.g. the three aforenamed toxic by-products, i.e. 3-MCPD fatty acid ester, 2-MCPD fatty acid ester and glycidol fatty acid ester, but at the same time only lead to triglycerides with undesirably high acid and hydroxyl values and relatively low triglyceride contents at low conversion rates and yields.

Consequently, instead of trying to avoid the formation of undesired and toxic by-products, rather cost-intensive and burdensome pre- and/or post-treatment methods are effected in order to remove these undesired and toxic by-products from the desired final product obtained under harsh esterification conditions (i.e. especially high temperatures over 180 °C and up to 230 °C and use of metal-based catalysts etc.). Such burdensome post-treatment methods comprise, inter alia, e.g. the addition of strongly alkaline reagents such as KOH, multistage distillation cascades, heat-treatment with strongly alkaline aqueous solutions etc. (e.g. WO 2019/038320 A1, WO 2014/012548 A1 etc.), which post-treatment methods also affect and deteriorate the product quality as such and overall yields and efficiency. For instance, WO 2021/070209 A1 discloses a complex multistage extraction process for the purification of triglycerides, using a saline / organic solvent two-phase extraction system also with chlorinated organic solvents. However, these post-treatment methods mostly also fail to remove last traces of these undesired and toxic by-products, which traces will then remain in the final product. This is of particular danger when applying high dosages of the fatty acid polyol esters, particularly fatty acid glycerol esters, e.g. MCTs such as tricaprylin, in pharmaceutical and nutritional applications (above all in case of parenteral application), which leads to an undesired accumulation of such traces of undesired and toxic by-products in the human body, thus also exceeding the authorised threshold levels of the undesired and toxic by-products as regulated by law.

Moreover, such cost-intensive and burdensome post-treatment methods which aim to effect a only subsequent or retroactive removal of the undesired and toxic by-products are also inefficient since these post-treatment methods often lead to an undesired entrainment of product and do also not increase or influence product quality or product purity such as the esterification degree or the residual content of other impurities (such as e.g. the total halogenated substance content or the total acid content).

For instance, the European Union (EU) has strictly limited the concentration of such toxic by-products and impurities for food-applications by respective legal regulations (e.g. EU Regulations Nos. 2020/1322 and 2018/290), especially for infant application. For example, the maximum permissible content of nephrotoxic 3-MCPD fatty acid esters in liquid infant formula, follow-on formula and foods for special medical purposes intended for infants and young children and young children formula is 15 mg / kg (i.e. 15 ppm) only while the maximum permissible content of genotoxic glycidol fatty acid esters for same formulations is even 6.0 mg / kg (i.e. 6.0 ppm) only. Consequently, the allowed exposure levels of these toxic by-products in nutritional applications are very restricted.

Accordingly, especially for monographed APIs (i.e. Active Pharmaceutical Ingredients) like e.g. MCT oils with a total daily intake of far greater than 10 g / day it is challenging to constantly stay below those maximum exposure levels. In case of parenteral application this will be even more challenging since the toxic substances may directly enter the human body.

A permanent or an even lifelong medication with fatty acid polyol esters, particularly fatty acid glycerol esters, such as medium-chain triglycerides (MCTs), especially tricaprylin but also others, can comprise daily dosages or intakes of even up to 100 g / day. For this purpose, particularly pure grades are required. In contrast to nutritional applications, in case of pharmaceutical applications patients suffering from serious diseases are taking this medication, whose state of health is impaired anyway, so that their level of tolerance is very low, also in view of toxic impurities.

Therefore, it would be desirable to conceive efficient production processes which directly produce high-grade fatty acid polyol esters, particularly fatty acid glycerol esters, such as MCTs (e.g. tricaprylin) etc., having high degrees of purity or minimum levels of by-products and impurities, respectively, as well as exhibiting at the same time high esterification degrees and good stabilities, especially storage-stabilities, and high active ingredient contents, especially without the necessity of additional cost-intensive and burdensome post-treatment operations.

US 2007/148746 A1 refers to a process for the enzymatic synthesis of esters of polyunsaturated, short-chain or sensitive fatty acids with a high triglyceride content, which is made up of an enzymatic synthesis of the mixture of triglycerides and partial glycerides starting from free fatty acid, methyl or ethyl esters and glycerol, a subsequent selective enzymatic back-hydrolysis of the partial glycerides to fatty acid and glycerol and distillation-based separation of the triglycerides, and to the triglyceride-rich ester mixture obtainable by this process.

Moreover, document F. A. Schäfer de Martini Soares et al.: "Chemical interesterification of blends of palm stearin, coconut oil, and canola oil: physiological properties", Journal of Agricultural and Food Chemistry, American Chemical Society, US, Vol. 60, No. 6, 15. February 2012, pages 1461-1469, refers to chemical interesterification of blends of palm stearin, coconut oil and canola oil and its physicochemical properties, wherein softening and melting points, solid fat content and consistency are examined.

Furthermore, document Selmi B. et al.: "Lipase-catalyzed synthesis of tricaprylin in a medium solely composed of substrates. Water production and elimination", Enzyme and Microbial Technology, Vol. 20, No. 5, 1. April 1997, pages 322-325, refers to the lipase-catalyzed synthesis of tricaprylin in a medium solely composed of substrate, wherein water production and elimination with respect to the aforementioned synthesis is investigated.

Document Da Rocha Ataide Terezinha et al.: "Chemical synthesis of tricaproin, trienantin and tricaprylin: Synthesis of medium-chain triacylglycerols", International Journal of Food Science and Technology, Vol. 41, Nr. 12, 18. June 2007, Seiten 1504-1508, refers to the optimization of the glycerol caproic acid / enantic acid / caprylic acid esterification conditions to obtain tricaproin, trienantin and tricaprylin in a medium solely composed of reagents, wherein a reaction without catalyst and solvent is provided.

Consequently, the prior art has not lacked attempts to find efficient production processes but failed to improve the efficiency and performance of existing processes. But so far, however, no efficient production processes have been conceived in the prior art. Also, access to such high-grade fatty acid polyol esters, particularly fatty acid glycerol esters, such as MCTs (e.g. tricaprylin) etc., is not or not easily possible according to the prior art.

The problem underlying the present invention is thus providing an efficient process for producing triglycerides of fatty acids (i.e. fatty acid glycerol triesters), especially with a high purity degree, wherein the aforementioned disadvantages and/or drawbacks of the prior art should be at least partially avoided or even at least essentially overcome.

Such production process should especially make the respective fatty acid polyol esters, particularly fatty acid glycerol esters, such as MCTs (e.g. tricaprylin) etc., accessible in an efficient way, especially with improved qualities and without significant amounts of toxic by-products or impurities, which production process should be feasible on an industrial level, especially on a large-scale level, and without excessive and cost-intensive post-treatment as envisaged in the prior art processes.

In a completely surprising way, applicant has now found out that triglycerides of fatty acids (i.e. fatty acid glycerol triesters), namely fatty acid polyol esters, particularly fatty acid glycerol esters, such as MCTs (e.g. tricaprylin) etc., especially with a high purity degree, can be directly and efficiently produced, above all in an economic and also industrially feasible way, when applying a process as defined in the claims. It could not be foreseen that the application of the inventive process comprising the specific measurements or their combination as defined in the claims would lead to a significant improvement in the production of the aforenamed compounds.

Thus, in order to solve the problem described hereinabove, the present invention therefore proposes a process for producing triglycerides of fatty acids (i.e. fatty acid glycerol triesters), especially with a high purity degree, according to Claim 1; further, especially special and/or advantageous embodiments of the inventive method are the subject-matter of the respective subclaims.

It goes without saying that following features, embodiments, advantages and the like, which are subsequently listed below only with regard to one aspect of the invention for the purpose of avoiding repetition, naturally also apply accordingly to the other aspects of the invention, without this requiring a separate mention.

Furthermore, it goes without saying that individual aspects and embodiments of the present invention are also considered disclosed in any combination with other aspects and embodiments of the present invention and, especially, any combination of features and embodiments, as it results from back references of all patent claims, is also considered extensively disclosed with regard to all resulting combinations possibilities. With respect to all relative or percentage weight-based data provided below, especially relative quantity or weight data, it should further be noted that within the scope of the present invention these are to be selected by the person skilled in the art such that they always add up to 100 % or 100 % by weight, respectively, including all components or ingredients, especially as defined below; however, this is self-evident for the person skilled in the art.

In addition, the skilled person may, if necessary, deviate from the following range specifications without leaving the scope of the present invention.

In addition, it applies that all values or parameters or the like specified in the following can be determined or identified in principle with standardized or explicitly specified determination methods or otherwise with the determination or measurement methods that are otherwise familiar to a person skilled in the art.

Having stated this, the present invention will be described in more detail hereinafter:
The subject-matter of the present invention - according to a **first** aspect of the present invention - is thus a process for producing triglycerides of fatty acids (i.e. fatty acid glycerol triesters), especially with a high purity degree,
   wherein 1,2,3-propantriol (glycerol, glycerine) of formula (I)

      CH₂(OH) - CH(OH) - CH₂(OH) (I)
   or its protected form or precursor, preferably solketal (isopropylidene glycerol), is used as starting material and is reacted and/or converted to the corresponding triglycerides of fatty acids,
   so as to produce, as a reaction product, one or more triglycerides of C₅-C₁₂-fatty acids, especially one or more triglycerides of C₆-C₁₂-fatty acids, of general formula (II)

      CH₂[O - C(O)R¹] - CH[O - C(O)R²] - CH₂[O - C(O)R³] (II)
   wherein in general formula (II) the radicals R¹, R² and R³, independently of one another, each represent a linear or branched, saturated or unsaturated aliphatic C₄-C₁₁-alkyl radical, especially C₅-C₁₁-alkyl radical, preferably a linear saturated aliphatic C₄-C₁₁-alkyl radical, especially C₅-C₁₁-alkyl radical,
   wherein the process comprises at least one esterification step, especially an esterification step using C₅-C₁₂-fatty acids, especially C₆-C₁₂-fatty acids, and/or their anhydrides and/or esters (preferably C₁-C₄-alkyl esters);
   wherein the process is characterized by feature and/or measurement (i), optionally in further combination with feature and/or measurement (ii) and/or feature and/or measurement (iii) and/or feature and/or measurement (iv):
      (i) a preferably selective derivatization of MCPD fatty acid esters (fatty acid esters of monochloropropanediols) and glycidol esters of fatty acids present in the resulting reaction product is performed and/or the resulting reaction product is treated and/or contacted with at least one nucleophilic agent selected from the group consisting of hydrogen sulfites (bisulfites, HSO₃⁻), sulfites (SO₃²⁻), hydrogen thiosulfates (HS₂O₃⁻), thiosulfates (S₂O₃²⁻), hydrogen phosphites (H₂PO₃⁻), phosphites (HPO₃²⁻) and phosphines as well as combinations thereof, more preferably with hydrogen sulfites (bisulfites, HSO₃⁻), in particular for a preferably selective derivatization of MCPD fatty acid esters (fatty acid esters of monochloropropanediols) and glycidol esters of fatty acids;
      (ii) the at least one esterification step is run and/or performed at temperatures not exceeding 180 °C, especially not exceeding 150 °C, particularly not exceeding 120 °C, preferably not exceeding 100 °C, more preferably not exceeding 80 °C, even more preferably not exceeding 50 °C;
      (iii) the 1,2,3-propantriol of formula (I) or its protected form, preferably solketal (isopropylidene glycerol), used as starting material is free of any chlorinated species and comprises a total chlorinated species content of at most 1 ppm, especially at most 0.5 ppm, preferably at most 0.1 ppm, based on the 1,2,3-propantriol of formula (I) or its protected form, preferably solketal (isopropylidene glycerol);
      (iv) the at least one esterification step is run and/or performed in the absence of any metal-based catalyst.

As stated above, the applicant has, quite surprisingly, discovered that the triglycerides of fatty acids (i.e. fatty acid glycerol triesters), especially triglycerides of C₅-C₁₂-fatty acids, particularly triglycerides of C₆-C₁₂-fatty acids, such as MCTs (e.g. tricaprylin) etc., may thus be produced in an efficient and economic way, particularly with a high purity degree, especially with a quality appropriate for pharmaceutical or clinical applications as well as nutritional applications.

The above-mentioned triglycerides of fatty acids (i.e. fatty acid glycerol triesters), especially triglycerides of C₅-C₁₂-fatty acids, particularly triglycerides of C₆-C₁₂-fatty acids, such as MCTs (e.g. tricaprylin) etc., are thus accessible, for the first time, in a direct and efficient manner with such high purity through the production process according to the invention.

Above all, also very surprisingly, when applying at the last one of the aforenamed features and/or measurements (i) to (iv), the formation of toxic by-products and impurities, such as e.g. genotoxic glycidyl esters of fatty acids and nephrotoxic fatty acid esters of monochloropropanediols such as 3-MCPD fatty acid esters and 2-MCPD fatty acid esters can be efficiently avoided or at least minimized a priori, so that burdensome and cost-intensive post-treatment operations for their removal, as envisaged in the prior art, are not required. Any potential traces of toxic by-products and impurities are easily and efficiently removed by the aforementioned measurement (i), as will be delineated in detail in the following.

For, surprisingly, as applicant has found out in a not expected manner, when applying at least one of the features and/or measurements (i) to (iv), especially a combination of at least two, preferably a combination of at least three, more preferably a combination of all four, of the above features and/or measurements (i) to (iv), triglycerides of fatty acids with a high purity degree result, i.e. irrespective of any specific reaction route for these substances.

Above all, absolutely surprisingly, when applying at least one of the features and/or measurements (i) to (iv), especially a combination of at least two, preferably a combination of at least three, more preferably a combination of all four, of the above features and/or measurements (i) to (iv), triglycerides of fatty acids with a high purity degree result, which at the same time unify a combination of five purity properties (1) to (5), namely: (1) a very low acid value (AV), (2) a very low hydroxyl value (OHV), (3) a very high triglyceride content, (4) a very low total content of MCPD fatty acid esters (fatty acid esters of monochloropropanediols) and (5) a very low total content of glycidol esters of fatty acids. Such combination of purity properties (1) to (5) was deemed incompatible or non-realizable so far in the prior art. However, as applicant has surprisingly found out, when applying at least one of the features and/or measurements (i) to (iv), especially a combination of at least two, preferably a combination of at least three, more preferably a combination of all four, of the above features and/or measurements (i) to (iv), this purity specification can be reached independently from any specific synthesis route.

**In** this respect, above the following finding by applicant is very surprising: **In** the prior art, it is mainly the high-temperature post-treatment procedure, especially multistage refinement treatment (above multistage distillation at elevated temperatures), that is deemed to be responsible for the formation of high degrees of impurities and by-products (predominantly caused by glycidol fatty acids esters and MCPD fatty acid esters); for, it is known that refinement treatment under application of heat or high temperatures leads to these toxic by-products. But, as applicant has now surprisingly found out, already the *de-novo* synthesis per se of fatty acid polyol esters, particularly fatty acid glycerol esters, such as medium-chain triglycerides (MCTs), especially tricaprylin but also others, produces high amounts of these undesired toxic by-products when - apart from the necessary subsequent multistage refinement post-treatment (above multistage distillation at elevated temperatures), which already produces higher amounts of these toxic by-products anyway - in addition drastic esterification conditions, especially high temperatures over 180 °C and up to 230 °C combined with the use of metal-based catalysts, are applied in order to achieve low acid and hydroxyl values but also high esterification degrees. Thus, the finding by applicant is completely astonishing that the avoidance intention with respect to these toxic by-products has to be started much earlier in process line (i.e. on behalf of synthesis itself and also already in the upstream selection of the purity grade of the glycerol-based starting materials).

Although much effort has been made in the last decade in finding optimized refinement conditions for natural oils and fats, in particular in palm oil, in order to reduce the content of toxic by-products, above all MCPD and glycidol fatty acid esters, there was no systematic attempt in the prior art to find adequate conditions in the case of a conventional condensation reaction for the manufacturing of synthetic triglycerides. However, a simple transfer of the teachings from the studies related to natural oil refinement is by far not sufficient since these teachings focus exclusively on an *a posteriori* or retroactive impurity reduction of by-products already formed and present in the final products but without taking into account the simultaneously existing need for a high-grade quality (i.e. the necessity of an *a priori* avoidance of the formation of these undesired toxic by-products in the origin of their formation). Furthermore, those prior teachings fail to give any root cause analysis regarding the origin of these toxic by-products, above all MCPD and glycidol fatty acid esters.

Consequently and absolutely surprisingly, as applicant has unexpectedly found out, when applying at least one of the features and/or measurements (i) to (iv), especially a combination of at least two, preferably a combination of at least three, more preferably a combination of all four, of the above features and/or measurements (i) to (iv), the abovedefined purity specification, unifying a combination of the abovenamed five purity properties (1) to (5), can be efficiently reached. Thus, according to the present invention, the aforenamed features and/or measurements, especially the aforenamed features and/or measurements (ii) to (iv), prevent the formation of toxic by-products, such as glycidol fatty acids esters and MCPD fatty acid esters, in its origin by avoiding the cause of their formation while predominantly feature and/or measurement (i) enables the efficient and selective removal of any traces of these toxic by-products nevertheless formed or entrained otherwise.

Thus, for the very first time, applicant has been able to provide a concept for producing high-purity triglycerides of fatty acids with high yields and conversion rates, which concept is independent from any specific synthesis route. **In** other words, applicant has been able, for the very first time, to provide a respective concept which is universally for this purpose.

By a preferably selective derivatization of halide-based impurities present in the resulting reaction product and/or treatment of the resulting reaction product with at least one nucleophilic agent, respectively, by selecting relatively mild esterification conditions in the absence of any metal-based catalyst and/or by selecting the purity grade of the glycerol starting material it is surprisingly possible to yield triglycerides of C₅-C₁₂-fatty acids, especially triglycerides of C₆-C₁₂-fatty acids, exhibiting a multiple combination of the product properties of high triglyceride contents, low acid and hydroxyl values as well as low total contents of MCPD and glycidol fatty acid esters while avoiding harsh esterification conditions and burdensome and inefficient pre- and/or post-treatment procedures.

Apart from this, the inventive process is at the same time highly flexible and compatible with known prior art production processes and plants for these substances since the aforenamed features and/or measurements (i) to (iv) can be easily implemented in or used to modify known production processes and plants for these substances.

**In** contrast to this, the prior art production of these substances by means of conventional synthesis processes with burdensome and cost-intensive post-treatment is very complex, expensive and inefficient since prior art synthesis processes produce quite large amounts of the aforenamed toxic by-products and impurities which then have to be separated off from the respective crude product in complex and mostly multi-stage post-treatment operations. Above all, the burdensome and cost-intensive post-treatment operations can also not increase the product quality with respect to high esterification degrees and low acid degrees in the final products. Also, the yields of the production process as such cannot be influenced in this way.

Within the scope of the present invention, it was possible for the first time to provide an efficiently working production process with which triglycerides of fatty acids (i.e. fatty acid glycerol triesters), especially triglycerides of C₅-C₁₂-fatty acids, particularly triglycerides of C₆-C₁₂-fatty acids, such as MCTs (e.g. tricaprylin) etc., can be produced with high content degrees of these substances, high esterification degrees and very low acid degrees while at the same time being free or at least essentially free of the aforenamed toxic by-products and impurities.

**In** addition, the production process according to the invention can be operated economically and can also be implemented on a large scale.

Especially, the inventive production process uses commercially available starting compounds and furthermore allows a relatively simple process management even in case of large-scale implementation.

**In** contrast to conventional prior art production methods requiring additional complex post-treatment operations, the production process according to the invention does not use such complex post-treatment operations but nevertheless achieves excellent yields, wherein the formation of toxic by-products is minimized or even avoided at all.

**In** addition, the inventive process is easy to be performed and economical. Especially, the production process according to the invention, especially esterification, is usually carried out in the absence of solvents and/or without any solvent (i.e. as a reaction in mass or as a reaction in substance or as a so-called *bulk reaction);* consequently, the reaction products obtained are not contaminated with solvent and no solvent has to be removed and disposed of or recycled in a costly and energy-intensive manner after the process or reaction has been carried out. Furthermore, no or at least essentially no toxic by-products are formed.

To sum up, as stated hereinbefore, the present invention thus refers to a process for producing triglycerides of fatty acids (i.e. fatty acid glycerol triesters), especially with a high purity degree,
wherein 1,2,3-propantriol (glycerol, glycerine) of formula (I)

   CH₂(OH) - CH(OH) - CH₂(OH) (I)
or its protected form or precursor, preferably solketal (isopropylidene glycerol), is used as starting material and is reacted and/or converted to the corresponding triglycerides of fatty acids,
so as to produce, as a reaction product, one or more triglycerides of C₅-C₁₂-fatty acids, especially one or more triglycerides of C₆-C₁₂-fatty acids, of general formula (II)

   CH₂[O - C(O)R¹] - CH[O - C(O)R²] - CH₂[O - C(O)R³] (II)
wherein in general formula (II) the radicals R¹, R² and R³, independently of one another, each represent a linear or branched, saturated or unsaturated aliphatic C₄-C₁₁-alkyl radical, especially C₅-C₁₁-alkyl radical, preferably a linear saturated aliphatic C₄-C₁₁-alkyl radical, especially C₅-C₁₁-alkyl radical,
wherein the process comprises at least one esterification step, especially an esterification step using C₅-C₁₂-fatty acids, especially C₆-C₁₂-fatty acids, and/or their anhydrides and/or esters (preferably C₁-C₄-alkyl esters).

As also stated hereinabove already, the inventive process is characterized by feature and/or measurement (i), optionally in further combination with feature and/or measurement (ii) and/or feature and/or measurement (iii) and/or feature and/or measurement (iv), which features and/or measurements (i) to (iv) will be explained in more detail hereinafter, namely:
(i) a preferably selective derivatization of halide-based impurities, namely of MCPD fatty acid esters (fatty acid esters of monochloropropanediols) and glycidol esters of fatty acids, present in the resulting reaction product is performed and/or the resulting reaction product is treated and/or contacted with at least one nucleophilic agent selected from the group consisting of hydrogen sulfites (bisulfites, HSO₃⁻), sulfites (SO₃² ), hydrogen thiosulfates (HS₂O₃⁻), thiosulfates (S₂O₃² ), hydrogen phosphites (H₂PO₃⁻), phosphites (HPO₃²⁻) and phosphines as well as combinations thereof, more preferably with hydrogen sulfites (bisulfites, HSO₃⁻), in particular for a preferably selective derivatization of halide-based impurities, namely of MCPD fatty acid esters (fatty acid esters of monochloropropanediols) and glycidol esters of fatty acids;
(ii) the at least one esterification step is run and/or performed at temperatures not exceeding 180 °C, especially not exceeding 150 °C, particularly not exceeding 120 °C, preferably not exceeding 100 °C, more preferably not exceeding 80 °C, even more preferably not exceeding 50 °C;
(iii) the 1,2,3-propantriol of formula (I) or its protected form, preferably solketal (isopropylidene glycerol), used as starting material is free of any chlorinated species and comprises a total chlorinated species content of at most 1 ppm, especially at most 0.5 ppm, preferably at most 0.1 ppm, based on the 1,2,3-propantriol of formula (I) or its protected form, preferably solketal (isopropylidene glycerol);
(iv) the at least one esterification step is run and/or performed in the absence of any metal-based catalyst.

As to feature and/or measurement (i), the following is to be emphasized and should be taken into account: According to said feature and/or measurement (i), it is envisaged that a preferably selective derivatization of halide-based impurities, namely of MCPD fatty acid esters (fatty acid esters of monochloropropanediols) and glycidol esters of fatty acids, present in the resulting reaction product is performed and/or the resulting reaction product is treated and/or contacted with at least one nucleophilic agent selected from the group consisting of hydrogen sulfites (bisulfites, HSO₃⁻), sulfites (SO₃² ), hydrogen thiosulfates (HS₂O₃⁻), thiosulfates (S₂O₃² ), hydrogen phosphites (H₂PO₃⁻), phosphites (HPO₃²⁻) and phosphines as well as combinations thereof, more preferably with hydrogen sulfites (bisulfites, HSO₃⁻), in particular for a preferably selective derivatization of halide-based impurities, namely of MCPD fatty acid esters (fatty acid esters of monochloropropanediols) and glycidol esters of fatty acids.

As applicant has surprisingly found out, when using the aforementioned nucleophilic agents, a preferably selective derivatization of halide-based impurities, namely of MCPD fatty acid esters and glycidol esters of fatty acids, is effected. This is even more surprising since these halide-based impurities are usually present in very low amounts only, especially only in traces; nevertheless, the aforedescribed treatment with the aforedefined nucleophilic agents usually leads to a selective derivatization of these substances according to the following reaction scheme, which derivatives can then be easily removed or eliminated, i.e. separated off from the final reaction product (see below explanations). The following reaction scheme shows, as a merely illustrating but not limiting example, the selective derivatization of halide-based impurities, namely the selective derivatization of a MCPD fatty acid ester (above reaction), on the one hand, and the selective derivatization of a glycidol fatty acid ester (below reaction), on the other hand, wherein sodium hydrogen sulfite (sodium bisulfite, NaHSO₃) is used (as a merely illustrating but not limiting example) as a nucleophilic agent and the radical R, independently of each other, denotes a linear or branched, saturated or unsaturated aliphatic C₄-C₁₁-alkyl radical, especially C₅-C₁₁-alkyl radical, preferably a linear saturated aliphatic C₄-C₁₁-alkyl radical, especially C₅-C₁₁-alkyl radical.

As may be seen from this reaction scheme, the nucleophilic agent replaces, via a so-called nucleophilic substitution, preferably via a so-called S_{N}2-like reaction mechanism, the organically bonded chlorine atom in the MCPD fatty acid ester and the chlorine atom is removed as sodium chloride, which can be easily separated off with the water-based phase together with the substitution product (above reaction). As may be seen from this reaction scheme, the nucleophilic agent can also react with the glycidol fatty acid ester under ring opening to yield also a water-soluble product (below reaction). Separation of the purified triglyceride product, after separation of the water-based phase, can e.g. be effected via distillation (e.g. short-path distillation).

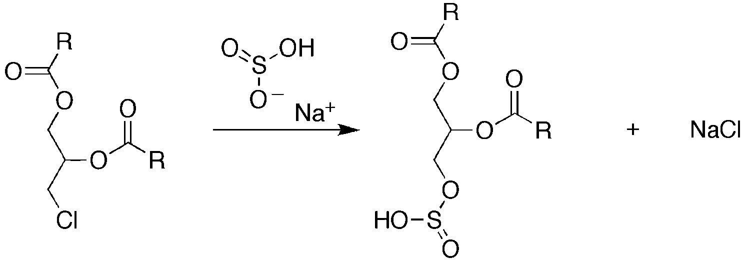

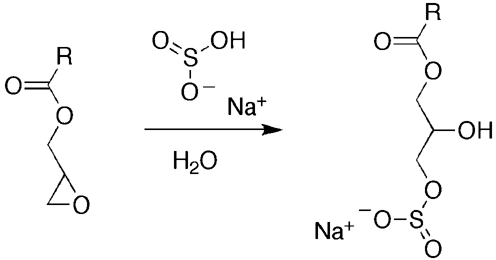

As shown by the above reaction scheme, the halide-based impurities (i.e. MCPD fatty acid esters and glycidol fatty acid esters) can be efficiently removed by selective reaction with the nucleophilic agent (here: e.g. bisulfite = hydrogen sulfite). The halide-based impurities are selectively converted (even if present in traces only) into water-soluble molecules which can then be easily removed, especially by e.g. washing out with water or short-path distillation.

Thus, by the inventive feature and/or measurement (i), halide-based impurities, especially MCPD and glycidol fatty acid esters, can be selectively removed by derivatization, however, without affecting or influencing the other relevant product properties, such as e.g. acid value (AV), hydroxyl value (OHV) and triglyceride content.

Thus, above feature and/or measurement (i) is an efficient means in order to selectively remove toxic halide-based impurities such as e.g. genotoxic glycidol esters of fatty acids and nephrotoxic fatty acid esters of MCPD. The resulting derivatization products can be easily removed, i.e. by washing out or by short-path distillation (wherein the derivatized products usually remain or are kept in the distillation residue (sump) in case of short-path distillation). As also shown in the above reaction scheme, the chlorine atom is preferably converted into harmless sodium chloride, which is easily washed out with the water phase.

Generally, it is preferred that the above feature and/or measurement (i) is performed as a one-stage or single-step treatment. However, particularly in case of higher degrees of impurifications, it is also possible to perform the treating and/or contacting of the triglycerides of fatty acids to be purified with the nucleophilic agent repeatedly, especially in two or more cycles and/or as a multistage process. Nevertheless, it is preferred to perform the treating and/or contacting of the triglycerides of fatty acids to be purified with the nucleophilic agent in one stage (i.e. without any repeated treating and/or contacting); particularly in case of higher degrees of impurifications, it is also possible, as an alternative embodiment, to use higher amounts or concentrations of the nucleophilic agent instead of performing a repeated treating and/or contacting.

Consequently, by the inventive feature and/or measurement (i), the selective elimination or removal even of low amounts, preferably traces, of toxic halide-based impurities is possible in an efficient and selective way, namely without influencing other required and desired properties of the resulting product (such as e.g. low acid values and low hydroxyl values as well as high triglyceride contents).

As to feature and/or measurement (ii), the following is to be emphasized and should be taken into account: According to said feature and/or measurement (ii), it is envisaged that the at least one esterification step is run and/or performed at temperatures not exceeding 180 °C, especially not exceeding 150 °C, particularly not exceeding 120 °C, preferably not exceeding 100 °C, more preferably not exceeding 80 °C, even more preferably not exceeding 50 °C.

Surprisingly, as applicant has found out, the limitation of the temperatures during the at least one esterification step is also an efficient means in order to avoid the formation of toxic impurities, namely MCPD and glycidol fatty acid esters, during esterification. Nevertheless, high conversion rates, high yields as well as high esterification levels, particularly also high triglyceride contents with low acid and hydroxyl values, are reached on behalf of the inventive process, especially when taking into consideration other features and/or measurements, particularly when applying the synthesis paths as described hereinafter.

Consequently, the inventive process renders it possible, for the first time, to run the at least one esterification step under relatively mild conditions, especially for the purpose of avoiding the formation of toxic side-products of the aforementioned kind, while at the same time ensuring high esterification levels, especially high triglyceride contents, and excellent conversion rates and yields.

Above all in combination with at least one of the other aforementioned features and/or measurements (i), (iii) and/or (iv), the inventive feature and/or measurement (ii) leads to excellent results.

As to feature and/or measurement (iii), the following is to be emphasized and should be taken into account: According to said feature and/or measurement (iii), it is envisaged that the 1,2,3-propantriol of formula (I) or its protected form, preferably solketal (isopropylidene glycerol), used as starting material is free of any chlorinated species and comprises a total chlorinated species content of at most 1 ppm, especially at most 0.5 ppm, preferably at most 0.1 ppm, based on the 1,2,3-propantriol of formula (I) or its protected form, preferably solketal (isopropylidene glycerol).

As applicant has surprisingly found out, the presence of chlorinated species, especially the so-called total chlorinated species content, has a crucial influence on the formation of toxic side-products in the final reaction product, namely with respect to the formation of toxic MCPD and glycidol fatty acid esters.

Thus, the selection of the starting materials in the form of 1,2,3-propantriol or its protective form, preferably solketal, as to their purity grades very surprisingly has a key role in avoiding the formation of toxic side-products (e.g. MCPD and glycidol fatty acid esters) during esterification. By selecting these starting material as being at least essentially free of any chlorinated species, particularly with the indicated total chlorinated species contents, the formation of these toxic side-products is efficiently prevented or at least minimized to a maximum extent.

In the prior art, it was completely unknown and not realized at all so far that the content of chlorinated species in the starting materials in the form of 1,2,3-propantriol or its protected form (e.g. solketal) has such decisive influence on the purity degree of the resulting final reaction product (i.e. the triglycerides).

Starting products in the form of 1,2,3-propantriol or its protected form, preferably solketal, with the required purities are in general available as marketed products.

1,2,3-propantriol with the required purity degree as used in the present invention may be obtained e.g. from petrochemical source originating from epichlorine-hydrine or from oleochemical source originating from e.g. hydrolytic splitting of natural fats and oils. The petrochemically or oleochemically derived glycerols with appropriate purity degrees and qualities are obtained mainly via purification by physical techniques (e.g. distillation and membrane-filtration such as membrane-ultrafiltration).

As experimental data by applicant surprisingly show, in contrast to the chlorinated species content of the glycerol or its protected derivative (e.g. solketal) as starting materials, the chlorinated species content of the remaining starting materials, i.e. the C₅-C₁₂-fatty acids, especially C₆-C₁₂-fatty acids, and/or their anhydrides and/or esters used for the at least esterification step, does not play such a key role. Esterification performed with the C₅-C₁₂-fatty acids, especially C₆-C₁₂-fatty acids, and/or their anhydrides and/or esters comprising total chlorinated species contents in the range of from 20 ppm to 100 ppm do not lead to the formation of toxic MCPD and glycidol esters during esterification provided that, at the same time, 1,2,3-propantriol or its protective form, preferably solketal, with the inventive purity grades (i.e. total chlorinated species content of at most 1 ppm, especially at most 0.5 ppm, preferably at most 0.1 ppm) is used. In turn, as further experimental data by applicant also surprisingly show, 1,2,3-propantriol or its protective form, preferably solketal, comprising total chlorinated species contents in the range of from 20 ppm to 100 ppm lead to the significant formation of toxic MCPD and glycidol fatty acid esters during esterification even if the remaining starting materials, i.e. the C₅-C₁₂-fatty acids, especially C₆-C₁₂-fatty acids, and/or their anhydrides and/or esters, comprise high purity grades (i.e. total chlorinated species content of at most 1 ppm, especially at most 0.5 ppm, preferably at most 0.1 ppm). Without being bound to any specific theory, this surprising phenomenon might possibly be explained by the fact that 1,2,3-propantriol or its protective form, preferably solketal, at least predominantly comprises the chlorinated species in organically bonded form where they contribute to form MCPD and glycidol fatty acid esters during esterification, whereas in case of the C₅-C₁₂-fatty acids, especially C₆-C₁₂-fatty acids, and/or their anhydrides and/or esters, these substances might at least predominantly comprise entrained chlorinated species in an inorganic form, especially as inorganic salts such as NaCl, which cannot contribute to form MCPD and glycidol fatty acid esters during esterification at all (or at most to a negligible extent only).

Consequently, on the whole, the specific selection of the aforenamed glycerol-based and solketal-based starting materials with respect to their purity degree, especially their content of chlorinated species, and the minimization of the total chlorinated species content in these starting materials also plays a key role for the purity and quality of the final reaction product.

As to feature and/or measurement (iv), the following is to be emphasized and should be taken into account: According to said feature and/or measurement (iv), it is envisaged that the at least one esterification step is run and/or performed in the absence of any metal-based catalyst.

Performing the at least one esterification step in the absence of any metal-based catalyst also is a key issue in the inventive process since such metal-based catalysts often introduce or entrain significant amounts of undesired halides, especially chlorides. The thus introduced or entrained halides, especially chlorides, may then lead to the undesired formation of toxic side-products, especially MCPD and glycidol fatty acid esters, during esterification. By avoiding such metal-based catalyst during esterification, the uncontrolled and undesired introduction or entrainment of halide-based impurities is thus efficiently avoided or prevented.

Furthermore, in contrast to enzyme-based catalysts used during esterification according to the inventive process according to particular embodiments, metal-based catalysts (such as e.g. tetrabutyl titanate) require relative high temperatures (e.g. between 190 °C and 230 °C) for esterification, thus leading to the undesired and uncontrolled formation of the aforementioned toxic side-products, especially MCPD and glycidol fatty acid esters.

Consequently, also the inventive feature and/or measurement (iv) significantly contributes to obtaining the final reaction product with the desired characteristics with respect to an excellent purity degree and other properties, such as e.g. low acid values, low hydroxyl values and high triglyceride contents.

According to the present invention, the process of the present invention produces triglycerides of fatty acids (i.e. fatty acid glycerol triesters) with a high purity degree.

Particularly, according to the process of the present invention, the reaction product, especially the one or more triglycerides of C₅-C₁₂-fatty acids, especially one or more triglycerides of C₆-C₁₂-fatty acids, of general formula (II)

CH₂[O - C(O)R¹] - CH[O - C(O)R²] - CH₂[O - C(O)R³] (II)

wherein in general formula (II) the radicals R¹, R² and R³, independently of one another, each represent a linear or branched, saturated or unsaturated aliphatic C₄-C₁₁-alkyl radical, especially C₅-C₁₁-alkyl radical, preferably a linear saturated aliphatic C₄-C₁₁-alkyl radical, especially C₅-C₁₁-alkyl radical,
is/are characterized by and/or exhibit(s) the following properties (1) to (5) in their combination and/or fulfil(s) the following product specification:
   (1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
   (2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
   (3) triglyceride content, especially determined according to Ph. Eur. 2.2.28 (European Pharmacopoeia 10.0): ≥ 99 %, especially ≥ 99.2 %, preferably ≥ 99.5 %, each based on the reaction product; and
   (4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the reaction product; and
   (5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.5 ppm, especially ≤ 0.2 ppm, preferably ≤ 0.1 ppm, each based on the reaction product.

Particularly, in other words, according to the present invention, the process is performed such and/or feature and/or measurement (i), optionally in further combination with feature and/or measurement (ii) and/or feature and/or measurement (iii) and/or feature and/or measurement (iv), is applied and/or performed with the proviso that the reaction product, especially the one or more triglycerides of C₅-C₁₂-fatty acids, especially one or more triglycerides of C₆-C₁₂-fatty acids, of general formula (II)

CH₂[O - C(O)R¹] - CH[O - C(O)R²] - CH₂[O - C(O)R³] (II)

wherein in general formula (II) the radicals R¹, R² and R³, independently of one another, each represent a linear or branched, saturated or unsaturated aliphatic C₄-C₁₁-alkyl radical, especially C₅-C₁₁-alkyl radical, preferably a linear saturated aliphatic C₄-C₁₁-alkyl radical, especially C₅-C₁₁-alkyl radical,
is/are characterized by and/or exhibit(s) the following properties (1) to (5) in their combination and/or fulfil(s) the following product specification:
   (1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
   (2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
   (3) triglyceride content, especially determined according to Ph. Eur. 2.2.28 (European Pharmacopoeia 10.0): ≥ 99 %, especially ≥ 99.2 %, preferably ≥ 99.5 %, each based on the reaction product; and
   (4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the reaction product; and
   (5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.5 ppm, especially ≤ 0.2 ppm, preferably ≤ 0.1 ppm, each based on the reaction product.

Surprisingly, the reaction product obtainable from the inventive process unifies the aforementioned five properties (1) to (5), which have been deemed so far incompatible with one another in their overall combination. For, according to prior art methods, when increasing the triglyceride content and lowering acid and hydroxyl values, usually high total contents of MCPD and glycidol fatty acid esters are effected due to drastic esterification conditions; in turn, when lowering the total contents of MCPD and glycidol fatty acid esters, according to the prior art, relatively low triglyceride contents with high acid and hydroxyl values are reached.

In contrast to this prior art, the present invention renders it, for the first time, possible to provide a combination of all aforementioned five properties (1) to (5) in one and the same product. Consequently, the reaction products resulting from the inventive process are highly suitable for food and pharmaceutical applications, even in parenteral applications with high dosages and/or concentrations.

According to the present invention, the reaction product resulting from the process of the present invention has a very low acid value (AV), namely an acid value (AV), especially determined according to Ph. Eur. 2.5.1 (European Pharmacopoeia 10.0), ≤ 0.5 mg KOH / g, especially ≤ 0.2 mg KOH / g, preferably ≤ 0.1 mg KOH / g. The parameter of the so-called acid value indicates the residual free acids originating from esterification. The lower the acid value, the better the product quality. Especially, very low acid values as envisaged by the present invention lead to a high stability of the reaction product, especially an excellent long-term stability and/or storage stability as well as an excellent shelf-life stability, of the resulting reaction product (i.e. triglycerides).

As indicated hereinabove, according to the present invention, the acid value (AV) is usually determined according to Ph. Eur. 2.5.1 (European Pharmacopoeia 10.0). The acid value (synonymously also called neutralization number or acid number or acidity) is the mass of potassium hydroxide (KOH) in milligram that is required to neutralize one gram of chemical substance; the acid number particularly is a measure of the number of carboxylic acid groups in a chemical compound (here: triglyceride). The indicated standard Ph. Eur. 2.5.1 is based on a titration method.

Apart from the parameter of the acid value (AV), also the hydroxyl value (OHV) of the inventive reaction products obtained from the process of the present invention plays a key role. According to the present invention, the inventive reaction product has also a very low hydroxyl value (OHV), namely a hydroxyl value (OHV), especially determined according to Ph. Eur. 2.5.3 (European Pharmacopoeia 10.0), ≤ 0.5 mg KOH / g, especially ≤ 0.2 mg KOH / g, preferably ≤ 0.1 mg KOH / g. **In** the present case, the hydroxyl value (OHV) is a measure for the esterification degree and indicates very high esterification levels in the inventive reaction products.

According to the present invention, the hydroxyl value (OHV) is especially determined according to Ph. Eur. 2.5.3 (European Pharmacopoeia 10.0). The hydroxyl value determined according to this method is defined as the number of milligrams of potassium hydroxide (KOH) required to neutralize the acetic acid taken up in acetylation of one gram of a chemical substance that contains free hydroxyl groups (here: triglycerides). Thus, the hydroxyl value is a measure of a content of free hydroxyl groups in a chemical substance (here: triglycerides), usually expressed in units of the mass of potassium hydroxide (KOH) in milligrams equivalent to the hydroxyl content of one gram of the chemical substance. The indicated determination method Ph. Eur. 2.5.3 (European Pharmacopoeia 10.0) comprises acetylation of the free hydroxyl groups of the reaction product (here: triglycerides) with acetic anhydride in pyridine solvent, wherein, after completion of the reaction, water is added and the remaining unreacted acetic anhydride is converted to acetic acid and measured by titration with potassium hydroxide.

Thus, in case of the inventive reaction products, the triglycerides of the present invention, apart from a very low acid value (AV), also comprise, at the same time, very low hydroxyl values (OHV), as indicated hereinabove, corresponding to high esterification degrees.

Furthermore, as a third parameter and/or characteristic (3), the reaction products exhibit a very high triglyceride content, namely a triglyceride content especially determined according to Ph. Eur. 2.2.28 (European Pharmacopoeia 10.0), ≥ 99 %, especially ≥ 99.2 %, preferably ≥ 99.5 %, each based on the reaction product. These extremely high values for the triglyceride content also indicate the high purity grade of the inventive reaction products. The triglyceride content of the inventive products is particularly determined according to Ph. Eur. 2.2.28 (European Pharmacopoeia 10.0). This method is based on determination via gas chromatography.

Apart from the parameters and/or characteristics of the low acid value (AV), low hydroxyl value (OHV) and high triglyceride content, the reaction products are also characterized by extremely low total contents of MCPD fatty acid esters and glycidol fatty acid esters: The total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]), is ≤ 0.5 ppm, especially ≤ 0.2 ppm, preferably ≤ 0.1 ppm, each based on the reaction product and; the total content of glycidol esters of fatty acids, especially determined according to method of DGF C-VI 18 (10) (Einheitsmethode der Deutschen Gesellschaft für Fettwissenschaft e.V. [Standard method of German Society for Fat Science]), is ≤ 0.5 ppm, especially ≤ 0.2 ppm, preferably ≤ 0.1 ppm, each based on the reaction product.

The indicated determination method of DGF C-VI 18 (10) allows a reliable quantification of these toxic impurities. Above all, the inventive reaction products resulting from the process of the present invention combine these very low total contents of toxic esters with high triglyceride content levels and low acid and hydroxide values, as indicated hereinabove.

Consequently, the fivefold combination of the aforementioned characteristics and/or parameters (1) to (5) is a unique feature of the reaction products obtained from the process of the present invention and delimits these reaction products from prior art reaction products of same kind. Thus, for the very first time, the process of the present invention allows producing triglycerides of C₆-C₁₂-fatty acids with high purity grades, indicated by low acid values and low hydroxyl values, while at the same time providing very high triglyceride contents and extremely low total contents of toxic MCPD and glycidol fatty acid esters.

According to a particular embodiment, the present invention relates to a process for producing triglycerides of fatty acids (i.e. fatty acid glycerol triesters), especially with a high purity degree, especially to the process as described or defined hereinbefore,
wherein 1,2,3-propantriol (glycerol, glycerine) of formula (I)

   CH₂(OH) - CH(OH) - CH₂(OH) (I)
or its protected form or precursor, preferably solketal (isopropylidene glycerol), is used as starting material and is reacted and/or converted to the corresponding triglycerides of fatty acids,
so as to produce, as a reaction product, one or more triglycerides of C₅-C₁₂-fatty acids, especially one or more triglycerides of C₆-C₁₂-fatty acids, of general formula (II)

   CH₂[O - C(O)R¹] - CH[O - C(O)R²] - CH₂[O - C(O)R³] (II)
wherein in general formula (II) the radicals R¹, R² and R³, independently of one another, each represent a linear or branched, saturated or unsaturated aliphatic C₄-C₁₁-alkyl radical, especially C₅-C₁₁-alkyl radical, preferably a linear saturated aliphatic C₄-C₁₁-alkyl radical, especially C₅-C₁₁-alkyl radical,
wherein the process comprises at least one esterification step, especially an esterification step using C₅-C₁₂-fatty acids, especially C₆-C₁₂-fatty acids, and/or their anhydrides and/or esters (preferably C₁-C₄-alkyl esters);
wherein the process is characterized by feature and/or measurement (i), optionally in further combination with feature and/or measurement (ii) and/or feature and/or measurement (iii) and/or feature and/or measurement (iv):
   (i) a preferably selective derivatization of halide-based impurities, namely of MCPD fatty acid esters (fatty acid esters of monochloropropanediols) and glycidol esters of fatty acids, present in the resulting reaction product is performed and/or the resulting reaction product is treated and/or contacted with at least one nucleophilic agent selected from the group consisting of hydrogen sulfites (bisulfites, HSO₃⁻), sulfites (SO₃² ), hydrogen thiosulfates (HS₂O₃⁻), thiosulfates (S₂O₃² ), hydrogen phosphites (H₂PO₃⁻), phosphites (HPO₃²⁻) and phosphines as well as combinations thereof, more preferably with hydrogen sulfites (bisulfites, HSO₃⁻), in particular for a preferably selective derivatization of halide-based impurities, namely of MCPD fatty acid esters (fatty acid esters of monochloropropanediols) and glycidol esters of fatty acids;
   (ii) the at least one esterification step is run and/or performed at temperatures not exceeding 180 °C, especially not exceeding 150 °C, particularly not exceeding 120 °C, preferably not exceeding 100 °C, more preferably not exceeding 80 °C, even more preferably not exceeding 50 °C;
   (iii) the 1,2,3-propantriol of formula (I) or its protected form, preferably solketal (isopropylidene glycerol), used as starting material is free of any chlorinated species and comprises a total chlorinated species content of at most 1 ppm, especially at most 0.5 ppm, preferably at most 0.1 ppm, based on the 1,2,3-propantriol of formula (I) or its protected form, preferably solketal (isopropylidene glycerol);
   (iv) the at least one esterification step is run and/or performed in the absence of any metal-based catalyst,
wherein the process is performed such and/or feature and/or measurement (i), optionally in further combination with feature and/or measurement (ii) and/or feature and/or measurement (iii) and/or feature and/or measurement (iv), is applied and/or performed with the proviso that the reaction product, especially the one or more triglycerides of C₅-C₁₂-fatty acids, especially one or more triglycerides of C₆-C₁₂-fatty acids, of above general formula (II), is/are characterized by and/or exhibit(s) the following properties (1) to (5) in their combination and/or fulfil(s) the following product specification:
   (1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
   (2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
   (3) triglyceride content, especially determined according to Ph. Eur. 2.2.28 (European Pharmacopoeia 10.0): ≥ 99 %, especially ≥ 99.2 %, preferably ≥ 99.5 %, each based on the reaction product; and
   (4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the reaction product; and
   (5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.5 ppm, especially ≤ 0.2 ppm, preferably ≤ 0.1 ppm, each based on the reaction product.

According to a further particular embodiment of the present invention, the inventive process is performed such and/or feature and/or measurement (i), optionally in further combination with feature and/or measurement (ii) and/or feature and/or measurement (iii) and/or feature and/or measurement (iv), is applied and/or performed with the proviso that the reaction product, especially the one or more triglycerides of C₅-C₁₂-fatty acids, especially one or more triglycerides of C₆-C₁₂-fatty acids, of above general formula (II), is/are characterized by and/or exhibit(s) the following properties (1) to (5) in their combination and/or fulfil(s) the following product specification:
(1) acid value (AV), especially determined according to Ph. Eur. 2.5.1 (European Pharmacopoeia 10.0): ≤ 0.1 mg KOH / g; and
(2) hydroxyl value (OHV), especially determined according to Ph. Eur. 2.5.3 (European Pharmacopoeia 10.0): ≤ 0.1 mg KOH / g; and
(3) triglyceride content, especially determined according to Ph. Eur. 2.2.28 (European Pharmacopoeia 10.0): ≥ 99.5 %, especially ≥ 99.6 %, preferably ≥ 99.8 %, each based on the reaction product; and
(4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.1 ppm based on the reaction product; and
(5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.1 ppm based on the reaction product.

Such reaction product, especially the one or more triglycerides of C₅-C₁₂-fatty acids, especially one or more triglycerides of C₆-C₁₂-fatty acids, of above general formula (II), is particularly appropriate for applications requiring high purity grades and at the same time high active ingredient levels, e.g. for nutritional and above all pharmaceutical applications and uses (e.g. even for parenteral applications in high dosages and/or with high concentrations).

As stated hereinabove, the inventive process comprises at least one esterification step, especially an esterification step using C₅-C₁₂-fatty acids, especially C₆-C₁₂-fatty acids, and/or their anhydrides and/or esters (preferably C₁-C₄-alkyl esters).

According to a particular embodiment of the inventive process, the C₅-C₁₂-fatty acids, especially C₆-C₁₂-fatty acids, and/or their anhydrides and/or esters (preferably C₁-C₄-alkyl esters) used in the at least one esterification step are represented by general formula (III)

R⁴-C(O)OH (III)

wherein in general formula (III) the radical R⁴ represents a linear or branched, saturated or unsaturated aliphatic C₄-C₁₁-alkyl radical, especially C₅-C₁₁-alkyl radical, preferably a linear saturated aliphatic C₄-C₁₁-alkyl radical, especially C₅-C₁₁-alkyl radical, including their anhydrides and esters (preferably C₁-C₄-alkyl esters) as well as combinations thereof.

According to a further particular embodiment of the inventive process, the C₅-C₁₂-fatty acids, especially C₆-C₁₂-fatty acids, and/or their anhydrides and/or esters (preferably C₁-C₄-alkyl esters) used in the at least one esterification are linear saturated C₅-C₁₂-fatty acids, especially linear saturated C₆-C₁₂-fatty acids, and/or their anhydrides and/or esters (preferably C₁-C₄-alkyl esters).

According to yet another further particular embodiment of the inventive process, the C₅-C₁₂-fatty acids, especially C₆-C₁₂-fatty acids, and/or their anhydrides and/or esters (preferably C₁-C₄-alkyl esters) used in the at least one esterification step are selected from the group consisting of valeric acid (pentanoic acid), caproic acid (hexanoic acid), enanthic acid (heptanoic acid), caprylic acid (octanoic acid), pelargonic acid (nonanoic acid), capric acid (decanoic acid), undecylic acid (undecanoic acid), lauric acid (dodecanoic acid) and/or their anhydrides and/or esters (preferably C₁-C₄-alkyl esters), especially caproic acid (hexanoic acid), enanthic acid (heptanoic acid), caprylic acid (octanoic acid), pelargonic acid (nonanoic acid), capric acid (decanoic acid), undecylic acid (undecanoic acid), lauric acid (dodecanoic acid) and/or their anhydrides and/or esters (preferably C₁-C₄-alkyl esters), preferably caproic acid (hexanoic acid), enanthic acid (heptanoic acid), caprylic acid (octanoic acid), capric acid (decanoic acid), lauric acid (dodecanoic acid) and/or their anhydrides and/or esters (preferably C₁-C₄-alkyl esters), more preferably caproic acid (hexanoic acid), enanthic acid (heptanoic acid), caprylic acid (octanoic acid), capric acid (decanoic acid) and/or their anhydrides and/or esters (preferably C₁-C₄-alkyl esters).

As already delineated hereinabove, according to the inventive process, there is provided and/or produced, as a reaction product, one or more triglycerides of C₅-C₁₂-fatty acids, especially one or more triglycerides of C₆-C₁₂-fatty acids, of general formula (II)

CH₂[O -C(O)R¹] -CH[O -C(O)R²] -CH₂[O-C(O)R³] (II)

wherein in general formula (II) the radicals R', R² and R³, independently of one another, each represent a linear or branched, saturated or unsaturated aliphatic C₄-C₁₁-alkyl radical, especially C₅-C₁₁-alkyl radical, preferably a linear saturated aliphatic C₄-C₁₁-alkyl radical, especially C₅-C₁₁-alkyl radical.

In this respect, according to a particular embodiment of the inventive process, it is envisaged that the reaction product is one or more triglycerides of C₅-C₁₂-fatty acids, especially one or more triglycerides of C₆-C₁₂-fatty acids, of above-defined general formula (II), however, with the proviso that all radicals R', R² and R³ are identical.

According to a further particular embodiment of the inventive process, it is envisaged
that the reaction product is triheptanoin (1,3-di(heptanoyloxy)propane-2-yl heptanoate); and/or
that in above general formula (II) the radicals R', R² and R³ each represent a linear saturated aliphatic C₆-alkyl radical; and/or
that in the at least one esterification step heptanoic acid (enanthic acid) and/or its anhydride and/or esters (preferably C₁-C₄-alkyl esters) is used.

Furthermore, according to a yet another particular embodiment of the inventive process, it is envisaged
that the reaction product is tricaprylin; and/or
that in above general formula (II) the radicals R', R² and R³ each represent a linear saturated aliphatic C₇-alkyl radical; and/or
that in the at least one esterification step octanoic acid (caprylic acid) and/or its anhydride and/or esters (preferably C₁-C₄-alkyl esters) is used.

Moreover, according to an alternative embodiment of the inventive process, it is envisaged that the reaction product is not tricaprylin.

Further, according to yet another alternative embodiment of the inventive process, it is envisaged that the reaction product is one or more triglycerides of C₅-C₁₂-fatty acids, especially one or more triglycerides of C₆-C₁₂-fatty acids, of above-defined general formula (II), however, with the proviso that at least two of the radicals R', R² and R³ are different from one another.

Finally, according to yet another alternative embodiment of the inventive process, it is envisaged that the reaction product is one or more triglycerides of C₅-C₁₂-fatty acids, especially one or more triglycerides of C₆-C₁₂-fatty acids, of above-defined general formula (II), however, with the proviso that all radicals R', R² and R³ are different from one another.

This shows that, according to the inventive process, a multitude of different triglycerides of fatty acids (i.e. fatty acid glycerol triesters) can be produced.

As already delineated hereinabove, according to the present invention, the inventive process is characterized by feature and/or measurement (i), optionally in further combination with feature and/or measurement (ii) and/or feature and/or measurement (iii) and/or feature and/or measurement (iv).

**In** this respect, according to a particular embodiment of the inventive process, it is envisaged that the inventive process is characterized by a combination of at least two, preferably by a combination of at least three, more preferably by a combination of all four, of the aforedefined features and/or measurements (i) to (iv).

According to another particular embodiment of the inventive process, it is envisaged that the inventive process is characterized by a combination of at least two, preferably by a combination of at least three, of the aforedefined features and/or measurements (ii) to (iv), namely in further combination with aforedefined feature and/or measurement (i).

Furthermore, according to yet another particular embodiment of the inventive process, it is envisaged that the inventive process is characterized by a combination of aforedefined feature and/or measurement (i) with at least two of the aforedefined features and/or measurements (ii) to (iv).

Finally, according to a further particular embodiment of the inventive process, it is envisaged that the inventive process is characterized by a combination of all of aforedefined features and/or measurements (i) to (iv).

As already delineated hereinabove, according to the present invention, when applying aforedefined feature and/or measurement (i), optionally in further combination with feature and/or measurement (ii) and/or feature and/or measurement (iii) and/or feature and/or measurement (iv), triglycerides of fatty acids (i.e. fatty acid glycerol triesters), especially with a high purity degree, are obtained in accordance with the above-referenced product specification.

According to a particular embodiment of the inventive process, it is particularly preferred that the whole and/or overall process, especially also including optional pre-treatment and/or post-treatment steps if any, is run and/or performed at temperatures not exceeding 180 °C, especially not exceeding 160 °C, particularly not exceeding 150 °C, preferably not exceeding 120 °C.

In other words, according to this particular embodiment of the inventive process, it is particularly preferred that the process is characterized by further feature and/or measurement (v):
(v) the whole and/or overall process, especially also including optional pre-treatment and/or post-treatment steps if any, is run and/or performed at temperatures not exceeding 180 °C, especially not exceeding 160 °C, particularly not exceeding 150 °C, preferably not exceeding 120 °C.

By this measurement (v), the formation of toxic by-products, such as e.g. MCPD or glycidol fatty acid esters can be efficiently prevented since such formation usually occurs at temperatures higher than these indicated values.

According to another particular embodiment of the inventive process, it is particularly preferred that the whole and/or overall process, especially also including optional pre-treatment and/or post-treatment steps if any, is run and/or performed in the absence of any metal-based catalyst.

In other words, according to this particular embodiment of the inventive process, it is particularly preferred that the process is characterized by further feature and/or measurement (vi):
(vi) the whole and/or overall process, especially also including optional pre-treatment and/or post-treatment steps if any, is run and/or performed in the absence of any metal-based catalyst.

Performing the whole and/or overall process, especially also including optional pre-treatment and/or post-treatment steps if any, in the absence of any metal-based catalyst is also a key issue in the inventive process since such metal-based catalysts often introduce or entrain significant amounts of undesired halides, especially chlorides. The thus introduced or entrained halides, especially chlorides, may then lead to the undesired formation of toxic side-products, especially MCPD and glycidol fatty acid esters, during esterification. By avoiding such metal-based catalyst over the whole and/or overall process, especially also including optional pre-treatment and/or post-treatment steps if any, the uncontrolled and undesired introduction or entrainment of halide-based impurities is thus efficiently avoided or prevented.

As already delineated hereinabove, the process of the present invention comprises at least one esterification step.

In general, the at least one esterification step may be performed and/or carried out as a one-stage esterification or, alternatively, as a two-stage or multistage esterification.

According to a particular embodiment of the inventive process, the at least one esterification step may be performed and/or carried out as a one-stage esterification (i.e. using anhydrides of C₅-C₁₂-fatty acids to be reacted with 1,2,3-propantriol of formula (I) or its protected form or precursor, preferably solketal). Alternatively, according to another particular embodiment of the inventive process, the at least one esterification step may also be performed and/or carried out as a two-stage or multistage esterification (i.e. in a first stage using C₅-C₁₂-fatty acids and/or their esters to be reacted with the 1,2,3-propantriol of formula (I) or its protected form or precursor, preferably solketal, followed in a subsequent stage by further esterification, especially further esterification of free and/or remaining OH-groups, preferably using anhydrides of C₅-C₁₂-fatty acids).

In general, according to a particular embodiment of the inventive process, it is preferred when the at least one esterification step is performed and/or carried out in the absence of solvents and/or without any solvent. This means that the at least one esterification step is carried out as a reaction/esterification in mass or as a reaction/esterification in substance or as a so-called *bulk reaction* (i.e. *bulk esterification).* This has the advantage that the reaction products obtained are not contaminated with any solvent and that no solvent has to be removed and disposed of or recycled in a costly and energy-intensive manner after the at least one esterification step has been carried out. Surprisingly, the at least one esterification step nevertheless proceeds with high conversion and yields and at least essentially without any significant by-product formation. In addition, avoiding the use of any solvents during the at least one esterification step also facilitates the recycling of the esterification catalyst if used and above all ensures that the recycling of the esterification catalyst is possible without its reactivation since solvents might otherwise deactivate or even poison such esterification catalyst.

The temperature ranges to be applied for the at least one esterification step may vary over a wide range. Usually, according to the present invention, it is preferred when the at least one esterification step is performed and/or carried out at moderate temperatures, i.e. at temperatures in the range of from 5 °C to 95 °C, especially in the range of from 10 °C to 85 °C, preferentially in the range of from 15 °C to 80 °C, more preferably in the range of from 20 °C to 75 °C, even more preferably in the range of from 25 °C to 70 °C. This moderate temperature regime applied for the at least one esterification step efficiently prevents the formation of particularly toxic by-products and ensures an efficient esterification. However, the skilled person may, if necessary or in a single or exceptional case, deviate from these range specifications without leaving the scope of the present invention.

Also the pressure ranges to be applied for the at least one esterification step may vary over a wide range. **In** general, according to the present invention, it is preferred when the at least one esterification step may be performed and/or carried out at a pressure in the range of from 0.0001 bar to 10 bar, especially in the range of from 0.001 bar to 5 bar, preferentially in the range of from 0.01 bar to 2 bar, more preferably in the range of from 0.05 bar to 1 bar, even more preferably at about 1 bar. This pressure regime applied for the at least one esterification step ensures an efficient esterification. However, the skilled person may, if necessary or in a single or exceptional case, deviate from these range specifications without leaving the scope of the present invention.

Basically, according to the present invention, it is possible that either the at least one esterification step is performed and/or carried out in the absence of any catalyst or, alternatively, that the at least one esterification step is performed and/or carried out at least partially in the presence of an enzyme as a catalyst and/or at least partially in the presence of an enzyme-based catalyst. **In** case that an enzyme as a catalyst and/or an enzyme-based catalyst is used, it is preferred that the catalyst is recycled after the at least one esterification step; this increases efficiency and process economy, especially when the inventive process is performed on a large-scale or industrial level.

In the present description, the wording or formulation "enzyme (used) as a catalyst" and "enzyme-based catalyst " are utilized as synonyms.

In this respect, according to a particular embodiment of the inventive process, the at least one esterification step may be performed and/or carried out in the absence of any catalyst when using anhydrides of C₅-C₁₂-fatty acids to be reacted with 1,2,3-propantriol of formula (I) or its protected form or precursor, preferably solketal. Alternatively, according to another and alternative particular embodiment of the inventive process, the at least one esterification step may also be performed and/or carried out at least partially in the presence of an enzyme as a catalyst and/or at least partially in the presence of an enzyme-based catalyst when using C₅-C₁₂-fatty acids and/or their esters to be reacted with the 1,2,3-propantriol of formula (I) or its protected form or precursor, preferably solketal; as delineated hereinbefore, in case that an enzyme as a catalyst and/or an enzyme-based catalyst is used, it is preferred that the catalyst is recycled after the at least one esterification step.

As already delineated hereinabove, according to a particular embodiment of the present invention, the at least one esterification step may be performed at least partially in the presence of an enzyme as a catalyst and/or at least partially in the presence of an enzyme-based catalyst (preferably in the absence of solvents and/or without any solvent as also indicated hereinabove). This ensures a high esterification efficiency, especially high conversion rates and yields with excellent esterification degrees and low acid and hydroxyl values at mild esterification conditions.

**In** general, when the at least one esterification step is performed at least partially in the presence of an enzyme as a catalyst and/or at least partially in the presence of an enzyme-based catalyst, the enzyme may especially be selected from synthetases (ligases), catalases, esterases, lipases and combinations thereof.

Especially, the enzyme may be derived from Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar and Pseudomonas sp. and combinations thereof, preferably from Candida antarctica, Mucor miehei (Rhizomucor miehei) and Thermomyces lanuginosus, more preferably from Candida Antarctica. These species lead to particularly good esterification results.

Furthermore, the enzyme may especially be used in immobilized form, especially immobilized on a carrier, preferentially on a polymeric carrier, preferably on a polymeric organic carrier, more preferably with hydrophobic properties, even more preferably on a poly(meth)acrylic resin-based carrier. This significantly improves handling and use as well as recycling of the enzyme as a catalyst.

Especially, as already delineated hereinabove, for the above reasons it is preferred when the enzyme is recycled after the at least one esterification step.

In general, when the at least one esterification step is performed at least partially in the presence of an enzyme as a catalyst and/or at least partially in the presence of an enzyme-based catalyst, the temperature ranges to be applied for the at least one esterification step may vary over a wide range. Especially, in such case, the at least one esterification step may be performed and/or carried out at temperatures in the range of from 5 °C to 95 °C, especially in the range of from 10 °C to 85 °C, preferentially in the range of from 15 °C to 80 °C, more preferably in the range of from 20 °C to 75 °C, even more preferably in the range of from 25 °C to 70 °C. However, the skilled person may, if necessary or in a single or exceptional case, deviate from these range specifications without leaving the scope of the present invention.

Also, when the at least one esterification step is performed at least partially in the presence of an enzyme as a catalyst and/or at least partially in the presence of an enzyme-based catalyst, the enzyme amounts to be applied for the at least one esterification step may vary over a wide range. Especially, in such case, the enzyme is used in amounts, based on the total amount of all starting compounds, in the range of from 0.0001 to 25 % by weight, especially in the range of from 0.001 to 20 % by weight, preferentially in the range of from 0.01 to 15 % by weight, preferably in the range of from 0.05 to 10 % by weight. However, the skilled person may, if necessary or in a single or exceptional case, deviate from these range specifications without leaving the scope of the present invention.

Especially, according to a particular embodiment of the present invention, the enzyme may be used in amounts, based on the total amount of all starting compounds, in the range of from 1 • 10⁻⁶ to 5 mol-%, especially in the range of from 1 • 10⁻⁵ to 2.5 mol-%, preferentially in the range of from 1 • 10⁴ to 1.5 mol-%, preferably in the range of from 1 • 10⁻³ to 1 mol-%. However, the skilled person may, if necessary or in a single or exceptional case, deviate from these range specifications without leaving the scope of the present invention.

Moreover, when the at least one esterification step is performed at least partially in the presence of an enzyme as a catalyst and/or at least partially in the presence of an enzyme-based catalyst, also the pressure ranges to be applied for the at least one esterification step may vary over a wide range. Especially, in such case, the at least one esterification step may be performed and/or carried out at a pressure in the range of from 0.0001 bar to 10 bar, especially in the range of from 0.001 bar to 5 bar, preferentially in the range of from 0.01 bar to 2 bar, more preferably in the range of from 0.05 bar to 1 bar, even more preferably at about 1 bar. However, the skilled person may, if necessary or in a single or exceptional case, deviate from these range specifications without leaving the scope of the present invention.

As also already delineated hereinabove, the present invention is based on a universal concept / process for producing high-purity triglycerides of fatty acids with high yields and high conversion rates, which concept / process is independent from any specific synthesis route. Thus, applicant's concept is universal to be applied for this purpose.

Consequently, a multitude of synthesis routes may be applied within the scope of the present invention, which possible synthesis routes will be explained in more detail hereinafter.

According to a particular embodiment of the present invention, the reaction product obtained or obtainable from the process of the present invention may be produced e.g. via one of the following synthesis routes (A) to (F):
(A) According to a (first) Synthesis Route (A), 1,2,3-propantriol (glycerol, glycerine) of formula (I)

   CH₂(OH) - CH(OH) - CH₂(OH) (I)

   is first reacted, preferably in the presence of an enzyme as a catalyst and/or in the presence of an enzyme-based catalyst (especially as defined hereinabove), with at least one linear or branched, saturated or unsaturated aliphatic C₅-C₁₂-fatty acid or its ester, especially with at least one linear or branched, saturated or unsaturated aliphatic C₆-C₁₂-fatty acid or its ester, preferably with at least one linear saturated aliphatic C₅-C₁₂-fatty acid or its ester, especially with at least one linear saturated aliphatic C₆-C₁₂-fatty acid or its ester, preferably so as to produce a diglyceride, more preferably a 1,3-diglyceride,
   followed by subsequent reaction, preferably in the absence of any catalyst, with at least one linear or branched, saturated or unsaturated aliphatic C₅-C₁₂-fatty acid anhydride, especially with at least one linear or branched, saturated or unsaturated aliphatic C₆-C₁₂-fatty acid anhydride, preferably with at least one linear saturated aliphatic C₅-C₁₂-fatty acid anhydride, especially with at least one linear saturated aliphatic C₆-C₁₂-fatty acid anhydride,
   so as to produce the reaction product of general formula (II) as defined hereinabove; or else
(B) according to a (second) Synthesis Route (B), 1,2,3-propantriol (glycerol, glycerine) of formula (I)

   CH₂(OH) - CH(OH) - CH₂(OH) (I)

   is first reacted, preferably in the presence of an enzyme as a catalyst and/or in the presence of an enzyme-based catalyst (especially as defined hereinabove), with at least one linear or branched, saturated or unsaturated aliphatic C₅-C₁₂-fatty acid or its ester, especially with at least one linear or branched, saturated or unsaturated aliphatic C₆-C₁₂-fatty acid or its ester, preferably with at least one linear saturated aliphatic C₅-C₁₂-fatty acid or its ester, especially with at least one linear saturated aliphatic C₆-C₁₂-fatty acid or its ester, preferably so as to produce a diglyceride, more preferably a 1,3-diglyceride,
   followed by subsequent reaction, preferably in the presence of an enzyme as a catalyst and/or in the presence of an enzyme-based catalyst (more preferably an enzyme for regioselective esterification of the 2-position of 1,3-diglyceride, such as e.g. *Pseudomonas fluorescens* lipase or *Burkholderia cepacia* lipase), with at least one linear or branched, saturated or unsaturated aliphatic C₅-C₁₂-fatty acid or its ester, especially with at least one linear or branched, saturated or unsaturated aliphatic C₆-C₁₂-fatty acid or its ester, preferably with at least one linear saturated aliphatic C₅-C₁₂-fatty acid or its ester, especially with at least one linear saturated aliphatic C₆-C₁₂-fatty acid or its ester,
   so as to produce the reaction product of general formula (II) as defined hereinabove; or else
(C) according to a (third) Synthesis Route (C), 1,2,3-propantriol (glycerol, glycerine) of formula (I)

   CH₂(OH) - CH(OH) - CH₂(OH) (I)

   is reacted, preferably in the absence of any catalyst, with at least one linear or branched, saturated or unsaturated aliphatic C₅-C₁₂-fatty acid anhydride, especially with at least one linear or branched, saturated or unsaturated aliphatic C₆-C₁₂-fatty acid anhydride, preferably with at least one linear saturated aliphatic C₅-C₁₂-fatty acid anhydride, especially with at least one linear saturated aliphatic C₆-C₁₂-fatty acid anhydride,
   so as to produce the reaction product of general formula (II) as defined hereinabove; or else
(D) according to a (fourth) Synthesis Route (D), a protected form or precursor of 1,2,3-propantriol of above formula (I), preferably solketal (isopropylidene glycerol), is reacted, preferably in the absence of any catalyst, with at least one linear or branched, saturated or unsaturated aliphatic C₅-C₁₂-fatty acid anhydride, especially with at least one linear or branched, saturated or unsaturated aliphatic C₆-C₁₂-fatty acid anhydride, preferably with at least one linear saturated aliphatic C₅-C₁₂-fatty acid anhydride, especially with at least one linear saturated aliphatic C₆-C₁₂-fatty acid anhydride,
   especially wherein a deprotection reaction, especially ring-opening (preferably acid-induced) and optionally removal of acetone, is performed after monoesterification of the free OH-group of the protected form or precursor of 1,2,3-propantriol of above formula (I), followed by further esterification with the at least one linear or branched, saturated or unsaturated aliphatic C₅-C₁₂-fatty acid anhydride, especially with the at least one linear or branched, saturated or unsaturated aliphatic C₆-C₁₂-fatty acid anhydride, preferably with the at least one linear saturated aliphatic C₅-C₁₂-fatty acid anhydride, especially with the at least one linear saturated aliphatic C₆-C₁₂-fatty acid anhydride,
   so as to produce the reaction product of general formula (II) as defined hereinabove; or else
(E) according to a (fifth) Synthesis Route (E), a protected form or precursor of 1,2,3-propantriol of above formula (I), preferably solketal (isopropylidene glycerol), is reacted, preferably in the presence of an enzyme as a catalyst and/or in the presence of an enzyme-based catalyst (especially as defined hereinabove), with at least one linear or branched, saturated or unsaturated aliphatic C₅-C₁₂-fatty acid or its ester, especially with at least one linear or branched, saturated or unsaturated aliphatic C₆-C₁₂-fatty acid or its ester, preferably with at least one linear saturated aliphatic C₅-C₁₂-fatty acid or its ester, especially with at least one linear saturated aliphatic C₆-C₁₂-fatty acid or its ester, followed by deprotection reaction, especially ring-opening (preferably acid-induced) and removal of acetone, preferably so as to produce a monoglyceride, more preferably a terminal monoglyceride and/or 1-monoglyceride,
   followed by subsequent reaction, preferably in the absence of any catalyst, with at least one linear or branched, saturated or unsaturated aliphatic C₅-C₁₂-fatty acid anhydride, especially with at least one linear or branched, saturated or unsaturated aliphatic C₆-C₁₂-fatty acid anhydride, preferably with at least one linear saturated aliphatic C₅-C₁₂-fatty acid anhydride, especially with at least one linear saturated aliphatic C₆-C₁₂-fatty acid anhydride,
   so as to produce the reaction product of general formula (II) as defined hereinabove; or else
(F) according to a (sixth) Synthesis Route (F), 1,2,3-propantriol (glycerol, glycerine) of formula (I)

   CH₂(OH) - CH(OH) - CH₂(OH) (I)

   is reacted, optionally in the presence of an enzyme as a catalyst and/or in the presence of an enzyme-based catalyst (especially as defined hereinabove), with at least one linear or branched, mono- or poly-unsaturated (preferably mono-unsaturated) aliphatic C₅-C₁₂-fatty acid and/or its esters and/or anhydrides, especially with at least one linear or branched, mono- or poly-unsaturated (preferably mono-unsaturated) aliphatic C₆-C₁₂-fatty acid and/or its esters and/or anhydrides, preferably with at least one linear mono- or poly-unsaturated (preferably mono-unsaturated) aliphatic C₅-C₁₂-fatty acid and/or its esters and/or anhydrides, especially with at least one linear mono- or poly-unsaturated (preferably mono-unsaturated) aliphatic C₆-C₁₂-fatty acid and/or its esters and/or anhydrides, preferably so as to produce triglycerides of mono- or poly-unsaturated (preferably mono-unsaturated) aliphatic C₅-C₁₂-fatty acids,
   followed by subsequent hydrogenation reaction, preferably in the presence of at least one hydrogenation catalyst, preferably so as to hydrogenate the double bonds of the mono- or poly-unsaturated (preferably mono-unsaturated) aliphatic C₅-C₁₂-fatty acid radicals of the triglycerides,
   so as to produce the reaction product of general formula (II) as defined hereinabove.

The above-described (first) Synthesis Route (A) may be illustrated, in a non-limiting way and for purposes of visualization only, by the following simplified reaction scheme, showing the production of the exemplary inventive reaction product tricaprylin.

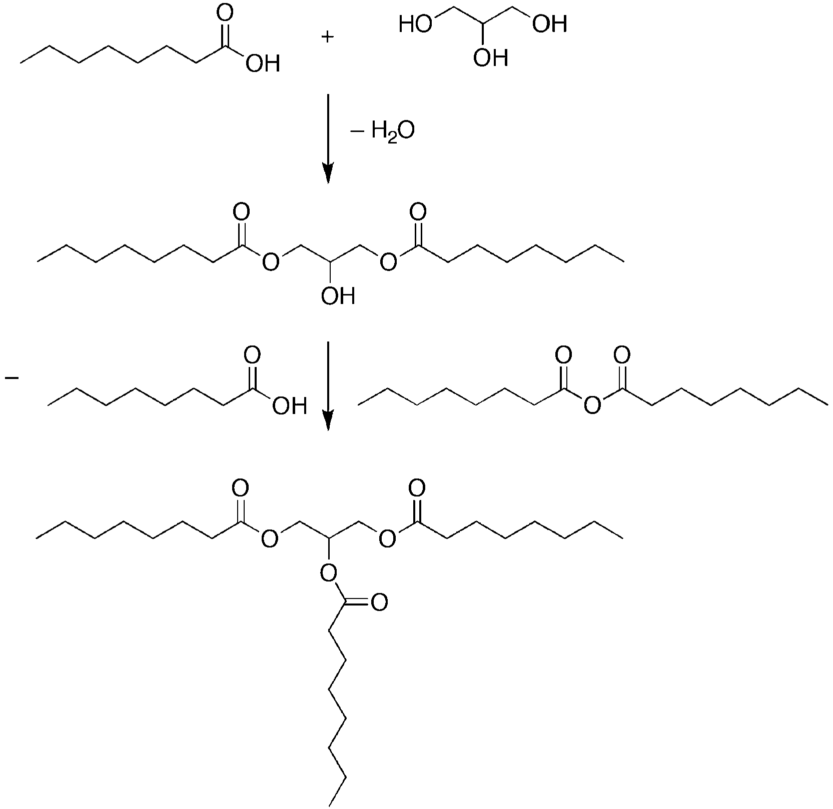

The above-described (second) Synthesis Route (B) may be illustrated, in a non-limiting way and for purposes of visualization only, by the following simplified reaction scheme, showing the production of the exemplary inventive reaction product tricaprylin.

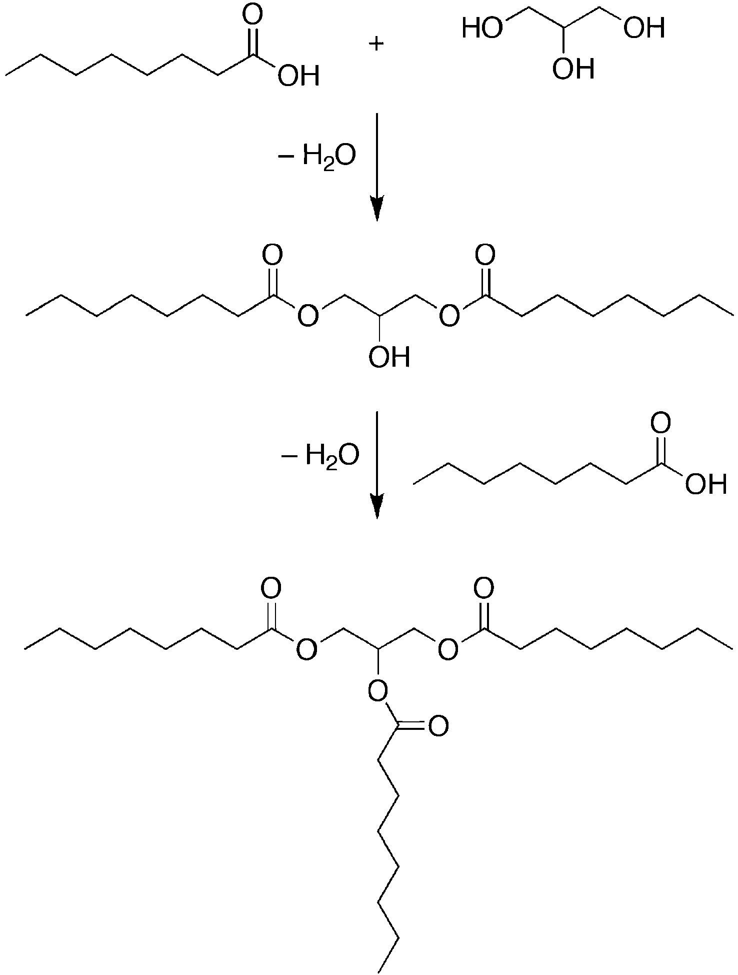

The above-described (third) Synthesis Route (C) may be illustrated, in a non-limiting way and for purposes of visualization only, by the following simplified reaction scheme, showing the production of the exemplary inventive reaction product tricaprylin.

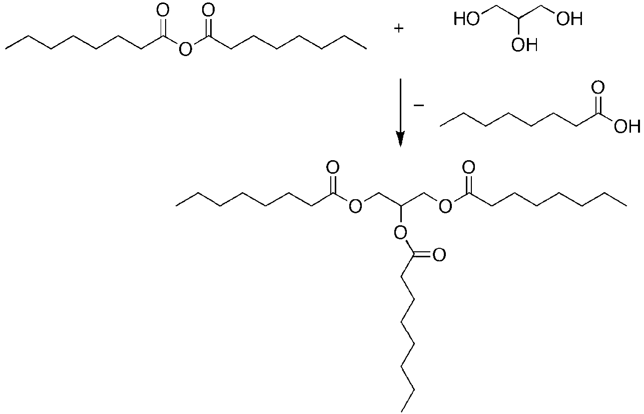

The above-described (fourth) Synthesis Route (D) may be illustrated, in a non-limiting way and for purposes of visualization only, by the following simplified reaction scheme, showing the production of the exemplary inventive reaction product tricaprylin.

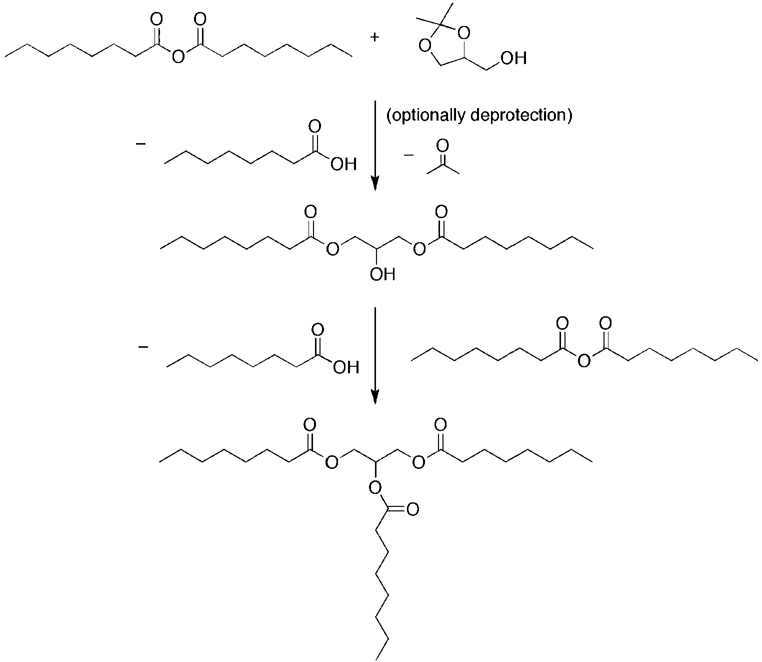

The above-described (fifth) Synthesis Route (E) may be illustrated, in a non-limiting way and for purposes of visualization only, by the following simplified reaction scheme, showing the production of the exemplary inventive reaction product tricaprylin.

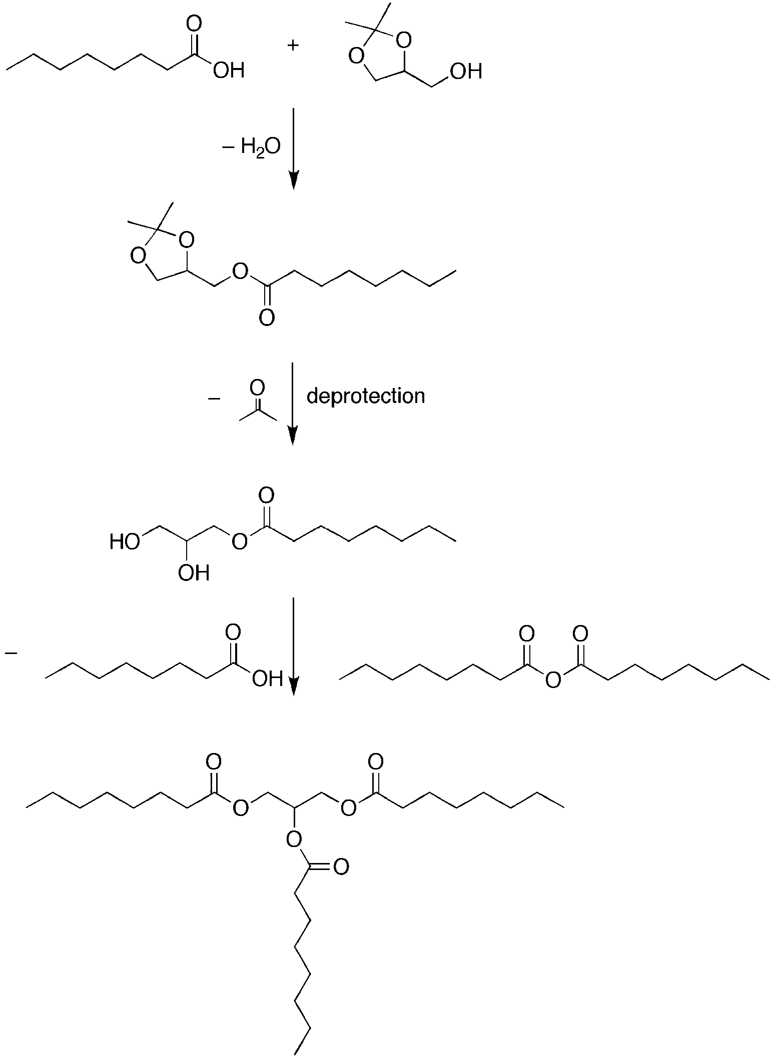

The above-described (sixth) Synthesis Route (F) may be illustrated, in a non-limiting way and for purposes of visualization only, by the following simplified reaction scheme, showing the production of the exemplary inventive reaction product tricaprylin.

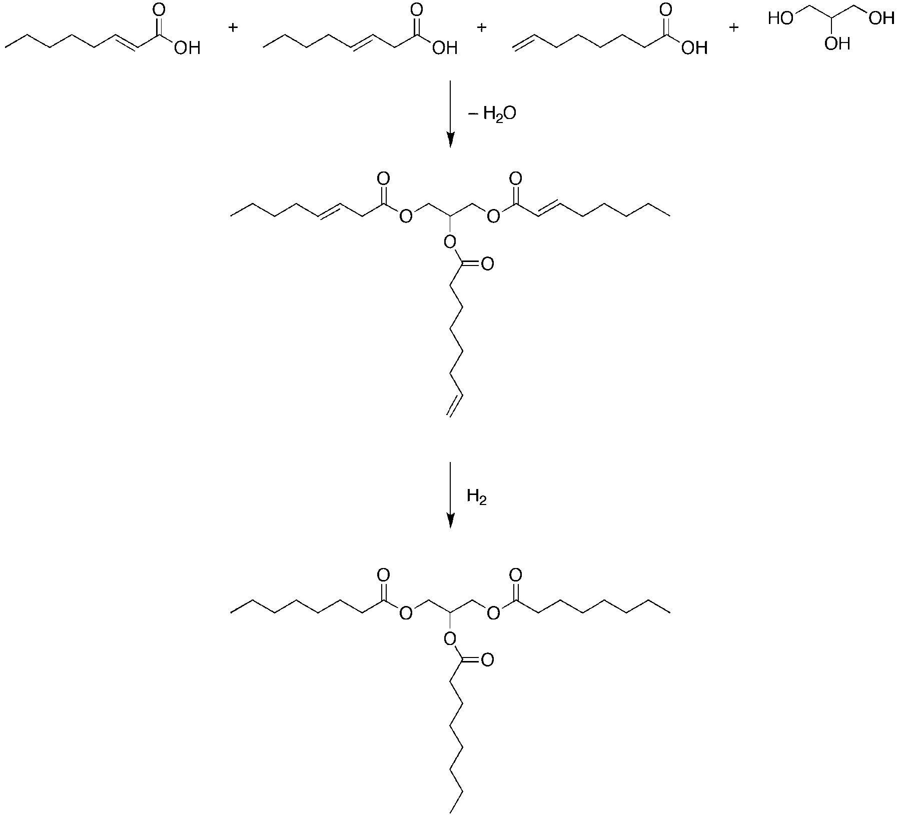

On the whole, applicant has been able to provide a universal concept and process, respectively, for producing high-purity triglycerides of fatty acids with high yields and high conversion rates, which concept / process is independent from any specific synthesis route. Thus, applicant's concept is universal to be applied for this purpose. The inventive process is at the same time highly flexible and compatible with known prior art production processes and plants for these substances since the aforenamed inventive features and/or measurements (i) to (iv) can be easily implemented in or used to modify known production processes and plants for these substances.

Above all, the inventive process produces triglycerides of fatty acids with a high purity degree, which at the same time unify a combination of at least five purity properties, namely: (1) a very low acid value (AV), (2) a very low hydroxyl value (OHV), (3) a very high triglyceride content, (4) a very low total content of MCPD fatty acid esters and (5) a very low total content of glycidol esters of fatty acids, wherein this purity specification can be reached independently from any specific synthesis route. Such combination of purity properties (1) to (5) was deemed incompatible or non-realizable so far; however, as applicant has surprisingly found out, the inventive process renders such combination of purity properties possible for the very first time.

In the present invention, there is also described the reaction product obtained or obtainable by the process according to the invention. In other words, according this aspect of the present invention, the present invention relates to a triglyceride of C₅-C₁₂-fatty acids, especially a triglyceride of C₆-C₁₂-fatty acids, obtainable or obtained by the process according to the present of the present invention as delineated and defined hereinabove.

Especially, in the present invention, there is described a triglyceride of C₅-C₁₂-fatty acids, especially a triglyceride of C₆-C₁₂-fatty acids, especially a triglyceride obtainable or obtained by the process according to the present of the present invention,
wherein the triglyceride of C₅-C₁₂-fatty acids, especially the triglyceride of C₆-C₁₂-fatty acids, corresponds to general formula (II)

   CH₂[O - C(O)R¹] - CH[O - C(O)R²] - CH₂[O - C(O)R³] (II)
wherein in general formula (II) the radicals R¹, R² and R³, independently of one another, each represent a linear or branched, saturated or unsaturated aliphatic C₄-C₁₁-alkyl radical, especially C₅-C₁₁-alkyl radical, preferably a linear saturated aliphatic C₄-C₁₁-alkyl radical, especially C₅-C₁₁-alkyl radical;
wherein the triglyceride of C₅-C₁₂-fatty acids, especially the triglyceride of C₆-C₁₂-fatty acids, of general formula (II) is characterized by and/or exhibits the following properties (1) to (5) in their combination and/or fulfils the following product specification:
   (1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
   (2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
   (3) triglyceride content, especially determined according to Ph. Eur. 2.2.28 (European Pharmacopoeia 10.0): ≥ 99 %, especially ≥ 99.2 %, preferably ≥ 99.5 %, each based on the triglyceride; and
   (4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the triglyceride; and
   (5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.5 ppm, especially ≤ 0.2 ppm, preferably ≤ 0.1 ppm, each based on the triglyceride.

Especially, according to the present invention, there is described a triglyceride of C₅-C₁₂-fatty acids, especially triglyceride of C₆-C₁₂-fatty acids, according to the above definition,
wherein the triglyceride of C₅-C₁₂-fatty acids, especially the triglyceride of C₆-C₁₂-fatty acids, of general formula (II) is characterized by and/or exhibits the following properties (1) to (5) in their combination and/or fulfils the following product specification:
(1) acid value (AV), especially determined according to Ph. Eur. 2.5.1 (European Pharmacopoeia 10.0): ≤ 0.1 mg KOH / g; and
(2) hydroxyl value (OHV), especially determined according to Ph. Eur. 2.5.3 (European Pharmacopoeia 10.0): ≤ 0.1 mg KOH / g; and
(3) triglyceride content, especially determined according to Ph. Eur. 2.2.28 (European Pharmacopoeia 10.0): ≥ 99.5 %, especially ≥ 99.6 %, preferably ≥ 99.8 %, each based on the triglyceride; and
(4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.1 ppm based on the triglyceride; and
(5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.1 ppm based on the triglyceride.

According to a particular embodiment of the present invention, there is also described a triglyceride of C₅-C₁₂-fatty acids, especially a triglyceride of C₆-C₁₂-fatty acids, according to the above definition, wherein, in general formula (II), all radicals R¹, R² and R³ are identical.

According to a further particular embodiment of the present invention, there is also described a triglyceride of C₅-C₁₂-fatty acids, especially a triglyceride of C₆-C₁₂-fatty acids, according to the above definition, wherein the triglyceride of C₅-C₁₂-fatty acids, especially the triglyceride of C₆-C₁₂-fatty acids, is triheptanoin (= 1,3-di(heptanoyloxy)propane-2-yl heptanoate).

According to yet another particular embodiment of the present invention, there is also described a triglyceride of C₅-C₁₂-fatty acids, especially a triglyceride of C₆-C₁₂-fatty acids, according to the above definition, wherein the triglyceride of C₅-C₁₂-fatty acids, especially the triglyceride of C₆-C₁₂-fatty acids, is tricaprylin.

Furthermore, according to yet another but alternative particular embodiment of the present invention, there is also described a triglyceride of C₅-C₁₂-fatty acids, especially a triglyceride of C₆-C₁₂-fatty acids, according to the above definition, wherein the triglyceride of C₅-C₁₂-fatty acids, especially the triglyceride of C₆-C₁₂-fatty acids, is not tricaprylin.

Moreover, according to yet another embodiment of the present invention, there is also described a triglyceride of C₅-C₁₂-fatty acids, especially a triglyceride of C₆-C₁₂-fatty acids, according to the above definition, wherein, in general formula (II), at least two of the radicals R¹, R² and R³ are different from one another (i.e. mixed triglyceride).

Also, according to yet another embodiment of the present invention, there is also described a triglyceride of C₅-C₁₂-fatty acids, especially a triglyceride of C₆-C₁₂-fatty acids, according to the above definition, wherein, in general formula (II), all radicals R¹, R² and R³ are different from one another (i.e. mixed triglyceride).

According to this inventive aspect, according to a particular embodiment of the present invention, there is also described a mixture of triglycerides, wherein the inventive mixture comprises at least two different triglycerides of C₅-C₁₂-fatty acids, especially at least two different triglycerides of C₆-C₁₂-fatty acids, as defined hereinabove.

In this respect, according to a particular embodiment of the present invention, the mixture may comprise e.g. two, three or more different triglycerides of C₅-C₁₂-fatty acids, especially triglycerides of C₆-C₁₂-fatty acids, as defined hereinabove.

Usually, the mixture is obtainable or obtained by the inventive process as defined hereinabove.

According to a particular embodiment of the present invention, the mixture may comprise triheptanoin (i.e. 1,3-di(heptanoyloxy)propane-2-yl heptanoate).

According to yet another particular embodiment of the present invention, the mixture may comprise tricaprylin.

According to yet another particular but alternative embodiment of the present invention, the mixture does not comprise tricaprylin.

Furthermore, according to another particular embodiment of the present invention, the mixture is characterized by:
a total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the mixture; and
a total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.5 ppm, especially ≤ 0.2 ppm, preferably ≤ 0.1 ppm, each based on the mixture.

Moreover, according to yet another particular embodiment of the present invention, the mixture is characterized by:
a total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.1 ppm based on the mixture; and
a total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.1 ppm based on the mixture.

Likewise, according to yet a further particular embodiment of the present invention, the mixture is usually characterized in that all triglyderides present in the mixture are characterized by and/or exhibit the following properties (1) to (5) in their combination and/or fulfil the following product specification:
(1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
(2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
(3) triglyceride content, especially determined according to Ph. Eur. 2.2.28 (European Pharmacopoeia 10.0): ≥ 99 %, especially ≥ 99.2 %, preferably ≥ 99.5 %, each based on the triglycerides; and
(4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the triglycerides; and
(5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.5 ppm, especially ≤ 0.2 ppm, preferably ≤ 0.1 ppm, each based on the triglycerides.

Finally, according to yet another particular embodiment of the present invention, the mixture is usually characterized in that all triglyderides present in the mixture are characterized by and/or exhibit the following properties (1) to (5) in their combination and/or fulfil the following product specification:
(1) acid value (AV), especially determined according to Ph. Eur. 2.5.1 (European Pharmacopoeia 10.0): ≤ 0.1 mg KOH / g; and
(2) hydroxyl value (OHV), especially determined according to Ph. Eur. 2.5.3 (European Pharmacopoeia 10.0): ≤ 0.1 mg KOH / g; and
(3) triglyceride content, especially determined according to Ph. Eur. 2.2.28 (European Pharmacopoeia 10.0): ≥ 99.5 %, especially ≥ 99.6 %, preferably ≥ 99.8 %, each based on the triglycerides; and
(4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.1 ppm based on the triglycerides; and
(5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.1 ppm based on the triglycerides.

The reaction products, i.e. the triglycerides of C₅-C₁₂-fatty acids, especially triglycerides of C₆-C₁₂-fatty acids, and their mixtures are linked to and unify a multitude of advantageous and particular properties and characteristics, which have already been delineated hereinabove in detail in context with the inventive process; thus, in order to avoid unnecessary repetitions, reference may be made to these above explanations.

Above all, the reaction products, i.e. the triglycerides of C₅-C₁₂-fatty acids, especially triglycerides of C₆-C₁₂-fatty acids, and their mixtures are particularly appropriate for applications requiring high purity grades and at the same time high active ingredient levels, e.g. for nutritional and above all also for pharmaceutical applications and uses (e.g. even for parenteral applications in high dosages and/or with high concentrations).

Furthermore, the reaction products, i.e. the triglycerides of C₅-C₁₂-fatty acids, especially triglycerides of C₆-C₁₂-fatty acids, and their mixtures unify the aforementioned five properties (1) to (5), which have been deemed so far incompatible with one another in their overall combination. For, according to prior art methods, when increasing the triglyceride content and lowering acid and hydroxyl values, usually high total contents of MCPD and glycidol fatty acid esters are effected due to drastic esterification conditions; in turn, when lowering the total contents of MCPD and glycidol fatty acid esters, according to the prior art, relatively low triglyceride contents with high acid and hydroxyl values are reached. In contrast to this prior art, the present invention renders it, for the first time, possible to provide a combination of all aforementioned five properties (1) to (5) in one and the same product. Consequently, the reaction products resulting from the inventive process are highly suitable for food and pharmaceutical applications, even in parenteral applications with high dosages and/or concentrations.

As to further details relating to this aspect of the invention, for avoidance of unnecessary repetition, reference may be made to the above explanations and remarks relating to the first aspect of the present invention (i.e. process of the present invention), which explanations and remarks accordingly apply *mutatis mutandis* also to this aspect of the present invention.

Likewise, the present invention also describes a pharmaceutical composition, especially a drug or medicament, comprising at least one triglyceride of C₅-C₁₂-fatty acids, especially at least one triglyceride of C₆-C₁₂-fatty acids, according to the above definition and/or a mixture according to the above definition, especially together with a physiologically acceptable excipient.

Especially, according to this aspect of the invention, there is described a pharmaceutical composition for the prophylactic and/or therapeutic treatment or for use in the prophylactic and/or therapeutic treatment of diseases of the human or animal body, especially diseases associated with a disorder of the energy metabolism, especially ketone body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus, migraine and migraine headaches, epilepsy, especially infantile spasms as well as effects or side-effects of chemotherapy.

In particular, according to this aspect of the invention, there is described a pharmaceutical composition for the prophylactic and/or therapeutic treatment or for use in the prophylactic and/or therapeutic treatment of diseases of the human body selected among Alzheimer's disease, especially mild to moderate forms of Alzheimer's disease; migraine and migraine headaches; as well as epilepsy, especially infantile spasms.

As to further details relating to this aspect of the invention, for avoidance of unnecessary repetition, reference may be made to the above explanations and remarks relating to the other aspects of the present invention, which explanations and remarks accordingly apply *mutatis mutandis* also to this aspect of the present invention.

Furthermore, there is also described a triglyceride of C₅-C₁₂-fatty acids, especially a triglyceride of C₆-C₁₂-fatty acids, according to the above definition and/or a mixture according to the above definition for the prophylactic and/or therapeutic treatment or for use in the prophylactic and/or therapeutic treatment of diseases of the human or animal body, especially diseases associated with a disorder of the energy metabolism, especially ketone body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus, migraine and migraine headaches, epilepsy, especially infantile spasms as well as effects or side-effects of chemotherapy.

Especially, according to this aspect of the invention, there is also described a triglyceride of C₅-C₁₂-fatty acids, especially a triglyceride of C₆-C₁₂-fatty acids, according to the above definition and/or a mixture according to the above definition for the prophylactic and/or therapeutic treatment or for use in the prophylactic and/or therapeutic treatment of diseases of the human body selected among Alzheimer's disease, especially mild to moderate forms of Alzheimer's disease; migraine and migraine headaches; as well as epilepsy, especially infantile spasms.

As to further details relating to this aspect of the invention, for avoidance of unnecessary repetition, reference may be made to the above explanations and remarks relating to other aspects of the present invention, which explanations and remarks accordingly apply *mutatis mutandis* also to this aspect of the present invention.

Further, there is also described a use of at least one triglyceride of C₅-C₁₂-fatty acids, especially at least one triglyceride of C₆-C₁₂-fatty acids, according to the above definition and/or of a mixture according to the above definition for the prophylactic and/or therapeutic treatment or for producing a medicament for the prophylactic and/or therapeutic treatment of diseases of the human or animal body, especially diseases associated with a disorder of the energy metabolism, especially ketone body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus, migraine and migraine headaches, epilepsy, especially infantile spasms as well as effects or side-effects of chemotherapy.

Especially, according to this aspect of the invention, there is also described a use of at least one triglyceride of C₅-C₁₂-fatty acids, especially at least one triglyceride of C₆-C₁₂-fatty acids, according to the above definition and/or of a mixture according to the above definition for the prophylactic and/or therapeutic treatment or for producing a medicament for the prophylactic and/or therapeutic treatment of diseases of the human body selected among Alzheimer's disease, especially mild to moderate forms of Alzheimer's disease; migraine and migraine headaches; as well as epilepsy, especially infantile spasms.

In particular, according to this aspect of the invention, there is also described a use of at least one triglyceride of C₅-C₁₂-fatty acids, especially at least one triglyceride of C₆-C₁₂-fatty acids, according to the above definition and/or of a mixture according to the above definition for the prophylactic and/or therapeutic treatment or for producing a medicament for the prophylactic and/or therapeutic treatment of or for the application for catabolic metabolic states, such as hunger, diets or low-carbohydrate nutrition.

As to further details relating to this aspect of the invention, for avoidance of unnecessary repetition, reference may be made to the above explanations and remarks relating to the other aspects of the present invention, which explanations and remarks accordingly apply *mutatis mutandis* also to this aspect of the present invention.

Furthermore, there is also described a food and/or a nutritive or food composition and/or a food product and/or a medical food comprising at least one triglyceride of C₅-C₁₂-fatty acids, especially at least one triglyceride of C₆-C₁₂-fatty acids, according to the above definition and/or a mixture according to the above definition.

Especially, according to this aspect of the invention, the food and/or the nutritive or food composition and/or the food product and/or the medical food may be e.g. a dietary supplement, a functional food, a novel food, a food additive, a food supplement, a dietary food, a power snack, an appetite suppressant or a strength and/or endurance sports supplement.

As to further details relating to this aspect of the invention, for avoidance of unnecessary repetition, reference may be made to the above explanations and remarks relating to the other aspects of the present invention, which explanations and remarks accordingly apply *mutatis mutandis* also to this sixth aspect of the present invention.

Likewise, there is also described a use of at least one triglyceride of C₅-C₁₂-fatty acids, especially at least one triglyceride of C₆-C₁₂-fatty acids, according to the above definition and/or of a mixture according to the above definition in a food and/or in a nutritive or food composition and/or in a food product and/or in a medical food.

Especially, according to this aspect of the invention, the food and/or the nutritive or food composition and/or the food product and/or the medical food may be e.g. a dietary supplement, a functional food, a novel food, a food additive, a food supplement, a dietary food, a power snack, an appetite suppressant or a strength and/or endurance sports supplement.

As to further details relating to this aspect of the invention, for avoidance of unnecessary repetition, reference may be made to the above explanations and remarks relating to the other aspects of the present invention, which explanations and remarks accordingly apply *mutatis mutandis* also to this aspect of the present invention.

Also, there is described a use of at least one triglyceride of C₅-C₁₂-fatty acids, especially at least one triglyceride of C₆-C₁₂-fatty acids, according to the above definition and/or of a mixture according to the above definition as an additive or aid, especially as a carrier or excipient, a solubilizer, a release agent, a surface-treatment agent, a transport agent, a lubricant, hydrophobic agent, a film-forming or protective agent or a viscosity regulator, preferably as a carrier or excipient, in pharmaceutical compositions, especially drugs or medicaments, in food, nutritive or food compositions, food products and/or medical foods as well as in cosmetic compositions.

Especially, according to this aspect of the invention, there is also described a use of at least one triglyceride of C₅-C₁₂-fatty acids, especially at least one triglyceride of C₆-C₁₂-fatty acids, according to the above definition and/or of a mixture according to the above definition as a carrier or excipient in pharmaceutical compositions, especially drugs or medicaments.

Furthermore, according to this aspect of the invention, there is also described a use of at least one triglyceride of C₅-C₁₂-fatty acids, especially at least one triglyceride of C₆-C₁₂-fatty acids, according to the above definition and/or of a mixture according to the above definition as an active substance (i.e. active ingredient) in pharmaceutical compositions, especially drugs or medicaments, in food, nutritive or food compositions, food products and/or medical foods or in cosmetic compositions, especially together with an excipient, particularly together with a physiologically acceptable excipient.

Moreover, according to this aspect of the invention, there is also described a use of at least one triglyceride of C₅-C₁₂-fatty acids, especially at least one triglyceride of C₆-C₁₂-fatty acids, according to the above definition and/or of a mixture according to the above definition as an active substance (i.e. active ingredient, API) in pharmaceutical compositions.

Further, according to this aspect of the invention, there is also described a use of at least one triglyceride of C₅-C₁₂-fatty acids, especially at least one triglyceride of C₆-C₁₂-fatty acids, according to the above definition and/or of a mixture according to the above definition as an active substance (i.e. active ingredient, API) in pharmaceutical compositions, especially drugs or medicaments, especially together with a physiologically acceptable excipient.

As to further details relating to this aspect of the invention, for avoidance of unnecessary repetition, reference may be made to the above explanations and remarks relating to the other aspects of the present invention, which explanations and remarks accordingly apply *mutatis mutandis* also to this aspect of the present invention.

Finally, there is also described a use of a nucleophilic agent for purifying triglycerides of fatty acids, particularly triglycerides of C₅-C₁₂-fatty acids, especially triglycerides of C₆-C₁₂-fatty acids, preferably of general formula (II) as defined hereinabove, and/or for a preferably selective removal or derivatization of halide-based impurities present in organic substances, especially in triglycerides of fatty acids, particularly in triglycerides of C₅-C₁₂-fatty acids, especially in triglycerides of C₆-C₁₂-fatty acids, preferably of general formula (II) as defined hereinabove.

Especially, according to this aspect of the invention, the nucleophilic agent may be e.g. a nucleophilic agent capable of removing halide-based impurities, preferably selected from the group consisting of hydrogen sulfites (bisulfites, HSO₃⁻), sulfites (SO₃²⁻), hydrogen thiosulfates (HS₂O₃⁻), thiosulfates (S₂O₃²⁻), hydrogen phosphites (H₂PO₃⁻), phosphites (HPO₃²⁻) and phosphines as well as combinations thereof, more preferably hydrogen sulfites (bisulfites, HSO₃⁻).

Usually, according to a particular embodiment according to this aspect of the invention, the triglycerides of fatty acids to be purified are treated and/or contacted with the nucleophilic agent.

Generally, it is preferred that the treating and/or contacting of the triglycerides of fatty acids to be purified with the nucleophilic agent is performed as a one-stage or single-step treatment. However, particularly in case of higher degrees of impurifications, it is also possible to perform the treating and/or contacting of the triglycerides of fatty acids to be purified with the nucleophilic agent repeatedly, especially in two or more cycles and/or as a multistage process. Nevertheless, it is preferred to perform the treating and/or contacting of the triglycerides of fatty acids to be purified with the nucleophilic agent in one stage (i.e. without any repeated treating and/or contacting); particularly in case of higher degrees of impurifications, it is also possible, as an alternative embodiment, to use higher amounts or concentrations of the nucleophilic agent instead of performing a repeated treating and/or contacting.

Especially, according to another particular embodiment according to this aspect of the invention, the nucleophilic agent is used for purifying triglycerides of fatty acids from halide-based, especially chlorine-based impurities and/or for removing halide-based, especially chlorine-based impurities from triglycerides of fatty acids, particularly chlorine-based impurities selected from the group consisting of MCPD fatty acid esters (fatty acid esters of monochloropropanediols), glycidol esters of fatty acids and combinations thereof.

In particular, according to yet another particular embodiment according to this aspect of the invention, the halide-based impurities are chlorine-based impurities, especially chlorine-based impurities selected from the group consisting of MCPD fatty acid esters (fatty acid esters of monochloropropanediols), glycidol esters of fatty acids and combinations thereof.

Particularly, according to a further particular embodiment according to this aspect of the invention, the triglycerides of fatty acids to be purified are usually treated and/or contacted with the nucleophilic agent, especially with a preferably water-based solution of the nucleophilic agent.

Especially, the triglycerides of fatty acids to be purified are treated and/or contacted with the nucleophilic agent under reaction conditions, especially at reaction temperatures and/or for reaction durations, sufficient to remove the impurities.

In particular, the triglycerides of fatty acids to be purified are treated and/or contacted with the nucleophilic agent at temperatures in the range of from 10 °C to 175 °C, preferably in the range of from 25 °C to 120 °C, more preferably in the range of from 50 °C to 100 °C, and/or for a duration in the range of from 0.001 to 50 hours, preferably in the range of from 0.01 to 30 hours, more preferably in the range of from 0.1 to 20 hours.

Thus, on the whole, nucleophilic agents, such as e.g. hydrogen sulfites (bisulfites, HSO₃⁻), sulfites (SO₃²⁻), hydrogen thiosulfates (HS₂O₃⁻), thiosulfates (S₂O₃²⁻), hydrogen phosphites (H₂PO₃⁻), phosphites (HPO₃²⁻) and phosphines as well as combinations thereof, are an excellent reactant agent for purifying triglycerides of fatty acids, particularly triglycerides of C₅-C₁₂-fatty acids, especially triglycerides of C₆-C₁₂-fatty acids, especially for removing halide-based impurities, especially chlorine-based impurities, from triglycerides of fatty acids, particularly chlorine-based impurities selected from the group consisting of MCPD fatty acid esters (fatty acid esters of monochloropropanediols), glycidol esters of fatty acids and combinations thereof (i.e. via selective derivatization of halide-based impurities).

As to further details relating to this aspect of the invention, for avoidance of unnecessary repetition, reference may be made to the above explanations and remarks relating to the other aspects of the present invention, which explanations and remarks accordingly apply *mutatis mutandis* also to this aspect of the present invention.

Further embodiments, modifications and variations of the present invention are readily recognizable or realizable by a person skilled in the art when reading the description, without leaving the scope of the present invention.

The present invention is illustrated by the following examples, which are not intended to limit the present invention in any way, but only to explain the exemplary and non-limiting implementation and configuration of the present invention.

### EXAMPLES:

### Examples of production

The process in accordance with the invention is illustrated by the following examples, wherein the different modes of synthesis have been applied, as will be described in detail hereinafter.

In all inventive Examples, the used starting materials 1,2,3-propantriol (glycerol, glycerine) and solketal (isopropylidene glycerol), respectively, are each at least essentially free of any chlorinated species, i.e. each comprise a total chlorinated species content of at most 0.1 ppm (based on the 1,2,3-propantriol or solketal, respectively). Furthermore, the total concentration of the sum of MCPD fatty acid esters and glycidol fatty acid esters is under the detection limit (≤ 0.1 ppm of total MCPD and glycidol esters in sum, based on glycerol or solketal, respectively).

Furthermore, in all inventive Examples, esterification is run at temperatures not exceeding 100 °C, preferably not exceeding 80 °C.

Also, in all inventive Examples, esterification is performed in the absence of any metal-based catalyst.

Finally, all inventive Examples include selective derivatization, i.e. chemical treatment with nucleophilic agents, in order to remove any traces of halide-based impurities, especially of MCPD fatty acid esters and glycidol fatty acid esters. In all inventive Examples, product purity requirements are met even without such treatment; however, such selective derivatization, i.e. chemical treatment with nucleophilic agents, allows even increase of purity grade since even present traces of these impurities are eliminated selectively.

### 1. Synthesis Route (A)

Tricaprylin is produced according to above-described Synthesis Route (A). For this purpose, the following procedure is applied:
Caprylic acid (i.e. saturated linear C₈-fatty acid or octanoic acid) is reacted with glycerol in the presence of an immobilized enzyme as a catalyst (CALB lipase on polymer support, derived from *Candida antarctica,* e. g. Novozym^{®} 435 from Sigma-Aldrich or Merck or Lipozym^{®} 435 from Strem Chemicals, Inc.; 1 % by weight, based on the reaction mixture) at 80 °C under vacuum (< 100 mbar). Caprylic acid is used in amounts of up to 300 mol-%, preferably of up to 100 mol-%, based on the OH-groups of the glycerol. Then the excess of caprylic acid is removed using a short-path-distillation (<< 180 °C, preferably 120 °C to 130 °C; << 0,1 mbar, preferably < 2 mbar). A dicaprylin, especially 1,3-dicaprylin (1,3-diglyceride of caprylic acid), is obtained.

Subsequently the dicaprylin is reacted with caprylic acid anhydride at temperatures below 180°C, preferably between 80 °C and 150 °C, until the hydroxyl value (OHV) is below 0.1 mg KOH / g. The resulting product is treated in a usual way to remove the caprylic acid formed as by-product (i.e. short-path distillation or the like).

The resulting tricaprylin product is analyzed as follows:
- acid value (AV) determined according to Ph. Eur. 2.5.1 (European Pharmacopoeia 10.0): ≤ 0.1 mg KOH / g,
- hydroxyl value (OHV) determined according to Ph. Eur. 2.5.3 (European Pharmacopoeia 10.0): ≤ 0.1 mg KOH / g,
- triglyceride content determined according to Ph. Eur. 2.2.28 (European Pharmacopoeia 10.0): ≥ 99.4 % based on the reaction product (i.e. tricaprylin),
- total content of MCPD fatty acid esters (fatty acid esters of monochloropropanediols) determined according to method of DGF C-VI 18 (10) (Einheitsmethode der Deutschen Gesellschaft für Fettwissenschaft [Standard method of German Society for Fat Science]): 0.14 ppm based on the reaction product (i.e. tricaprylin),
- total content of glycidol esters of fatty acids determined according to method of DGF C-VI 18 (10) (Einheitsmethode der Deutschen Gesellschaft für Fettwissenschaft e.V. [Standard method of German Society for Fat Science]): 0.12 ppm based on the reaction product (i.e. tricaprylin).

The tricaprylin product is then further subjected to a treatment with sodium hydrogen sulfite (NaHSO₃) in the form of a water-based solution (80 °C, 6 hours) as a nucleophilic agent (selective derivatization of halide-based impurities, i.e. MCPD fatty acid esters and glycidol fatty acid esters). After separating the water-based phase and drying the resulting tricaprylin product, the product quality as a whole is further increased since the triglyceride content is increased to values ≥ 99.5 % and the total content of MCPD fatty acid esters and that of glycidol esters of fatty acids is each below the detection limit (i.e. ≤ 0.1 ppm each). Optionally, the thus treated product may also be further treated with bleaching earth and/or activated carbon, optionally followed by deodorization (e.g. using pressurized steam). The resulting tricaprylin product, after treatment with sodium hydrogen sulfite (NaHSO₃) and optional further treatments, is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.5 % based on tricaprylin,
- total content of MCPD fatty acid esters (DGF C-VI 18 (10)): ≤ 0.1 ppm based on tricaprylin,
- total content of glycidol esters of fatty acids (DGF C-VI 18 (10)): ≤ 0.1 ppm based on tricaprylin.

Comparable results are reached when, instead of sodium hydrogen sulfite (NaHSO₃), also other nucleophilic agents are used (i.e. sodium hydrogen thiosulfate NaHS₂O₃, sodium hydrogen phosphite NaH₂PO₃ and phosphines). The obtained results are comparable.

The aforedescribed production procedure is repeated in an analogous manner, however, using different starting materials, namely the respective C₁-C₄-esters of caprylic acid. In each approach comparable results are obtained.

In an analogous manner, also triheptanoin, on the one hand, and a medium-chain triglyceride (MCT) on the basis of caproic acid (C₆) / caprylic acid (C₈) / capric acid (C₁₀), on the other hand, are produced each with comparable results (characterization after treatment with hydrogen sulfite both for triheptanoin and for MCT each: acid value: ≤ 0.1 mg KOH / g; hydroxyl value: ≤ 0.1 mg KOH / g; triglyceride content: ≥ 99.5 %; total content of MCPD fatty acid esters: ≤ 0.1 ppm; total content of glycidol esters: ≤ 0.1 ppm).

### Comparative Procedure (not of the invention)

In a non-inventive manner, Synthesis Route (A) for tricaprylin is performed with the following deviations: The glycerol used as starting material comprises a total chlorinated species content of about 30 ppm. The esterification catalyst used is a metal-based catalyst on the basis of tetrabutyl titanate (TBT). Esterification is performed in the temperature range between 190 °C and 230 °C.

The resulting tricaprylin is analyzed as follows: acid value (Ph. Eur. 2.5.1): > 0.6 mg KOH / g, hydroxyl value (Ph. Eur. 2.5.3): > 0.7 mg KOH / g, triglyceride content (Ph. Eur. 2.2.28): 97.5 % based on tricaprylin, total content of MCPD fatty acid esters (DGF C-VI 18 (10)): 0.9 ppm based on tricaprylin, total content of glycidol esters of fatty acids (DGF C-VI 18 (10)): 0.7 ppm based on tricaprylin.

Subsequent treatment with sodium hydrogen sulfite (NaHSO₃) in the form of a water-based solution (80 °C, 6 hours) can lower both the total content of MCPD fatty acid esters and total content of glycidol esters of fatty acids to values of about 0.4 ppm each, however, whereas not improving or influencing the parameters of acid value, hydroxyl value and triglyceride content.

### 2. Synthesis Route (B)

Tricaprylin is produced according to above-described Synthesis Route (B). For this purpose, the following procedure is applied:
Caprylic acid (i.e. saturated linear C₈-fatty acid or octanoic acid) is reacted with glycerol in the presence of an immobilized enzyme as a catalyst (CALB lipase on polymer support, derived from *Candida antarctica,* e. g. Novozym^{®} 435 from Sigma-Aldrich or Merck or Lipozym^{®} 435 from Strem Chemicals, Inc.; 1 % by weight, based on the reaction mixture) at 80 °C under vacuum (< 100 mbar). Caprylic acid is used in amounts of up to 300 mol-%, preferably of up to 100 mol-%, based on the OH-groups of the glycerol. Then the excess of caprylic acid is removed using a short-path-distillation (<< 180 °C, preferably 120 °C to 130 °C; << 0,1 mbar, preferably < 2 mbar). A dicaprylin, especially 1,3-dicaprylin (1,3-diglyceride of caprylic acid), is obtained.

Subsequently the dicaprylin is reacted with caprylic acid in the presence of an enzyme, especially an enzyme for regioselective esterification of the 2-position if 1,3-diglyceride (e.g. *Pseudomonas fluorescens* lipase in a first approach and, in an alternative second approach, *Burkholderia cepacia* lipase, each immobilized on chitosan) at 50 °C until the hydroxyl value (OHV) is below 0.1 mg KOH / g.

The resulting tricaprylin product is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.5 % based on tricaprylin,
- total content of MCPD fatty acid esters (DGF C-VI 18 (10)): 0.17 ppm based on tricaprylin,
- total content of glycidol esters of fatty acids (DGF C-VI 18 (10)): 0.19 ppm based on tricaprylin.

The tricaprylin product is then further subjected to a treatment with sodium hydrogen sulfite (NaHSO₃) in the form of a water-based solution (80 °C, 6 hours) as a nucleophilic agent (selective derivatization of halide-based impurities, i.e. MCPD fatty acid esters and glycidol fatty acid esters). After separating the water-based phase and drying the resulting tricaprylin product, the product quality as a whole is further increased since the total content of MCPD fatty acid esters and that of glycidol esters of fatty acids is each below the detection limit (i.e. ≤ 0.1 ppm each). Optionally, the thus treated product may also be further treated with bleaching earth and/or activated carbon, optionally followed by deodorization (e.g. using pressurized steam).

The resulting tricaprylin product, after treatment with sodium hydrogen sulfite (NaHSO₃) and optional further treatments, is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.5 % based on tricaprylin,
- total content of MCPD fatty acid esters (DGF C-VI 18 (10)): ≤ 0.1 ppm based on tricaprylin,
- total content of glycidol esters of fatty acids (DGF C-VI 18 (10)): ≤ 0.1 ppm based on tricaprylin.

The aforedescribed production procedure is repeated in an analogous manner, however, using different starting materials, namely the respective C₁-C₄-esters of caprylic acid. In each approach comparable results are obtained.

In an analogous manner, also triheptanoin, on the one hand, and a medium-chain triglyceride (MCT) on the basis of caproic acid (C₆) / caprylic acid (C₈) / capric acid (C₁₀), on the other hand, are produced each with comparable results (characterization after treatment with hydrogen sulfite both for triheptanoin and for MCT each: acid value: ≤ 0.1 mg KOH / g; hydroxyl value: ≤ 0.1 mg KOH / g; triglyceride content: ≥ 99.5 %; total content of MCPD fatty acid esters: ≤ 0.1 ppm; total content of glycidol esters: ≤ 0.1 ppm).

### 3. Synthesis Route (C)

Tricaprylin is produced according to above-described Synthesis Route (C). For this purpose, the following procedure is applied:
Caprylic acid anhydride (i.e. linear saturated C₈-fatty acid anhydride or octanoic acid anhydride) is reacted with glycerol below 180 °C, preferably between 80 °C and 150 °C, until the hydroxyl value (OHV) is below 0.1 mg KOH / g. Then, the formed caprylic acid (by-product) is removed using a short-path-distillation (<< 180 °C, preferably 120 °C to 130 °C; << 0,1 mbar, preferably < 2 mbar).

The resulting tricaprylin product is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.5 % based on tricaprylin,
- total content of MCPD fatty acid esters (DGF C-VI 18 (10)): 0.16 ppm based on tricaprylin,
- total content of glycidol esters of fatty acids (DGF C-VI 18 (10)): 0.12 ppm based on tricaprylin.

The tricaprylin product is then further subjected to a treatment with sodium hydrogen sulfite (NaHSO₃) in the form of a water-based solution (80 °C, 6 hours) as a nucleophilic agent (selective derivatization of halide-based impurities, i.e. MCPD fatty acid esters and glycidol fatty acid esters). After separating the water-based phase and drying the resulting tricaprylin product, the product quality as a whole is further increased since the total content of MCPD fatty acid esters and that of glycidol esters of fatty acids is each below the detection limit (i.e. ≤ 0.1 ppm each). Optionally, the thus treated product may also be further treated with bleaching earth and/or activated carbon, optionally followed by deodorization (e.g. using pressurized steam).

The resulting tricaprylin product, after treatment with sodium hydrogen sulfite (NaHSO₃) and optional further treatments, is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.5 % based on tricaprylin,
- total content of MCPD fatty acid esters (DGF C-VI 18 (10)): ≤ 0.1 ppm based on tricaprylin,
- total content of glycidol esters of fatty acids (DGF C-VI 18 (10)): ≤ 0.1 ppm based on tricaprylin.

In an analogous manner, also triheptanoin, on the one hand, and a medium-chain triglyceride (MCT) on the basis of caproic acid (C₆) / caprylic acid (C₈) / capric acid (C₁₀), on the other hand, are produced each with comparable results (characterization after treatment with hydrogen sulfite both for triheptanoin and for MCT each: acid value: ≤ 0.1 mg KOH / g; hydroxyl value: ≤ 0.1 mg KOH / g; triglyceride content: ≥ 99.5 %; total content of MCPD fatty acid esters: ≤ 0.1 ppm; total content of glycidol esters: ≤ 0.1 ppm).

### 4. Synthesis Route (D)

Tricaprylin is produced according to above-described Synthesis Route (D). For this purpose, the following procedure is applied:
Solketal and caprylic acid anhydride (i.e. linear saturated C₈-fatty acid anhydride or octanoic acid anhydride) are combined in a flask and the system is evacuated. The mixture is reacted for 6 hours at temperatures below 180 °C, preferably between 80 °C and 150 °C. Subsequently, the caprylic acid formed as a by-product is distilled off at < 20 mbar and 130 °C. A solketal monoester of caprylic acid is obtained with a purity of < 95 %.

Subsequently, the solketal monoester of caprylic acid is subjected to an acid-induced deprotection reaction (i.e. ring-opening). Either diluted sulfuric acid or diluted phosphoric acid is used to adjust the pH value preferably to about 1 and the acetone formed as a by-product from deprotection is removed.

Then, the resulting glycerol monoester of caprylic acid (i.e. monoglyceride of caprylic acid) is further reacted with caprylic acid anhydride for 90 min at 80 °C to yield the triglyceride (i.e. tricaprylin). After the reaction is completed, the caprylic acid formed as a by-product is distilled off at < 20 mbar and 130 °C.

The resulting tricaprylin product is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.2 % based on tricaprylin,
- total content of MCPD fatty acid esters (DGF C-VI 18 (10)): 0.15 ppm based on tricaprylin,
- total content of glycidol esters of fatty acids (DGF C-VI 18 (10)): 0.13 ppm based on tricaprylin.

The tricaprylin product is then further subjected to a treatment with sodium hydrogen sulfite (NaHSO₃) in the form of a water-based solution (80 °C, 6 hours) as a nucleophilic agent (selective derivatization of halide-based impurities, i.e. MCPD fatty acid esters and glycidol fatty acid esters). After separating the water-based phase and drying the resulting tricaprylin product, the product quality as a whole is further increased since the triglyceride content is increased to values ≥ 99.5 % and the total content of MCPD fatty acid esters and that of glycidol esters of fatty acids is each below the detection limit (i.e. ≤ 0.1 ppm each). Optionally, the thus treated product may also be further treated with bleaching earth and/or activated carbon, optionally followed by deodorization (e.g. using pressurized steam). The resulting tricaprylin product, after treatment with sodium hydrogen sulfite (NaHSO₃) and optional further treatments, is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.5 % based on tricaprylin,
- total content of MCPD fatty acid esters (DGF C-VI 18 (10)): ≤ 0.1 ppm based on tricaprylin,
- total content of glycidol esters of fatty acids (DGF C-VI 18 (10)): ≤ 0.1 ppm based on tricaprylin.

In an analogous manner, also triheptanoin, on the one hand, and a medium-chain triglyceride (MCT) on the basis of caproic acid (C₆) / caprylic acid (C₈) / capric acid (C₁₀), on the other hand, are produced each with comparable results (characterization after treatment with hydrogen sulfite both for triheptanoin and for MCT each: acid value: ≤ 0.1 mg KOH / g; hydroxyl value: ≤ 0.1 mg KOH / g; triglyceride content: ≥ 99.5 %; total content of MCPD fatty acid esters: ≤ 0.1 ppm; total content of glycidol esters: ≤ 0.1 ppm).

### 5. Synthesis Route (E)

Tricaprylin is produced according to above-described Synthesis Route (E). For this purpose, the following procedure is applied:
Solketal and caprylic acid (i.e. linear saturated C₈-fatty acid or octanoic acid) are mixed with an immobilized enzyme as a catalyst (CALB lipase on polymer support, derived from *Candida antarctica,* e. g. Novozym^{®} 435 from Sigma-Aldrich or Merck or Lipozym^{®} 435 from Strem Chemicals, Inc.; 1 % by weight, based on the reaction mixture) and reacted for 6 hours at 80 °C under N₂-atmosphere. Then the enzyme is filtered off. A solketal monoester of caprylic acid is obtained.

Subsequently, the solketal monoester of caprylic acid is subjected to an acid-induced deprotection reaction (i.e. ring-opening). Either diluted sulfuric acid or diluted phosphoric acid is used to adjust the pH value preferably to about 1 and the acetone formed as a by-product from deprotection is removed.

Then, the resulting glycerol monoester of caprylic acid (i.e. monoglyceride of caprylic acid) is further reacted with caprylic acid anhydride for 90 min at 80 °C to yield the triglyceride (i.e. tricaprylin). After the reaction is completed, the caprylic acid formed as a by-product is distilled off at < 20 mbar and 130 °C.

The resulting tricaprylin product is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.4 % based on tricaprylin,
- total content of MCPD fatty acid esters (DGF C-VI 18 (10)): 0.15 ppm based on tricaprylin,
- total content of glycidol esters of fatty acids (DGF C-VI 18 (10)): 0.12 ppm based on tricaprylin.

The tricaprylin product is then further subjected to a treatment with sodium hydrogen sulfite (NaHSO₃) in the form of a water-based solution (80 °C, 6 hours) as a nucleophilic agent (selective derivatization of halide-based impurities, i.e. MCPD fatty acid esters and glycidol fatty acid esters). After separating the water-based phase and drying the resulting tricaprylin product, the product quality as a whole is further increased since the triglyceride content is increased to values ≥ 99.5 % and the total content of MCPD fatty acid esters and that of glycidol esters of fatty acids is each below the detection limit (i.e. ≤ 0.1 ppm each). Optionally, the thus treated product may also be further treated with bleaching earth and/or activated carbon, optionally followed by deodorization (e.g. using pressurized steam). The resulting tricaprylin product, after treatment with sodium hydrogen sulfite (NaHSO₃) and optional further treatments, is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.5 % based on tricaprylin,
- total content of MCPD fatty acid esters (DGF C-VI 18 (10)): ≤ 0.1 ppm based on tricaprylin,
- total content of glycidol esters of fatty acids (DGF C-VI 18 (10)): ≤ 0.1 ppm based on tricaprylin.

In an analogous manner, also triheptanoin, on the one hand, and a medium-chain triglyceride (MCT) on the basis of caproic acid (C₆) / caprylic acid (C₈) / capric acid (C₁₀), on the other hand, are produced each with comparable results (characterization after treatment with hydrogen sulfite both for triheptanoin and for MCT each: acid value: ≤ 0.1 mg KOH / g; hydroxyl value: ≤ 0.1 mg KOH / g; triglyceride content: ≥ 99.5 %; total content of MCPD fatty acid esters: ≤ 0.1 ppm; total content of glycidol esters: ≤ 0.1 ppm).

### 6. Synthesis Route (F)

Tricaprylin is produced according to above-described Synthesis Route (F). For this purpose, the following procedure is applied:
2-Octenoic acid is esterified with glycerol (≤ 0,1 ppm of total sum of MCPD fatty acid esters and glycidol esters; ≤ 0,1 ppm total chlorinated species content), using an excess of 2-octenoic acid (≤ 50 mol-%, preferably ≤ 30mol-%, based on the OH-groups of the glycerol) in the presence of an immobilized enzyme as a catalyst (CALB lipase on polymer support, derived from *Candida antarctica,* e. g. Novozym^{®} 435 from Sigma-Aldrich or Merck or Lipozym^{®} 435 from Strem Chemicals, Inc.; ≤ 0,5 % by weight, preferably ≤ 0,1 % by weight, based on the reaction mixture) at 70 °C under vacuum (≤ 100 mbar). The water formed as a reaction by-product is continuously withdrawn. Esterification is performed until a hydroxyl value (OHV) below 0,1 mg KOH / g is reached.

Then, the excess of 2-octenoic acid is removed using a short-path-distillation (<< 180 °C, << 10 mbar, preferably 120 to 130 °C, < 2 mbar). The triglyceride of 2-octenoic acid is obtained.

Subsequently, the triglyceride of 2-octenoic acid is hydrogenated using a usual hydrogenation catalyst (i.e. transition metal catalyst, e.g. Pd, Pt, Ru, Rh, Ni etc., preferably on a catalyst support) with concentrations of ≤ 10 % by weight, based on the triglyceride of 2-octenoic acid. Hydrogenation is carried out at temperatures between 50 °C and 180 °C, preferably between 80 °C and 120 °C, and at a hydrogen pressure below 50 bar, preferably between 5 bar and 30 bar. Hydrogenation is continued until the iodine value of the reaction mixture is below 1.0 g iodine / 100 g. Afterwards, the catalyst is filtered out and the reaction product is chemically washed/treated and then treated with bleaching earth/activated carbon which is afterwards filtered out. The chemical washing/treatment is performed as described hereinabove with sodium hydrogen sulfite (NaHSO₃) in the form of a water-based solution (80 °C, 6 hours).

Subsequently, the product is deodorized using pressurized steam (HPW-steam) to remove any residual traces of 2-octenoic acid at temperatures between 120 °C and below 180 °C and at a pressure of ≤ 10 mbar.

The resulting tricaprylin product after treatment with sodium hydrogen sulfite (NaHSO₃) product is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.5 % based on tricaprylin,
- total content of MCPD fatty acid esters (DGF C-VI 18 (10)): ≤ 0.1 ppm based on tricaprylin,
- total content of glycidol esters of fatty acids (DGF C-VI 18 (10)): ≤ 0.1 ppm based on tricaprylin.

The aforedescribed production procedure is repeated in an analogous manner, however, using different starting materials, namely 4-octenoic acid and 7-octenoid acid and a triple mixture of 2-octenoid acid, 4-octenoic acid and 7-octenoid acid. In each approach comparable results are obtained.

In an analogous manner, also triheptanoin, on the one hand, and a medium-chain triglyceride (MCT) on the basis of caproic acid (C₆) / caprylic acid (C₈) / capric acid (C₁₀), on the other hand, are produced each with comparable results (characterization after treatment with hydrogen sulfite both for triheptanoin and for MCT each: acid value: ≤ 0.1 mg KOH / g; hydroxyl value: ≤ 0.1 mg KOH / g; triglyceride content: ≥ 99.5 %; total content of MCPD fatty acid esters: ≤ 0.1 ppm; total content of glycidol esters: ≤ 0.1 ppm).

### Inventive feature and/or measurement (i): Selective derivatization / Treatment with at least one nucleophilic agent

To show the effectiveness of the inventive measurement (i), especially the selective derivatization of halide-based impurities, i.e. the selective removal of halide-based impurities (especially MCPD and glycidol fatty acid esters), commercially available triglycerides are subjected to the treatment according to inventive measurement (i).

A commercial product on the basis of a MCT oil (triglyceride) has a glycidol ester content of 70 ppm. After 6 hours of treatment with aqueous sodium hydrogen sulfite at 80 °C the glycidol ester content is reduced to 9.11 ppm; thus, a reduction by a factor of about 7.5 is achieved.

Another commercial product on the basis of a MCT oil (triglyceride) (MCT 60/40) has a 3-MCPD fatty acid ester content of 8 ppm. After 6 hours of treatment with aqueous sodium hydrogen sulfite at 80 °C the 3-MCPD content is reduced to 2.21 ppm; thus, a reduction by a factor of about 3.5 is achieved.

A further commercial product (Miglyol^{®} 812N, C₈ / C₁₀ - triglyceride) has a 3-MCPD fatty acid ester content of 1.56 ppm. After 15 hours of treatment with 12 % aqueous sodium hydrogen sulfite at 80 °C the 3-MCPD content is reduced to 0.16 ppm; thus, a reduction by a factor of about 9.7 is achieved.

Comparable results are reached when, instead of sodium hydrogen sulfite (NaHSO₃), also other nucleophilic agents are used (i.e. sodium hydrogen thiosulfate NaHS₂O₃, sodium hydrogen phosphite NaH₂PO₃ or phosphines). The obtained results are comparable.

Thus, the inventive feature and/or measurement (i) effectively reduces the content of halide-based impurities, especially MCPD and glycidol fatty acid ester. However, only on the basis of inventive measurement (i) alone, it is not always sufficient to fulfil the overall product specifications comprising a fivefold purity / grade requirement with aforedefined items (1) to (5), especially not when starting form commercial or prior art triglycerides with low purity grades; however, especially at least the features of (4) the total content of MCPD fatty acid esters below 0.1 ppm and (5) the total content of glycidol esters below 0.1 ppm can be efficiently adjusted and/or effected by this measurement (i).

### Conclusions

Applicant has been able to provide a universal concept / process for producing high-purity triglycerides of fatty acids with high yields and high conversion rates, which concept / process is independent from any specific synthesis route. Thus, applicant's concept is universal to be applied for this purpose.

The inventive process is at the same time highly flexible and compatible with known prior art production processes and plants for these substances since the aforenamed inventive features and/or measurements (i) to (iv) can be easily implemented in or used to modify known production processes and plants for these substances.

Above all, the inventive process produces triglycerides of fatty acids with a high purity degree, which at the same time unify a combination of at least five purity properties, namely: (1) a very low acid value (AV), (2) a very low hydroxyl value (OHV), (3) a very high triglyceride content, (4) a very low total content of MCPD fatty acid esters and (5) a very low total content of glycidol esters of fatty acids, wherein this purity specification can be reached independently from any specific synthesis route. Such combination of purity properties (1) to (5) was deemed incompatible or non-realizable so far; however, as applicant has surprisingly found out, the inventive process renders such combination of purity properties possible for the very first time.

Furthermore, nucleophilic agents, such as e.g. hydrogen sulfites (bisulfites, HSO₃⁻), sulfites (SO₃²⁻), hydrogen thiosulfates (HS₂O₃⁻), thiosulfates (S₂O₃²⁻), hydrogen phosphites (H₂PO₃⁻), phosphites (HPO₃²⁻) and phosphines as well as combinations thereof, are an excellent reactant agent for purifying triglycerides of fatty acids, particularly triglycerides of C₅-C₁₂-fatty acids, especially triglycerides of C₆-C₁₂-fatty acids, especially for removing halide-based impurities, especially chlorine-based impurities, from triglycerides of fatty acids, particularly chlorine-based impurities selected from the group consisting of MCPD fatty acid esters (fatty acid esters of monochloropropanediols), glycidol esters of fatty acids and combinations thereof (i.e. selective derivatization of halide-based impurities).

## Claims

1. A process for producing triglycerides of fatty acids, especially with a high purity degree, wherein 1,2,3-propantriol of formula (I)
CH₂(OH) - CH(OH) - CH₂(OH) (I)
or its protected form or precursor, preferably solketal, is used as starting material and is reacted and/or converted to the corresponding triglycerides of fatty acids,
so as to produce, as a reaction product, one or more triglycerides of C₅-C₁₂-fatty acids, especially one or more triglycerides of C₆-C₁₂-fatty acids, of general formula (II)
CH₂[O - C(O)R¹] - CH[O - C(O)R²] - CH₂[O - C(O)R³] (II)
wherein in general formula (II) the radicals R¹, R² and R³, independently of one another, each represent a linear or branched, saturated or unsaturated aliphatic C₄-C₁₁-alkyl radical, especially C₅-C₁₁-alkyl radical, preferably a linear saturated aliphatic C₄-C₁₁-alkyl radical, especially C₅-C₁₁-alkyl radical,
wherein the process comprises at least one esterification step, especially an esterification step using C₅-C₁₂-fatty acids, especially C₆-C₁₂-fatty acids, and/or their anhydrides and/or esters;
wherein the process is **characterized by** feature and/or measurement (i), optionally in further combination with feature and/or measurement (ii) and/or feature and/or measurement (iii) and/or feature and/or measurement (iv):
(i) a preferably selective derivatization of MCPD fatty acid esters and glycidol esters of fatty acids present in the resulting reaction product is performed and/or the resulting reaction product is treated and/or contacted with at least one nucleophilic agent selected from the group consisting of hydrogen sulfites (HSO₃⁻), sulfites (SO₃²⁻), hydrogen thiosulfates (HS₂O₃⁻), thiosulfates (S₂O₃²⁻), hydrogen phosphites (H₂PO₃⁻), phosphites (HPO₃²⁻) and phosphines as well as combinations thereof, more preferably with hydrogen sulfites (HSO₃⁻), in particular for a preferably selective derivatization of MCPD fatty acid esters and glycidol esters of fatty acids;
(ii) the at least one esterification step is run and/or performed at temperatures not exceeding 180 °C, especially not exceeding 150 °C, particularly not exceeding 120 °C, preferably not exceeding 100 °C, more preferably not exceeding 80 °C, even more preferably not exceeding 50 °C;
(iii) the 1,2,3-propantriol of formula (I) or its protected form, preferably solketal, used as starting material is free of any chlorinated species and comprises a total chlorinated species content of at most 1 ppm, especially at most 0.5 ppm, preferably at most 0.1 ppm, based on the 1,2,3-propantriol of formula (I) or its protected form, preferably solketal;
(iv) the at least one esterification step is run and/or performed in the absence of any metal-based catalyst.

2. The process according to Claim 1,
wherein the reaction product, especially the one or more triglycerides of C₅-C₁₂-fatty acids, especially one or more triglycerides of C₆-C₁₂-fatty acids, of general formula (II)
CH₂[O - C(O)R¹] - CH[O - C(O)R²] - CH₂[O - C(O)R³] (II)
wherein in general formula (II) the radicals R¹, R² and R³, independently of one another, each represent a linear or branched, saturated or unsaturated aliphatic C₄-C₁₁-alkyl radical, especially C₅-C₁₁-alkyl radical, preferably a linear saturated aliphatic C₄-C₁₁-alkyl radical, especially C₅-C₁₁-alkyl radical,
is/are **characterized by** and/or exhibit(s) the following properties (1) to (5) in their combination and/or fulfil(s) the following product specification:
(1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
(2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
(3) triglyceride content, especially determined according to Ph. Eur. 2.2.28 (European Pharmacopoeia 10.0): ≥ 99 %, especially ≥ 99.2 %, preferably ≥ 99.5 %, each based on the reaction product; and
(4) total content of MCPD fatty acid esters, especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the reaction product; and
(5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.5 ppm, especially ≤ 0.2 ppm, preferably ≤ 0.1 ppm, each based on the reaction product; and/or
wherein the process is performed such and/or feature and/or measurement (i), optionally in further combination with feature and/or measurement (ii) and/or feature and/or measurement (iii) and/or feature and/or measurement (iv) are applied and/or performed with the proviso that the reaction product, especially the one or more triglycerides of C₅-C₁₂-fatty acids, especially one or more triglycerides of C₆-C₁₂-fatty acids, of general formula (II)
CH₂[O - C(O)R¹] - CH[O - C(O)R²] - CH₂[O - C(O)R³] (II)
wherein in general formula (II) the radicals R¹, R² and R³, independently of one another, each represent a linear or branched, saturated or unsaturated aliphatic C₄-C₁₁-alkyl radical, especially C₅-C₁₁-alkyl radical, preferably a linear saturated aliphatic C₄-C₁₁-alkyl radical, especially C₅-C₁₁-alkyl radical,
is/are **characterized by** and/or exhibit(s) the following properties (1) to (5) in their combination and/or fulfil(s) the following product specification:
(1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
(2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
(3) triglyceride content, especially determined according to Ph. Eur. 2.2.28 (European Pharmacopoeia 10.0): ≥ 99 %, especially ≥ 99.2 %, preferably ≥ 99.5 %, each based on the reaction product; and
(4) total content of MCPD fatty acid esters, especially determined according to method of DGF C-VI 18 (10) (Einheitsmethode der Deutschen Gesellschaft für Eettwissenschaft [Standard method of German Society for Fat Science]): ≤ 0.5 ppm, especially ≤ 0.2 ppm, preferably ≤ 0.1 ppm, each based on the reaction product; and
(5) total content of glycidol esters of fatty acids, especially determined according to method of DGF C-VI 18 (10) (Einheitsmethode der Deutschen Gesellschaft für Fett-wissenschaft e.V. [Standard method of German Society for Fat Science]): ≤ 0.5 ppm, especially ≤ 0.2 ppm, preferably ≤ 0.1 ppm, each based on the reaction product.

3. The process for producing triglycerides of fatty acids, especially with a high purity degree, according to Claim 1 or Claim 2,
wherein 1,2,3-propantriol of formula (I)
CH₂(OH) - CH(OH) - CH₂(OH) (I)
or its protected form or precursor, preferably solketal, is used as starting material and is reacted and/or converted to the corresponding triglycerides of fatty acids,
so as to produce, as a reaction product, one or more triglycerides of C₅-C₁₂-fatty acids, especially one or more triglycerides of C₆-C₁₂-fatty acids, of general formula (II)
CH₂[O - C(O)R¹] - CH[O - C(O)R²] - CH₂[O - C(O)R³] (II)
wherein in general formula (II) the radicals R¹, R² and R³, independently of one another, each represent a linear or branched, saturated or unsaturated aliphatic C₄-C₁₁-alkyl radical, especially C₅-C₁₁-alkyl radical, preferably a linear saturated aliphatic C₄-C₁₁-alkyl radical, especially C₅-C₁₁-alkyl radical,
wherein the process comprises at least one esterification step, especially an esterification step using C₅-C₁₂-fatty acids, especially C₆-C₁₂-fatty acids, and/or their anhydrides and/or esters;
wherein the process is **characterized by** feature and/or measurement (i), optionally in further combination with feature and/or measurement (ii) and/or feature and/or measurement (iii) and/or feature and/or measurement (iv):
(i) a preferably selective derivatization of MCPD fatty acid esters and glycidol esters of fatty acids present in the resulting reaction product is performed and/or the resulting reaction product is treated and/or contacted with at least one nucleophilic agent selected from the group consisting of hydrogen sulfites (HSO₃⁻), sulfites (SO₃²⁻), hydrogen thiosulfates (HS₂O₃⁻), thiosulfates (S₂O₃²⁻), hydrogen phosphites (H₂PO₃⁻), phosphites (HPO₃²⁻) and phosphines as well as combinations thereof, more preferably with hydrogen sulfites (HSO₃⁻), in particular for a preferably selective derivatization of MCPD fatty acid esters and glycidol esters of fatty acids;
(ii) the at least one esterification step is run and/or performed at temperatures not exceeding 180 °C, especially not exceeding 150 °C, particularly not exceeding 120 °C, preferably not exceeding 100 °C, more preferably not exceeding 80 °C, even more preferably not exceeding 50 °C;
(iii) the 1,2,3-propantriol of formula (I) or its protected form, preferably solketal, used as starting material is free of any chlorinated species and comprises a total chlorinated species content of at most 1 ppm, especially at most 0.5 ppm, preferably at most 0.1 ppm, based on the 1,2,3-propantriol of formula (I) or its protected form, preferably solketal;
(iv) the at least one esterification step is run and/or performed in the absence of any metal-based catalyst,
wherein the process is performed such and/or feature and/or measurement (i), optionally in further combination with feature and/or measurement (ii) and/or feature and/or measurement (iii) and/or feature and/or measurement (iv) are applied and/or performed with the proviso that the reaction product, especially the one or more triglycerides of C₅-C₁₂-fatty acids, especially one or more triglycerides of C₆-C₁₂-fatty acids, of above general formula (II), is/are **characterized by** and/or exhibit(s) the following properties (1) to (5) in their combination and/or fulfil(s) the following product specification:
(1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
(2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
(3) triglyceride content, especially determined according to Ph. Eur. 2.2.28 (European Pharmacopoeia 10.0): ≥ 99 %, especially ≥ 99.2 %, preferably ≥ 99.5 %, each based on the reaction product; and
(4) total content of MCPD fatty acid esters, especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the reaction product; and
(5) total content of glycidol esters of fatty acids, especially determined according to method of DGF C-VI 18 (10) (Einheitsmethode der Deutschen Gesellschaft für Fett-wissenschaft e.V. [Standard method of German Society for Fat Science]): ≤ 0.5 ppm, especially ≤ 0.2 ppm, preferably ≤ 0.1 ppm, each based on the reaction product.

4. The process according to any of the preceding claims,
wherein the process is performed such and/or feature and/or measurement (i), optionally in further combination with feature and/or measurement (ii) and/or feature and/or measurement (iii) and/or feature and/or measurement (iv) are applied and/or performed with the proviso that the reaction product, especially the one or more triglycerides of C₅-C₁₂-fatty acids, especially one or more triglycerides of C₆-C₁₂-fatty acids, of above general formula (II), is/are **characterized by** and/or exhibit(s) the following properties (1) to (5) in their combination and/or fulfil(s) the following product specification:
(1) acid value (AV), especially determined according to Ph. Eur. 2.5.1 (European Pharmacopoeia 10.0): ≤ 0.1 mg KOH / g; and
(2) hydroxyl value (OHV), especially determined according to Ph. Eur. 2.5.3 (European Pharmacopoeia 10.0): ≤ 0.1 mg KOH / g; and
(3) triglyceride content, especially determined according to Ph. Eur. 2.2.28 (European Pharmacopoeia 10.0): ≥ 99.5 %, especially ≥ 99.6 %, preferably ≥ 99.8 %, each based on the reaction product; and
(4) total content of MCPD fatty acid esters, especially determined according to method of DGF C-VI 18 (10) (Einheitsmethode der Deutschen Gesellschaft für Fettwissenschaft [Standard method of German Society for Fat Science]): ≤ 0.1 ppm based on the reaction product; and
(5) total content of glycidol esters of fatty acids, especially determined according to method of DGF C-VI 18 (10) (Einheitsmethode der Deutschen Gesellschaft für Fett-wissenschaft e.V. [Standard method of German Society for Fat Science]): ≤ 0.1 ppm based on the reaction product.

5. The process according to any of the preceding claims,
wherein the C₅-C₁₂-fatty acids, especially C₆-C₁₂-fatty acids, and/or their anhydrides and/or esters used in the at least one esterification step are represented by general formula (III)
R⁴ - C (O)OH (III)
wherein in general formula (III) the radical R⁴ represents a linear or branched, saturated or unsaturated aliphatic C₄-C₁₁-alkyl radical, especially C₅-C₁₁-alkyl radical, preferably a linear saturated aliphatic C₄-C₁₁-alkyl radical, especially C₅-C₁₁-alkyl radical, including their anhydrides and esters as well as combinations thereof; and/or wherein the C₅-C₁₂-fatty acids, especially C₆-C₁₂-fatty acids, and/or their anhydrides and/or esters used in the at least one esterification are linear saturated C₅-C₁₂-fatty acids, especially linear saturated C₆-C₁₂-fatty acids, and/or their anhydrides and/or esters; and/or
wherein the C₅-C₁₂-fatty acids, especially C₆-C₁₂-fatty acids, and/or their anhydrides and/or esters used in the at least one esterification step are selected from the group consisting of valeric acid, caproic acid, enanthic acid, caprylic acid, pelargonic acid, capric acid, undecylic acid, lauric acid and/or their anhydrides and/or esters, especially caproic acid, enanthic acid, caprylic acid, pelargonic acid, capric acid, undecylic acid, lauric acid and/or their anhydrides and/or esters, preferably caproic acid, enanthic acid, caprylic acid, capric acid, lauric acid and/or their anhydrides and/or esters, more preferably caproic acid, enanthic acid, caprylic acid, capric acid and/or their anhydrides and/or esters.

6. The process according to any of Claims 1 to 5,
wherein the reaction product is one or more triglycerides of C₅-C₁₂-fatty acids, especially one or more triglycerides of C₆-C₁₂-fatty acids, of above-defined general formula (II), however, with the proviso that all radicals R¹, R² and R³ are identical.

7. The process according to any of Claims 1 to 6,
wherein the reaction product is triheptanoin; and/or
wherein in above general formula (II) the radicals R¹, R² and R³ each represent a linear saturated aliphatic C₆-alkyl radical; and/or
wherein in the at least one esterification step heptanoic acid and/or its anhydride and/or esters is used.

8. The process according to any of Claims 1 to 6,
wherein the reaction product is tricaprylin; and/or
wherein in above general formula (II) the radicals R¹, R² and R³ each represent a linear saturated aliphatic C₇-alkyl radical; and/or
wherein in the at least one esterification step octanoic acid and/or its anhydride and/or esters is used.

9. The process according to any of Claims 1 to 7,
wherein the reaction product is not tricaprylin.

10. The process according to any of Claims 1 to 5,
wherein the reaction product is one or more triglycerides of C₅-C₁₂-fatty acids, especially one or more triglycerides of C₆-C₁₂-fatty acids, of above-defined general formula (II), however, with the proviso that at least two of the radicals R¹, R² and R³ are different from one another.

11. The process according to any of Claims 1 to 5 and 10,
wherein the reaction product is one or more triglycerides of C₅-C₁₂-fatty acids, especially one or more triglycerides of C₆-C₁₂-fatty acids, of above-defined general formula (II), however, with the proviso that all radicals R¹, R² and R³ are different from one another.

12. The process according to any of the preceding claims,
wherein the process is **characterized by** a combination of feature and/or measurement (i) with at least two of the features and/or measurements (ii) to (iv); and/or wherein the process is **characterized by** a combination of all of features and/or measurements (i) to (iv).

13. The process according to any of the preceding claims,
wherein the whole and/or overall process, especially also including optional pre-treatment and/or post-treatment steps if any, is run and/or performed at temperatures not exceeding 180 °C, especially not exceeding 160 °C, particularly not exceeding 150 °C, preferably not exceeding 120 °C; and/or
wherein the process is **characterized by** further feature and/or measurement (v):
(v) the whole and/or overall process, especially also including optional pre-treatment and/or post-treatment steps if any, is run and/or performed at temperatures not exceeding 180 °C, especially not exceeding 160 °C, particularly not exceeding 150 °C, preferably not exceeding 120 °C.

14. The process according to any of the preceding claims,
wherein the whole and/or overall process, especially also including optional pre-treatment and/or post-treatment steps if any, is run and/or performed in the absence of any metal-based catalyst; and/or
wherein the process is **characterized by** further feature and/or measurement (vi):
(vi) the whole and/or overall process, especially also including optional pre-treatment and/or post-treatment steps if any, is run and/or performed in the absence of any metal-based catalyst.

15. The process according to any of the preceding claims,
wherein the at least one esterification step is performed and/or carried out as a one-stage esterification or, alternatively, as a two-stage or multistage esterification.

16. The process according to any of the preceding claims,
wherein the at least one esterification step is performed and/or carried out as a one-stage esterification, using anhydrides of C₅-C₁₂-fatty acids to be reacted with 1,2,3-propantriol of formula (I) or its protected form or precursor, preferably solketal, or, alternatively, wherein the at least one esterification step is performed and/or carried out as a two-stage or multistage esterification, in a first stage using C₅-C₁₂-fatty acids and/or their esters to be reacted with the 1,2,3-propantriol of formula (I) or its protected form or precursor, preferably solketal, followed in a subsequent stage by further esterification, especially further esterification of free and/or remaining OH-groups, preferably using anhydrides of C₅-C₁₂-fatty acids.

17. The process according to any of the preceding claims,
wherein the at least one esterification step is performed and/or carried out in the absence of solvents and/or without any solvent.

18. The process according to any of the preceding claims,
wherein the at least one esterification step is performed and/or carried out at temperatures in the range of from 5 °C to 95 °C, especially in the range of from 10 °C to 85 °C, preferentially in the range of from 15 °C to 80 °C, more preferably in the range of from 20 °C to 75 °C, even more preferably in the range of from 25 °C to 70 °C; and/or
wherein the at least one esterification step is performed and/or carried out at a pressure in the range of from 0.0001 bar to 10 bar, especially in the range of from 0.001 bar to 5 bar, preferentially in the range of from 0.01 bar to 2 bar, more preferably in the range of from 0.05 bar to 1 bar, even more preferably at 1 bar.

19. The process according to any of the preceding claims,
wherein the at least one esterification step is performed and/or carried out in the absence of any catalyst or, alternatively, wherein the at least one esterification step is performed and/or carried out at least partially in the presence of an enzyme as a catalyst and/or at least partially in the presence of an enzyme-based catalyst;
especially wherein the catalyst is recycled after the at least one esterification step.

20. The process according to any of the preceding claims,
wherein the at least one esterification step is performed and/or carried out in the absence of any catalyst when using anhydrides of C₅-C₁₂-fatty acids to be reacted with 1,2,3-propantriol of formula (I) or its protected form or precursor, preferably solketal, or, alternatively, wherein the at least one esterification step is performed and/or carried out at least partially in the presence of an enzyme as a catalyst and/or at least partially in the presence of an enzyme-based catalyst when using C₅-C₁₂-fatty acids and/or their esters to be reacted with the 1,2,3-propantriol of formula (I) or its protected form or precursor, preferably solketal;
especially wherein the catalyst is recycled after the at least one esterification step.

21. The process according to any of the preceding claims,
wherein the at least one esterification step is performed and/or carried out at least partially in the presence of an enzyme as a catalyst and/or at least partially in the presence of an enzyme-based catalyst;
especially wherein the enzyme is selected from synthetases, catalases, esterases, lipases and combinations thereof; and/or
especially wherein the enzyme is derived from Candida antarctica, Mucor miehei, Thermomyces lanuginosus, Candida rugosa, Aspergillus oryzae, Pseudomonas cepacia, Pseudomonas fluorescens, Rhizopus delemar and Pseudomonas sp. and combinations thereof, preferably from Candida antarctica, Mucor miehei and Thermomyces lanuginosus, more preferably from Candida antarctica; and/or especially wherein the enzyme is used in immobilized form, especially immobilized on a carrier, preferentially on a polymeric carrier, preferably on a polymeric organic carrier, more preferably with hydrophobic properties, even more preferably on a poly(meth)acrylic resin-based carrier; and/or
especially wherein the enzyme is recycled after the at least one esterification step; and/or
especially wherein the at least one esterification step is performed and/or carried out at temperatures in the range of from 5 °C to 95 °C, especially in the range of from 10 °C to 85 °C, preferentially in the range of from 15 °C to 80 °C, more preferably in the range of from 20 °C to 75 °C, even more preferably in the range of from 25 °C to 70 °C; and/or especially wherein the enzyme is used in amounts, based on the total amount of all starting compounds, in the range of from 0.0001 to 25 % by weight, especially in the range of from 0.001 to 20 % by weight, preferentially in the range of from 0.01 to 15 % by weight, preferably in the range of from 0.05 to 10 % by weight; and/or
especially wherein the enzyme is used in amounts, based on the total amount of all starting compounds, in the range of from 1 • 10⁻⁶ to 5 mol-%, especially in the range of from 1 • 10⁻⁵ to 2.5 mol-%, preferentially in the range of from 1 • 10⁻¹ to 1.5 mol-%, preferably in the range of from 1 • 10⁻³ to 1 mol-%; and/or
especially wherein the at least one esterification step is performed and/or carried out at a pressure in the range of from 0.0001 bar to 10 bar, especially in the range of from 0.001 bar to 5 bar, preferentially in the range of from 0.01 bar to 2 bar, more preferably in the range of from 0.05 bar to 1 bar, even more preferably at 1 bar,

22. The process of any of the preceding claims,
wherein the reaction product is produced via one of the following synthesis routes (A) to (F):
(A) wherein, according to a Synthesis Route (A), 1,2,3-propantriol of formula (I)
CH₂(OH) - CH(OH) - CH₂(OH) (I)
is first reacted, preferably in the presence of an enzyme as a catalyst and/or in the presence of an enzyme-based catalyst, with at least one linear or branched, saturated or unsaturated aliphatic C₅-C₁₂-fatty acid or its ester, especially with at least one linear or branched, saturated or unsaturated aliphatic C₆-C₁₂-fatty acid or its ester, preferably with at least one linear saturated aliphatic C₅-C₁₂-fatty acid or its ester, especially with at least one linear saturated aliphatic C₆-C₁₂-fatty acid or its ester, preferably so as to produce a diglyceride, more preferably a 1,3-diglyceride,
followed by subsequent reaction, preferably in the absence of any catalyst, with at least one linear or branched, saturated or unsaturated aliphatic C₅-C₁₂-fatty acid anhydride, especially with at least one linear or branched, saturated or unsaturated aliphatic C₆-C₁₂-fatty acid anhydride, preferably with at least one linear saturated aliphatic C₅-C₁₂-fatty acid anhydride, especially with at least one linear saturated aliphatic C₆-C₁₂-fatty acid anhydride,
so as to produce the reaction product of general formula (II) as defined hereinabove;
or else
(B) wherein, according to a Synthesis Route (B), 1,2,3-propantriol of formula (I)
CH₂(OH) - CH(OH) - CH₂(OH) (I)
is first reacted, preferably in the presence of an enzyme as a catalyst and/or in the presence of an enzyme-based catalyst, with at least one linear or branched, saturated or unsaturated aliphatic C₅-C₁₂-fatty acid or its ester, especially with at least one linear or branched, saturated or unsaturated aliphatic C₆-C₁₂-fatty acid or its ester, preferably with at least one linear saturated aliphatic C₅-C₁₂-fatty acid or its ester, especially with at least one linear saturated aliphatic C₆-C₁₂-fatty acid or its ester, preferably so as to produce a diglyceride, more preferably a 1,3-diglyceride,
followed by subsequent reaction, preferably in the presence of an enzyme as a catalyst and/or in the presence of an enzyme-based catalyst, with at least one linear or branched, saturated or unsaturated aliphatic C₅-C₁₂-fatty acid or its ester, especially with at least one linear or branched, saturated or unsaturated aliphatic C₆-C₁₂-fatty acid or its ester, preferably with at least one linear saturated aliphatic C₅-C₁₂-fatty acid or its ester, especially with at least one linear saturated aliphatic C₆-C₁₂-fatty acid or its ester,
so as to produce the reaction product of general formula (II) as defined hereinabove;
or else
(C) wherein, according to a Synthesis Route (C), 1,2,3-propantriol of formula (I)
CH₂(OH) - CH(OH) - CH₂(OH) (I)
is reacted, preferably in the absence of any catalyst, with at least one linear or branched, saturated or unsaturated aliphatic C₅-C₁₂-fatty acid anhydride, especially with at least one linear or branched, saturated or unsaturated aliphatic C₆-C₁₂-fatty acid anhydride, preferably with at least one linear saturated aliphatic C₅-C₁₂-fatty acid anhydride, especially with at least one linear saturated aliphatic C₆-C₁₂-fatty acid anhydride,
so as to produce the reaction product of general formula (II) as defined hereinabove;
or else
(D) wherein, according to a Synthesis Route (D), a protected form or precursor of 1,2,3-propantriol of above formula (I), preferably solketal, is reacted, preferably in the absence of any catalyst, with at least one linear or branched, saturated or unsaturated aliphatic C₅-C₁₂-fatty acid anhydride, especially with at least one linear or branched, saturated or unsaturated aliphatic C₆-C₁₂-fatty acid anhydride, preferably with at least one linear saturated aliphatic C₅-C₁₂-fatty acid anhydride, especially with at least one linear saturated aliphatic C₆-C₁₂-fatty acid anhydride,
especially wherein a deprotection reaction, especially ring-opening and optionally removal of acetone, is performed after monoesterification of the free OH-group of the protected form or precursor of 1,2,3-propantriol of above formula (I), followed by further esterification with the at least one linear or branched, saturated or unsaturated aliphatic C₅-C₁₂-fatty acid anhydride, especially with the at least one linear or branched, saturated or unsaturated aliphatic C₆-C₁₂-fatty acid anhydride, preferably with the at least one linear saturated aliphatic C₅-C₁₂-fatty acid anhydride, especially with the at least one linear saturated aliphatic C₆-C₁₂-fatty acid anhydride,
so as to produce the reaction product of general formula (II) as defined hereinabove;
or else
(E) wherein, according to a Synthesis Route (E), a protected form or precursor of 1,2,3-propantriol of above formula (I), preferably solketal, is reacted, preferably in the presence of an enzyme as a catalyst and/or in the presence of an enzyme-based catalyst, with at least one linear or branched, saturated or unsaturated aliphatic C₅-C₁₂-fatty acid or its ester, especially with at least one linear or branched, saturated or unsaturated aliphatic C₆-C₁₂-fatty acid or its ester, preferably with at least one linear saturated aliphatic C₅-C₁₂-fatty acid or its ester, especially with at least one linear saturated aliphatic C₆-C₁₂-fatty acid or its ester, followed by deprotection reaction, especially ring-opening and removal of acetone, preferably so as to produce a monoglyceride, more preferably a terminal monoglyceride and/or 1-monoglyceride,
followed by subsequent reaction, preferably in the absence of any catalyst, with at least one linear or branched, saturated or unsaturated aliphatic C₅-C₁₂-fatty acid anhydride, especially with at least one linear or branched, saturated or unsaturated aliphatic C₆-C₁₂-fatty acid anhydride, preferably with at least one linear saturated aliphatic C₅-C₁₂-fatty acid anhydride, especially with at least one linear saturated aliphatic C₆-C₁₂-fatty acid anhydride,
so as to produce the reaction product of general formula (II) as defined hereinabove;
or else
(F) wherein, according to a Synthesis Route (F), 1,2,3-propantriol of formula (I)
CH₂(OH) - CH(OH) - CH₂(OH) (I)
is reacted, optionally in the presence of an enzyme as a catalyst and/or in the presence of an enzyme-based catalyst, with at least one linear or branched, mono- or poly-unsaturated aliphatic C₅-C₁₂-fatty acid and/or its esters and/or anhydrides, especially with at least one linear or branched, mono- or poly-unsaturated aliphatic C₆-C₁₂-fatty acid and/or its esters and/or anhydrides, preferably with at least one linear mono- or poly-unsaturated aliphatic C₅-C₁₂-fatty acid and/or its esters and/or anhydrides, especially with at least one linear mono- or poly-unsaturated aliphatic C₆-C₁₂-fatty acid and/or its esters and/or anhydrides, preferably so as to produce triglycerides of mono- or poly-unsaturated aliphatic C₅-C₁₂-fatty acids,
followed by subsequent hydrogenation reaction, preferably in the presence of at least one hydrogenation catalyst, preferably so as to hydrogenate the double bonds of the mono- or poly-unsaturated aliphatic C₅-C₁₂-fatty acid radicals of the triglycerides,
so as to produce the reaction product of general formula (II) as defined hereinabove.

## Patentansprüche

1. Verfahren zur Herstellung von Triglyceriden von Fettsäuren, insbesondere mit hohem Reinheitsgrad,
wobei 1,2,3-Propantriol der Formel (I)
CH₂(OH) - CH(OH) - CH₂(OH) (I)
oder dessen geschützte Form oder Vorläufer, vorzugsweise Solketal, als Ausgangsmaterial verwendet und zu den entsprechenden Triglyceriden von Fettsäuren umgesetzt und/oder umgewandelt wird,
um als Reaktionsprodukt ein oder mehrere Triglyceride von C₅-C₁₂-Fettsäuren, insbesondere ein oder mehrere Triglyceride von C₆-C₁₂-Fettsäuren, der allgemeinen Formel (II)
CH₂[O - C(O)R¹] - CH [O - C(O)R²] - CH₂[O - C(O)R³] (II)
zu bilden,
wobei in der allgemeinen Formel (II) die Reste R¹, R² und R³, jeweils unabhängig voneinander, einen linearen oder verzweigten, gesättigten oder ungesättigten aliphatischen C₄-C₁₁-Alkylrest, insbesondere C₅-C₁₁-Alkylrest, vorzugsweise einen linearen, gesättigten aliphatischen C₄-C₁₁-Alkylrest, insbesondere C₅-C₁₁-Alkylrest, darstellen,
wobei das Verfahren mindestens einen Veresterungsschritt umfasst, insbesondere einen Veresterungsschritt unter Verwendung von C₅-C₁₂-Fettsäuren, insbesondere C₆-C₁₂-Fettsäuren, und/oder deren Anhydriden und/oder Estern;
wobei das Verfahren durch Merkmal und/oder Maßnahme (i) gekennzeichnet ist, wahlweise in weiterer Kombination mit Merkmal und/oder Maßnahme (ii) und/oder Merkmal und/oder Maßnahme (iii) und/oder Merkmal und/oder Maßnahme (iv):
(i) eine vorzugsweise selektive Derivatisierung von im resultierenden Reaktionsprodukt vorhandenen MCPD-Fettsäureestern und Glycidolestern von Fettsäuren wird durchgeführt und/oder das resultierende Reaktionsprodukt wird mit mindestens einem nukleophilen Behandlungsmittel, ausgewählt aus der Gruppe bestehend aus Hydrogensulfiten (HSO₃⁻), Sulfiten (SO₃²⁻), Hydrogenthiosulfaten (HS₂O₃⁻), Thiosulfaten (S₂O₃²⁻), Hydrogenphosphiten (H₂PO₃⁻), Phosphiten (HPO₃²⁻) und Phosphinen sowie Kombinationen davon, vorzugsweise mit Hydrogensulfit (HSO₃⁻), behandelt und/oder in Kontakt gebracht, insbesondere zur vorzugsweise selektiven Derivatisierung von MCPD-Fettsäureestern und Glycidolestern von Fettsäuren;
(ii) der mindestens eine Veresterungsschritt erfolgt und/oder wird durchgeführt bei Temperaturen von nicht mehr als 180 °C, insbesondere nicht mehr als 150 °C, vorzugsweise nicht mehr als 120 °C, bevorzugt nicht mehr als 100 °C, besonders bevorzugt nicht mehr als 80 °C, noch weiter bevorzugt nicht mehr als 50 °C;
(iii) das als Ausgangsmaterial verwendete 1,2,3-Propantriol der Formel (I) oder dessen geschützte Form, vorzugsweise Solketal, ist frei von jeglichen chlorierten Spezies und weist einen Gesamtgehalt an chlorierten Spezies von höchstens 1 ppm, insbesondere höchstens 0,5 ppm, vorzugsweise höchstens 0,1 ppm, bezogen auf das 1,2,3-Propantriol der Formel (I) oder dessen geschützte Form, vorzugsweise Solketal, auf;
(iv) der mindestens eine Veresterungsschritt erfolgt und/oder wird durchgeführt in Abwesenheit jeglicher metallbasierter Katalysatoren.

2. Verfahren nach Anspruch 1,
wobei das Reaktionsprodukt, insbesondere das eine oder die mehreren Triglyceride von C₅-C₁₂-Fettsäuren, insbesondere das eine oder die mehreren Triglyceride von C₆-C₁₂-Fettsäuren, der allgemeinen Formel (II)
CH₂[O - C(O)R¹] - CH[O - C(O)R²] - CH₂[O - C(O)R³] (II)
wobei in der allgemeinen Formel (II) die Reste R¹, R² und R³, jeweils unabhängig voneinander, einen linearen oder verzweigten, gesättigten oder ungesättigten aliphatischen C₄-C₁₁-Alkylrest, insbesondere C₅-C₁₁-Alkylrest, vorzugsweise einen linearen, gesättigten aliphatischen C₄-C₁₁-Alkylrest, insbesondere C₅-C₁₁-Alkylrest, darstellen,
durch die folgenden Eigenschaften (1) bis (5) in ihrer Kombination gekennzeichnet ist/sind und/oder diese aufweist/aufweisen und/oder der folgenden Produktspezifikation genügt/genügen:
(1) Säurezahl (AV), insbesondere bestimmt gemäß Ph. Eur. 2.5.1 (Europäisches Arzneibuch 10.0): ≤ 0,5 mg KOH / g, insbesondere ≤ 0,2 mg KOH / g, vorzugsweise ≤ 0,1 mg KOH / g; und
(2) Hydroxylzahl (OHV), insbesondere bestimmt gemäß Ph. Eur. 2.5.3 (Europäisches Arzneibuch 10.0): ≤ 0,5 mg KOH / g, insbesondere ≤ 0,2 mg KOH / g, vorzugsweise ≤ 0,1 mg KOH / g; und
(3) Triglyceridgehalt, insbesondere bestimmt gemäß Ph. Eur. 2.2.28 (Europäisches Arzneibuch 10.0): ≥ 99 %, insbesondere ≥ 99,2 %, vorzugsweise ≥ 99,5 %, jeweils bezogen auf das Reaktionsprodukt; und
(4) Gesamtgehalt an MCPD-Fettsäureestern, insbesondere bestimmt nach der Methode DGF C-VI 18 (10) (Einheitsmethode der Deutschen Gesellschaft für Fettwissenschaft): ≤ 0,5 ppm, insbesondere ≤ 0,2 ppm, vorzugsweise ≤ 0,1 ppm, jeweils bezogen auf das Reaktionsprodukt; und
(5) Gesamtgehalt an Glycidolestern von Fettsäuren, insbesondere bestimmt nach der Methode DGF C-VI 18 (10) (Einheitsmethode der Deutschen Gesellschaft für Fettwissenschaft): ≤ 0,5 ppm, insbesondere ≤ 0,2 ppm, vorzugsweise ≤ 0,1 ppm, jeweils bezogen auf das Reaktionsprodukt; und/oder
wobei das Verfahren derart durchgeführt wird und/oder Merkmal und/oder Maßnahme (i), wahlweise in weiterer Kombination mit Merkmal und/oder Maßnahme (ii) und/oder Merkmal und/oder Maßnahme (iii) und/oder Merkmal und/oder Maßnahme (iv), angewendet und/oder durchgeführt werden, mit der Maßgabe, dass das Reaktionsprodukt, insbesondere das eine oder die mehreren Triglyceride von C₅-C₁₂-Fettsäuren, insbesondere das eine oder die mehreren Triglyceride von C₆-C₁₂-Fettsäuren, der allgemeinen Formel (II)
CH₂[O - C(O)R¹ ] - CH [O - C(O)R² ] - CH₂ [O - C(O)R³ ] (II)
wobei in der allgemeinen Formel (II) die Reste R¹, R² und R³, jeweils unabhängig voneinander, einen linearen oder verzweigten, gesättigten oder ungesättigten aliphatischen C₄-C₁₁-Alkylrest, insbesondere C₅-C₁₁-Alkylrest, vorzugsweise einen linearen, gesättigten aliphatischen C₄-C₁₁-Alkylrest, insbesondere C₅-C₁₁-Alkylrest, darstellen,
durch die folgenden Eigenschaften (1) bis (5) in ihrer Kombination gekennzeichnet ist/sind und/oder diese aufweist/aufweisen und/oder der folgenden Produktspezifikation genügt/genügen:
(1) Säurezahl (AV), insbesondere bestimmt gemäß Ph. Eur. 2.5.1 (Europäisches Arzneibuch 10.0): ≤ 0,5 mg KOH / g, insbesondere ≤ 0,2 mg KOH / g, vorzugsweise ≤ 0,1 mg KOH / g; und
(2) Hydroxylzahl (OHV), insbesondere bestimmt gemäß Ph. Eur. 2.5.3 (Europäisches Arzneibuch 10.0): ≤ 0,5 mg KOH / g, insbesondere ≤ 0,2 mg KOH / g, vorzugsweise ≤ 0,1 mg KOH / g; und
(3) Triglyceridgehalt, insbesondere bestimmt gemäß Ph. Eur. 2.2.28 (Europäisches Arzneibuch 10.0): ≥ 99 %, insbesondere ≥ 99,2 %, vorzugsweise ≥ 99,5 %, jeweils bezogen auf das Reaktionsprodukt; und
(4) Gesamtgehalt an MCPD-Fettsäureestern, insbesondere bestimmt nach der Methode DGF C-VI 18 (10) (Einheitsmethode der Deutschen Gesellschaft für Fettwissenschaft): ≤ 0,5 ppm, insbesondere ≤ 0,2 ppm, vorzugsweise ≤ 0,1 ppm, jeweils bezogen auf das Reaktionsprodukt; und
(5) Gesamtgehalt an Glycidolestern von Fettsäuren, insbesondere bestimmt nach der Methode DGF C-VI 18 (10) (Einheitsmethode der Deutschen Gesellschaft für Fettwissenschaft): ≤ 0,5 ppm, insbesondere ≤ 0,2 ppm, vorzugsweise ≤ 0,1 ppm, jeweils bezogen auf das Reaktionsprodukt.

3. Verfahren zur Herstellung von Triglyceriden von Fettsäuren, insbesondere mit hohem Reinheitsgrad, gemäß Anspruch 1 oder Anspruch 2,
wobei 1,2,3-Propantriol der Formel (I)
CH₂(OH) - CH(OH) - CH₂(OH) (I)
oder dessen geschützte Form oder Vorläufer, vorzugsweise Solketal, als Ausgangsmaterial verwendet und zu den entsprechenden Triglyceriden von Fettsäuren umgesetzt und/oder umgewandelt wird,
um als Reaktionsprodukt ein oder mehrere Triglyceride von C₅-C₁₂-Fettsäuren, insbesondere ein oder mehrere Triglyceride von C₆-C₁₂-Fettsäuren, der allgemeinen Formel (II)
CH₂[O - C(O)R¹] - CH [O - C(O)R²] - CH₂[O - C(O)R³] (II)
zu bilden,
wobei in der allgemeinen Formel (II) die Reste R¹, R² und R³, jeweils unabhängig voneinander, einen linearen oder verzweigten, gesättigten oder ungesättigten aliphatischen C₄-C₁₁-Alkylrest, insbesondere C₅-C₁₁-Alkylrest, vorzugsweise einen linearen, gesättigten aliphatischen C₄-C₁₁-Alkylrest, insbesondere C₅-C₁₁-Alkylrest darstellen,
wobei das Verfahren mindestens einen Veresterungsschritt umfasst, insbesondere einen Veresterungsschritt unter Verwendung von C₅-C₁₂-Fettsäuren, insbesondere C₆-C₁₂-Fettsäuren, und/oder deren Anhydriden und/oder Estern;
wobei das Verfahren durch Merkmal und/oder Maßnahme (i) gekennzeichnet ist, wahlweise in weiterer Kombination mit Merkmal und/oder Maßnahme (ii) und/oder Merkmal und/oder Maßnahme (iii) und/oder Merkmal und/oder Maßnahme (iv):
(i) eine vorzugsweise selektive Derivatisierung von im resultierenden Reaktionsprodukt vorhandenen MCPD-Fettsäureestern und Glycidolestern von Fettsäuren wird durchgeführt und/oder das resultierende Reaktionsprodukt wird mit mindestens einem nukleophilen Behandlungsmittel, ausgewählt aus der Gruppe bestehend aus Hydrogensulfiten (HSO₃⁻), Sulfiten (SO₃²⁻), Hydrogenthiosulfaten (HS₂O₃⁻), Thiosulfaten (S₂O₃²⁻), Hydrogenphosphiten (H₂PO₃⁻), Phosphiten (HPO₃²⁻) und Phosphinen sowie Kombinationen davon, vorzugsweise mit Hydrogensulfit (HSO₃⁻), behandelt und/oder in Kontakt gebracht, insbesondere zur vorzugsweise selektiven Derivatisierung von MCPD-Fettsäureestern und Glycidolestern von Fettsäuren;
(ii) der mindestens eine Veresterungsschritt erfolgt und/oder wird durchgeführt bei Temperaturen von nicht mehr als 180 °C, insbesondere nicht mehr als 150 °C, vorzugsweise nicht mehr als 120 °C, bevorzugt nicht mehr als 100 °C, besonders bevorzugt nicht mehr als 80 °C, noch weiter bevorzugt nicht mehr als 50 °C;
(iii) das als Ausgangsmaterial verwendete 1,2,3-Propantriol der Formel (I) oder dessen geschützte Form, vorzugsweise Solketal, ist frei von jeglichen chlorierten Spezies und weist einen Gesamtgehalt an chlorierten Spezies von höchstens 1 ppm, insbesondere höchstens 0,5 ppm, vorzugsweise höchstens 0,1 ppm, bezogen auf das 1,2,3-Propantriol der Formel (I) oder dessen geschützte Form, vorzugsweise Solketal, auf;
(iv) der mindestens eine Veresterungsschritt erfolgt und/oder wird durchgeführt in Abwesenheit jeglicher metallbasierter Katalysatoren,
wobei das Verfahren derart durchgeführt wird und/oder Merkmal und/oder Maßnahme (i), wahlweise in weiterer Kombination mit Merkmal und/oder Maßnahme (ii) und/oder Merkmal und/oder Maßnahme (iii) und/oder Merkmal und/oder Maßnahme (iv), angewendet und/oder durchgeführt werden, mit der Maßgabe, dass das Reaktionsprodukt, insbesondere das eine oder die mehreren Triglyceride von C₅-C₁₂-Fettsäuren, insbesondere das eine oder die mehreren Triglyceride von C₆-C₁₂-Fettsäuren, der vorgenannten allgemeinen Formel (II) durch die folgenden Eigenschaften (1) bis (5) in ihrer Kombination gekennzeichnet ist/sind und/oder diese aufweist/aufweisen und/oder der folgenden Produktspezifikation genügt/genügen:
(1) Säurezahl (AV), insbesondere bestimmt gemäß Ph. Eur. 2.5.1 (Europäisches Arzneibuch 10.0): ≤ 0,5 mg KOH / g, insbesondere ≤ 0,2 mg KOH / g, vorzugsweise ≤ 0,1 mg KOH / g; und
(2) Hydroxylzahl (OHV), insbesondere bestimmt gemäß Ph. Eur. 2.5.3 (Europäisches Arzneibuch 10.0): ≤ 0,5 mg KOH / g, insbesondere ≤ 0,2 mg KOH / g, vorzugsweise ≤ 0,1 mg KOH / g; und
(3) Triglyceridgehalt, insbesondere bestimmt gemäß Ph. Eur. 2.2.28 (Europäisches Arzneibuch 10.0): ≥ 99 %, insbesondere ≥ 99,2 %, vorzugsweise ≥ 99,5 %, jeweils bezogen auf das Reaktionsprodukt; und
(4) Gesamtgehalt an MCPD-Fettsäureestern, insbesondere bestimmt nach der Methode DGF C-VI 18 (10) (Einheitsmethode der Deutschen Gesellschaft für Fettwissenschaft): ≤ 0,5 ppm, insbesondere ≤ 0,2 ppm, vorzugsweise ≤ 0,1 ppm, jeweils bezogen auf das Reaktionsprodukt; und
(5) Gesamtgehalt an Glycidolestern von Fettsäuren, insbesondere bestimmt nach der Methode DGF C-VI 18 (10) (Einheitsmethode der Deutschen Gesellschaft für Fettwissenschaft): ≤ 0,5 ppm, insbesondere ≤ 0,2 ppm, vorzugsweise ≤ 0,1 ppm, jeweils bezogen auf das Reaktionsprodukt.

4. Verfahren nach einem der vorangehenden Ansprüche,
wobei das Verfahren derart durchgeführt wird und/oder Merkmal und/oder Maßnahme (i), wahlweise in weiterer Kombination mit Merkmal und/oder Maßnahme (ii) und/oder Merkmal und/oder Maßnahme (iii) und/oder Merkmal und/oder Maßnahme (iv), angewendet und/oder durchgeführt werden, mit der Maßgabe, dass das Reaktionsprodukt, insbesondere das eine oder die mehreren Triglyceride von C₅-C₁₂-Fettsäuren, insbesondere das eine oder die mehreren Triglyceride von C₆-C₁₂-Fettsäuren, der vorgenannten allgemeinen Formel (II) durch die folgenden Eigenschaften (1) bis (5) in ihrer Kombination gekennzeichnet ist/sind und/oder diese aufweist/aufweisen und/oder der folgenden Produktspezifikation genügt/genügen:
(1) Säurezahl (AV), insbesondere bestimmt gemäß Ph. Eur. 2.5.1 (Europäisches Arzneibuch 10.0): ≤ 0,1 mg KOH / g; und
(2) Hydroxylzahl (OHV), insbesondere bestimmt gemäß Ph. Eur. 2.5.3 (Europäisches Arzneibuch 10.0): ≤ 0,1 mg KOH / g; und
(3) Triglyceridgehalt, insbesondere bestimmt gemäß Ph. Eur. 2.2.28 (Europäisches Arzneibuch 10.0): ≥ 99,5 %, insbesondere ≥ 99,6 %, vorzugsweise ≥ 99,8 %, jeweils bezogen auf das Reaktionsprodukt; und
(4) Gesamtgehalt an MCPD-Fettsäureestern, insbesondere bestimmt nach der Methode DGF C-VI 18 (10) (Einheitsmethode der Deutschen Gesellschaft für Fettwissenschaft): ≤ 0,1 ppm, bezogen auf das Reaktionsprodukt; und
(5) Gesamtgehalt an Glycidolestern von Fettsäuren, insbesondere bestimmt nach der Methode DGF C-VI 18 (10) (Einheitsmethode der Deutschen Gesellschaft für Fettwissenschaft): ≤ 0,1 ppm, bezogen auf das Reaktionsprodukt.

5. Verfahren nach einem der vorangehenden Ansprüche,
wobei die in dem mindestens einen Veresterungsschritt verwendeten C₅-C₁₂-Fettsäuren, insbesondere C₆-C₁₂-Fettsäuren, und/oder deren Anhydride und/oder Ester durch die allgemeine Formel (III) dargestellt werden
R⁴- C(O)OH (III)
wobei in der allgemeinen Formel (III) der Rest R⁴ einen linearen oder verzweigten, gesättigten oder ungesättigten aliphatischen C₄-C₁₁-Alkylrest, insbesondere C₅-C₁₁-Alkylrest, vorzugsweise einen linearen gesättigten aliphatischen C₄-C₁₁-Alkylrest, insbesondere C₅-C₁₁-Alkylrest, einschließlich deren Anhydride und Ester sowie Kombinationen davon, darstellt; und/oder
wobei die bei der mindestens einen Veresterung verwendeten C₅-C₁₂-Fettsäuren, insbesondere C₆-C₁₂-Fettsäuren, und/oder deren Anhydride und/oder Ester lineare gesättigte C₅-C₁₂-Fettsäuren, insbesondere lineare gesättigte C₆-C₁₂-Fettsäuren, und/oder deren Anhydride und/oder Ester sind; und/oder
wobei die bei dem mindestens einen Veresterungsschritt verwendeten C₅-C₁₂-Fettsäuren, insbesondere C₆-C₁₂-Fettsäuren, und/oder deren Anhydride und/oder Ester ausgewählt sind aus der Gruppe bestehend aus Valeriansäure, Capronsäure, Enanthsäure, Caprylsäure, Pelargonsäure, Caprinsäure, Undecylsäure, Laurinsäure und/oder deren Anhydriden und/oder Estern, insbesondere Capronsäure, Enanthsäure, Caprylsäure, Pelargonsäure, Caprinsäure, Undecylsäure, Laurinsäure und/oder deren Anhydriden und/oder Estern, vorzugsweise Capronsäure, Enanthsäure, Caprylsäure, Caprinsäure, Laurinsäure und/oder deren Anhydriden und/oder Estern, weiter bevorzugt Capronsäure, Enanthsäure, Caprylsäure, Caprinsäure und/oder deren Anhydriden und/oder Estern.

6. Verfahren nach einem der Ansprüche 1 bis 5,
wobei das Reaktionsprodukt ein oder mehrere Triglyceride von C₅-C₁₂-Fettsäuren, insbesondere ein oder mehrere Triglyceride von C₆-C₁₂-Fettsäuren, der oben definierten allgemeinen Formel (II) ist, jedoch mit der Maßgabe, dass alle Reste R¹, R² und R³ identisch sind.

7. Verfahren nach einem der Ansprüche 1 bis 6,
wobei das Reaktionsprodukt Triheptanoin ist; und/oder
wobei in der vorgenannten allgemeinen Formel (II) die Reste R¹, R² und R³ jeweils einen linearen, gesättigten aliphatischen C₆-Alkylrest darstellen; und/oder
wobei in dem mindestens einen Veresterungsschritt Heptansäure und/oder deren Anhydrid und/oder Ester verwendet wird.

8. Verfahren nach einem der Ansprüche 1 bis 6,
wobei das Reaktionsprodukt Tricaprylin ist; und/oder
wobei in der vorgenannten allgemeinen Formel (II) die Reste R¹, R² und R³ jeweils einen linearen, gesättigten aliphatischen C₇-Alkylrest darstellen; und/oder
wobei in dem mindestens einen Veresterungsschritt Octansäure und/oder deren Anhydrid und/oder Ester verwendet wird.

9. Verfahren nach einem der Ansprüche 1 bis 7,
wobei das Reaktionsprodukt nicht Tricaprylin ist.

10. Verfahren nach einem der Ansprüche 1 bis 5,
wobei das Reaktionsprodukt ein oder mehrere Triglyceride von C₅-C₁₂-Fettsäuren, insbesondere ein oder mehrere Triglyceride von C₆-C₁₂-Fettsäuren, der oben definierten allgemeinen Formel (II) ist, jedoch mit der Maßgabe, dass sich mindestens zwei der Reste R¹, R² und R³ voneinander unterscheiden.

11. Verfahren nach einem der Ansprüche 1 bis 5 und 10,
wobei das Reaktionsprodukt ein oder mehrere Triglyceride von C₅-C₁₂-Fettsäuren, insbesondere ein oder mehrere Triglyceride von C₆-C₁₂-Fettsäuren, der oben definierten allgemeinen Formel (II) ist, jedoch mit der Maßgabe, dass sich alle Reste R¹, R² und R³ voneinander unterscheiden.

12. Verfahren nach einem der vorangehenden Ansprüche,
wobei das Verfahren durch eine Kombination des Merkmals und/oder der Maßnahme (i) mit mindestens zwei der Merkmale und/oder Maßnahmen (ii) bis (iv) gekennzeichnet ist; und/oder
wobei das Verfahren durch eine Kombination aller Merkmale und/oder Maßnahmen (i) bis (iv) gekennzeichnet ist.

13. Verfahren nach einem der vorangehenden Ansprüche,
wobei das vollständige und/oder das gesamte Verfahren, insbesondere auch unter Einbeziehung etwaiger optionaler Vorbehandlungs- und/oder Nachbehandlungsschritte, bei Temperaturen von nicht mehr als 180 °C, insbesondere nicht mehr als 160 °C, vorzugsweise nicht mehr als 150 °C, bevorzugt nicht mehr als 120 °C, erfolgt und/oder durchgeführt wird; und/oder
wobei das Verfahren durch ein weiteres Merkmal und/oder eine weitere Maßnahme (v) gekennzeichnet ist:
(v) das vollständige und/oder das gesamte Verfahren, insbesondere auch unter Einbeziehung etwaiger optionaler Vorbehandlungs- und/oder Nachbehandlungsschritte, erfolgt und/oder wird durchgeführt bei Temperaturen von nicht mehr als 180 °C, insbesondere nicht mehr als 160 °C, vorzugsweise nicht mehr als 150 °C, bevorzugt nicht mehr als 120 °C.

14. Verfahren nach einem der vorangehenden Ansprüche,
wobei das vollständige und/oder das gesamte Verfahren, insbesondere auch unter Einbeziehung etwaiger optionaler Vorbehandlungs- und/oder Nachbehandlungsschritte, in Abwesenheit jeglicher metallbasierter Katalysatoren erfolgt und/oder durchgeführt wird; und/oder
wobei das Verfahren durch das weitere Merkmal und/oder die weitere Maßnahme (vi) gekennzeichnet ist:
(vi) das vollständige und/oder das gesamte Verfahren, insbesondere auch unter Einbeziehung etwaiger optionaler Vorbehandlungs- und/oder Nachbehandlungsschritte, erfolgt und/oder wird durchgeführt in Abwesenheit jeglicher metallbasierter Katalysatoren.

15. Verfahren nach einem der vorangehenden Ansprüche,
wobei der mindestens eine Veresterungsschritt als einstufige Veresterung oder alternativ als zweistufige oder mehrstufige Veresterung erfolgt und/oder durchgeführt wird.

16. Verfahren nach einem der vorangehenden Ansprüche,
wobei der mindestens eine Veresterungsschritt als einstufige Veresterung unter Verwendung von Anhydriden von C₅-C₁₂-Fettsäuren zur Reaktion mit 1,2,3-Propantriol der Formel (I) oder dessen geschützten Form oder Vorläufer, vorzugsweise Solketal, erfolgt und/oder durchgeführt wird, oder wobei, alternativ, der mindestens eine Veresterungsschritt als zweistufige oder mehrstufige Veresterung unter Verwendung von C₅-C₁₂-Fettsäuren und/oder deren Estern zur Reaktion mit dem 1,2,3-Propantriol der Formel (I) oder dessen geschützten Form oder Vorläufer, vorzugsweise Solketal, in einer ersten Stufe, gefolgt von einer weiteren Veresterung, insbesondere einer weiteren Veresterung freier und/oder verbleibender OH-Gruppen, in einer nachfolgenden Stufe, vorzugsweise unter Verwendung von Anhydriden von C₅-C₁₂-Fettsäuren, erfolgt und/oder durchgeführt wird.

17. Verfahren nach einem der vorangehenden Ansprüche,
wobei der mindestens eine Veresterungsschritt in Abwesenheit von Lösungsmitteln und/oder ohne jegliches Lösungsmittel erfolgt und/oder durchgeführt wird.

18. Verfahren nach einem der vorangehenden Ansprüche,
wobei der mindestens eine Veresterungsschritt bei Temperaturen im Bereich von 5 °C bis 95 °C, insbesondere im Bereich von 10 °C bis 85 °C, vorzugsweise im Bereich von 15 °C bis 80 °C, weiter bevorzugt im Bereich von 20 °C bis 75 °C, noch weiter bevorzugt im Bereich von 25 °C bis 70 °C, erfolgt und/oder durchgeführt wird; und/oder
wobei der mindestens eine Veresterungsschritt bei einem Druck im Bereich von 0,0001 bar bis 10 bar, insbesondere im Bereich von 0,001 bar bis 5 bar, vorzugsweise im Bereich von 0,01 bar bis 2 bar, weiter bevorzugt im Bereich von 0,05 bar bis 1 bar, noch weiter bevorzugt bei 1 bar, erfolgt und/oder durchgeführt wird.

19. Verfahren nach einem der vorangehenden Ansprüche,
wobei der mindestens eine Veresterungsschritt in Abwesenheit jeglicher Katalysatoren erfolgt und/oder durchgeführt wird, oder wobei, alternativ, der mindestens eine Veresterungsschritt zumindest teilweise in Gegenwart eines Enzyms als Katalysator und/oder zumindest teilweise in Gegenwart eines enzymbasierten Katalysators erfolgt und/oder durchgeführt wird;
insbesondere wobei der Katalysator nach dem mindestens einen Veresterungsschritt recycliert wird.

20. Verfahren nach einem der vorangehenden Ansprüche,
wobei der mindestens eine Veresterungsschritt bei Verwendung von Anhydriden von C₅-C₁₂-Fettsäuren zur Reaktion mit dem 1,2,3-Propantriol der Formel (I) oder dessen geschützten Form oder Vorläufer, vorzugsweise Solketal, in Abwesenheit jeglicher Katalysatoren erfolgt und/oder durchgeführt wird, oder wobei, alternativ, der mindestens eine Veresterungsschritt bei Verwendung von C₅-C₁₂-Fettsäuren und/oder deren Estern zur Reaktion mit dem 1,2,3-Propantriol der Formel (I) oder dessen geschützten Form oder Vorläufer, vorzugsweise Solketal, zumindest teilweise in Gegenwart eines Enzyms als Katalysator und/oder zumindest teilweise in Gegenwart eines enzymbasierten Katalysators erfolgt und/oder durchgeführt wird;
insbesondere wobei der Katalysator nach dem mindestens einen Veresterungsschritt recycliert wird.

21. Verfahren nach einem der vorangehenden Ansprüche,
wobei der mindestens eine Veresterungsschritt zumindest teilweise in Gegenwart eines Enzyms als Katalysator und/oder zumindest teilweise in Gegenwart eines enzymbasierten Katalysators erfolgt und/oder durchgeführt wird;
insbesondere wobei das Enzym aus Synthetasen, Katalasen, Esterasen, Lipasen und Kombinationen davon ausgewählt ist; und/oder
insbesondere wobei das Enzym von Candida antarctica, Mucor miehei, Thermomyces lanuginosus, Candida rugosa, Aspergillus oryzae, Pseudomonas cepacia, Pseudomonas fluorescens, Rhizopus delemar und Pseudomonas sp. sowie Kombinationen davon, vorzugsweise von Candida antarctica, Mucor miehei und Thermomyces lanuginosus, weiter bevorzugt von Candida antarctica, stammt; und/oder
insbesondere wobei das Enzym in immobilisierter Form verwendet wird, insbesondere immobilisiert auf einem Träger, vorzugsweise auf einem polymeren Träger, bevorzugt auf einem polymeren organischen Träger, weiter bevorzugt mit hydrophoben Eigenschaften, noch weiter bevorzugt auf einem Träger auf Basis eines Poly(meth)acrylHarzes; und/oder
insbesondere wobei das Enzym nach dem mindestens einen Veresterungsschritt recycliert wird; und/oder
insbesondere wobei der mindestens eine Veresterungsschritt bei Temperaturen im Bereich von 5 °C bis 95 °C, insbesondere im Bereich von 10 °C bis 85 °C, vorzugsweise im Bereich von 15 °C bis 80 °C, weiter bevorzugt im Bereich von 20 °C bis 75 °C, noch weiter bevorzugt im Bereich von 25 °C bis 70 °C, erfolgt und/oder durchgeführt wird; und/oder
insbesondere wobei das Enzym in Mengen, bezogen auf die Gesamtmenge aller Ausgangsverbindungen, im Bereich von 0,0001 bis 25 Gew.-%, insbesondere im Bereich von 0,001 bis 20 Gew.-%, vorzugsweise im Bereich von 0,01 bis 15 Gew.-%, bevorzugt im Bereich von 0,05 bis 10 Gew.-%, verwendet wird; und/oder
insbesondere wobei das Enzym in Mengen, bezogen auf die Gesamtmenge aller Ausgangsverbindungen, im Bereich von 1 • 10⁻⁶ bis 5 Mol-%, insbesondere im Bereich von 1 • 10⁻⁵ bis 2,5 Mol-%, vorzugsweise im Bereich von 1 • 10⁻⁴ bis 1,5 Mol-%, bevorzugt im Bereich von 1 • 10⁻³ bis 1 Mol-%, verwendet wird; und/oder
insbesondere wobei der mindestens eine Veresterungsschritt bei einem Druck im Bereich von 0,0001 bar bis 10 bar, insbesondere im Bereich von 0,001 bar bis 5 bar, vorzugsweise im Bereich von 0,01 bar bis 2 bar, weiter bevorzugt im Bereich von 0,05 bar bis 1 bar, noch weiter bevorzugt bei 1 bar, erfolgt und/oder durchgeführt wird.

22. Verfahren nach einem der vorangehenden Ansprüche,
wobei das Reaktionsprodukt über einen der folgenden Synthesewege (A) bis (F) hergestellt wird:
(A) wobei, gemäß einem Syntheseweg (A), 1,2,3-Propantriol der Formel (I)
CH₂(OH) - CH(OH) - CH₂(OH) (I)
vorzugsweise in Gegenwart eines Enzyms als Katalysator und/oder in Gegenwart eines enzymbasierten Katalysators, zunächst mit mindestens einer linearen oder verzweigten, gesättigten oder ungesättigten aliphatischen C₅-C₁₂-Fettsäure oder deren Ester, insbesondere mit mindestens einer linearen oder verzweigten, gesättigten oder ungesättigten aliphatischen C₆-C₁₂-Fettsäure oder deren Ester, vorzugsweise mit mindestens einer linearen, gesättigten aliphatischen C₅-C₁₂-Fettsäure oder deren Ester, insbesondere mit mindestens einer linearen, gesättigten aliphatischen C₆-C₁₂-Fettsäure oder deren Ester, umgesetzt wird, vorzugsweise unter Bildung eines Diglycerids, bevorzugt eines 1,3-Diglycerids,
gefolgt von einer anschließenden Reaktion, vorzugsweise in Abwesenheit jeglicher Katalysatoren, mit mindestens einem linearen oder verzweigten, gesättigten oder ungesättigten aliphatischen C₅-C₁₂-Fettsäureanhydrid, insbesondere mit mindestens einem linearen oder verzweigten, gesättigten oder ungesättigten aliphatischen C₆-C₁₂-Fettsäureanhydrid, vorzugsweise mit mindestens einem linearen, gesättigten aliphatischen C₅-C₁₂-Fettsäureanhydrid, insbesondere mit mindestens einem linearen, gesättigten aliphatischen C₆-C₁₂-Fettsäureanhydrid,
um das Reaktionsprodukt der allgemeinen Formel (II) zu bilden, wie zuvor definiert; oder aber
(B) wobei, gemäß einem Syntheseweg (B), 1,2,3-Propantriol der Formel (I)
CH₂(OH) - CH(OH) - CH₂(OH) (I)
vorzugsweise in Gegenwart eines Enzyms als Katalysator und/oder in Gegenwart eines enzymbasierten Katalysators, zunächst mit mindestens einer linearen oder verzweigten, gesättigten oder ungesättigten aliphatischen C₅-C₁₂-Fettsäure oder deren Ester, insbesondere mit mindestens einer linearen oder verzweigten, gesättigten oder ungesättigten aliphatischen C₆-C₁₂-Fettsäure oder deren Ester, vorzugsweise mit mindestens einer linearen, gesättigten aliphatischen C₅-C₁₂-Fettsäure oder deren Ester, insbesondere mit mindestens einer linearen, gesättigten aliphatischen C₆-C₁₂-Fettsäure oder deren Ester, umgesetzt wird, vorzugsweise unter Bildung eines Diglycerids, bevorzugt eines 1,3-Diglycerids, gefolgt von einer anschließenden Reaktion, vorzugsweise in Gegenwart eines Enzyms als Katalysator und/oder in Gegenwart eines enzymbasierten Katalysators, mit mindestens einer linearen oder verzweigten, gesättigten oder ungesättigten aliphatischen C₅-C₁₂-Fettsäure oder deren Ester, insbesondere mit mindestens einer linearen oder verzweigten, gesättigten oder ungesättigten aliphatischen C₆-C₁₂-Fettsäure oder deren Ester, vorzugsweise mit mindestens einer linearen, gesättigten aliphatischen C₅-C₁₂-Fettsäure oder deren Ester, insbesondere mit mindestens einer linearen, gesättigten aliphatischen C₆-C₁₂-Fettsäure oder deren Ester,
um das Reaktionsprodukt der allgemeinen Formel (II) zu erhalten, wie zuvor definiert;
oder aber
(C) wobei, gemäß einem Syntheseweg (C), 1,2,3-Propantriol der Formel (I)
CH₂(OH) - CH(OH) - CH₂(OH) (I)
vorzugsweise in Abwesenheit jeglicher Katalysatoren, zunächst mit mindestens einem linearen oder verzweigten, gesättigten oder ungesättigten aliphatischen C₅-C₁₂-Fettsäureanhydrid, insbesondere mit mindestens einem linearen oder verzweigten, gesättigten oder ungesättigten aliphatischen C₆-C₁₂-Fettsäure-anhydrid, vorzugsweise mit mindestens einem linearen, gesättigten aliphatischen C₅-C₁₂-Fettsäureanhydrid, insbesondere mit mindestens einem linearen, gesättigten aliphatischen C₆-C₁₂-Fettsäureanhydrid, umgesetzt wird,
um das Reaktionsprodukt der allgemeinen Formel (II) zu erhalten, wie zuvor definiert;
oder aber
(D) wobei, gemäß einem Syntheseweg (D), eine geschützte Form oder ein Vorläufer von 1,2,3-Propantriol der vorgenannten Formel (I), vorzugsweise Solketal, vorzugsweise in Abwesenheit jeglicher Katalysatoren, mit mindestens einem linearen oder verzweigten, gesättigten oder ungesättigten aliphatischen C₅-C₁₂-Fettsäureanhydrid, insbesondere mit mindestens einem linearen oder verzweigten, gesättigten oder ungesättigten aliphatischen C₆-C₁₂-Fettsäureanhydrid, vorzugsweise mit mindestens einem linearen gesättigten aliphatischen C₅-C₁₂-Fettsäure-anhydrid, insbesondere mit mindestens einem linearen, gesättigten, aliphatischen C₆-C₁₂-Fettsäureanhydrid, umgesetzt wird,
insbesondere wobei nach Monoveresterung der freien OH-Gruppe der geschützten Form oder des Vorläufers von 1,2,3-Propantriol der vorgenannten Formel (I) eine Entschützungsreaktion, insbesondere eine Ringöffnung und gegebenenfalls eine Entfernung von Aceton, durchgeführt wird, gefolgt von weiterer Veresterung mit dem mindestens einen linearen oder verzweigten, gesättigten oder ungesättigten aliphatischen C₅-C₁₂-Fettsäureanhydrid, insbesondere mit dem mindestens einen linearen oder verzweigten, gesättigten oder ungesättigten aliphatischen C₆-C₁₂-Fettsäureanhydrid, vorzugsweise mit dem mindestens einen linearen gesättigten aliphatischen C₅-C₁₂-Fettsäureanhydrid, insbesondere mit dem mindestens einen linearen, gesättigten aliphatischen C₆-C₁₂-Fettsäureanhydrid,
um das Reaktionsprodukt der allgemeinen Formel (II) zu erhalten, wie zuvor definiert;
oder aber
(E) wobei, gemäß einem Syntheseweg (E), eine geschützte Form oder ein Vorläufer von 1,2,3-Propantriol der vorgenannten Formel (I), vorzugsweise Solketal, vorzugsweise in Gegenwart eines Enzyms als Katalysator und/oder in Gegenwart eines enzymbasierten Katalysators, mit mindestens einer linearen oder verzweigten, gesättigten oder ungesättigten aliphatischen C₅-C₁₂-Fettsäure oder deren Ester, insbesondere mit mindestens einer linearen oder verzweigten, gesättigten oder ungesättigten aliphatischen C₆-C₁₂-Fettsäure oder deren Ester, vorzugsweise mit mindestens einer linearen gesättigten aliphatischen C₅-C₁₂-Fettsäure oder deren Ester, insbesondere mit mindestens einer linearen, gesättigten aliphatischen C₆-C₁₂-Fettsäure oder deren Ester, umgesetzt wird, gefolgt von einer Entschützungsreaktion, insbesondere einer Ringöffnung und Entfernung von Aceton, vorzugsweise unter Bildung eines Monoglycerids, bevorzugt eines terminalen Monoglycerids und/oder eines 1-Monoglycerids,
gefolgt von einer anschließenden Reaktion, vorzugsweise in Abwesenheit jeglicher Katalysatoren, mit mindestens einem linearen oder verzweigten, gesättigten oder ungesättigten aliphatischen C₅-C₁₂-Fettsäureanhydrid, insbesondere mit mindestens einem linearen oder verzweigten, gesättigten oder ungesättigten aliphatischen C₆-C₁₂-Fettsäureanhydrid, vorzugsweise mit mindestens einem linearen, gesättigten aliphatischen C₅-C₁₂-Fettsäureanhydrid, insbesondere mit mindestens einem linearen, gesättigten aliphatischen C₆-C₁₂-Fettsäureanhydrid,
um das Reaktionsprodukt der allgemeinen Formel (II) zu erhalten, wie zuvor definiert;
oder aber
(F) wobei, gemäß einem Syntheseweg (F), 1,2,3-Propantriol der Formel (I)
CH₂(OH) - CH(OH) - CH₂(OH) (I)
gegebenenfalls in Gegenwart eines Enzyms als Katalysator und/oder in Gegenwart eines enzymbasierten Katalysators, mit mindestens einer linearen oder verzweigten, einfach oder mehrfach ungesättigten aliphatischen C₅-C₁₂-Fettsäure und/oder deren Estern und/oder Anhydriden, insbesondere mit mindestens einer linearen oder verzweigten, einfach oder mehrfach ungesättigten aliphatischen C₆-C₁₂-Fettsäure und/oder deren Estern und/oder Anhydriden, vorzugsweise mit mindestens einer linearen, einfach oder mehrfach ungesättigten aliphatischen C₅-C₁₂-Fettsäure und/oder deren Estern und/oder Anhydriden, insbesondere mit mindestens einer linearen, einfach oder mehrfach ungesättigten aliphatischen C₆-C₁₂-Fettsäure und/oder deren Estern und/oder Anhydriden, umgesetzt wird, vorzugsweise unter Bildung von Triglyceriden von einfach oder mehrfach ungesättigten aliphatischen C₅-C₁₂-Fettsäuren,
gefolgt von einer anschließenden Hydrierungsreaktion, vorzugsweise in Gegenwart mindestens eines Hydrierungskatalysators, vorzugsweise unter Hydrierung der Doppelbindungen der einfach oder mehrfach ungesättigten aliphatischen C₅-C₁₂-Fettsäurereste der Triglyceride,
um das Reaktionsprodukt der allgemeinen Formel (II) zu bilden, wie zuvor definiert.

## Revendications

1. Procédé pour la production de triglycérides d'acides gras, en particulier à haut degré de pureté,
où 1,2,3-propanetriol de formule (I)
CH₂(OH) - CH(OH) - CH₂(OH) (I)
ou sa forme protégée ou son précurseur, de préférence le solcétal, est utilisé comme réactif et transformé et/ou converti en les triglycérides d'acides gras correspondants,
pour obtenir comme produit de réaction un ou plusieurs triglycérides d'acides gras en C₅-C₁₂, en particulier un ou plusieurs triglycérides d'acides gras en C₆-C₁₂, de formule générale (II)
CH₂[O - C(O)R¹] - CH [O - C(O)R²] - CH₂[O - C(O)R³] (II)
où, dans la formule générale (II), les restes R¹, R² et R³ représentent, chacun indépendamment les uns des autres, un reste alkyle en C₄-C₁₁ aliphatique linéaire ou ramifié, saturé ou insaturé, en particulier un reste alkyle en C₅-C₁₁, de préférence un reste alkyle en C₄-C₁₁ aliphatique linéaire, saturé, en particulier un reste alkyle en C₅-C₁₁,
où le procédé comprend au moins une étape d'estérification, en particulier une étape d'estérification utilisant des acides gras en C₅-C₁₂, en particulier des acides gras en C₆-C₁₂, et/ou leurs anhydrides et/ou esters;
où le procédé est **caractérisé par** la propriété et/ou la mesure (i), facultativement en combinaison supplémentaire avec la propriété et/ou la mesure (ii) et/ou la propriété et/ou la mesure (iii) et/ou la propriété et/ou la mesure (iv):
(i) une dérivatisation de préférence sélective d'esters d'acides gras de MCPD et d'esters de glycidol d'acides gras présents dans le produit de réaction résultant est effectuée et/ou le produit de réaction résultant est traité et/ou mis en contact avec au moins un agent de traitement nucléophile, choisi dans le groupe constitué par les hydrogénosulfites (HSO₃⁻), les sulfites (SO₃²⁻), les hydrogénothiosulfates (HS₂O₃⁻), les thiosulfates (S₂O₃²⁻), les hydrogénophosphites (H₂PO₃⁻), les phosphites (HPO₃²⁻) et les phosphines ainsi que leurs combinaisons, de préférence avec l'hydrogénosulfite (HSO₃⁻), en particulier pour la dérivatisation de préférence sélective d'esters d'acides gras de MCPD et d'esters de glycidol d'acides gras;
(ii) l'au moins une étape d'estérification a lieu et/ou est effectuée à des températures ne dépassant pas 180 °C, en particulier ne dépassant pas 150 °C, de préférence ne dépassant pas 120 °C, de préférence ne dépassant pas 100 °C, de manière particulièrement préférée ne dépassant pas 80 °C, de manière encore plus préférée ne dépassant pas 50 °C;
(iii) le 1,2,3-propanetriol de formule (I) utilisé comme réactif ou sa forme protégée, de préférence le solcétal, est exempt de toute espèce chlorée et présente une teneur totale en espèce chlorée d'au plus 1 ppm, en particulier d'au plus 0,5 ppm, de préférence d'au plus 0,1 ppm, par rapport au 1,2,3-propanetriol de formule (I) ou à sa forme protégée, de préférence le solcétal;
(iv) l'au moins une étape d'estérification a lieu et/ou est effectuée en l'absence de tout catalyseur à base de métal.

2. Procédé selon la revendication 1,
où le produit de réaction, en particulier le ou les triglycérides d'acides gras en C₅-C₁₂, en particulier le ou les triglycérides d'acides gras en C₆-C₁₂, de formule générale (II)
CH₂[O - C(O)R¹] - CH[O - C(O)R²] - CH₂[O - C(O)R³] (II)
où, dans la formule générale (II), les restes R¹, R² et R³ représentent, chacun indépendamment les uns des autres, un reste alkyle en C₄-C₁₁ aliphatique linéaire ou ramifié, saturé ou insaturé, en particulier un reste alkyle en C₅-C₁₁, de préférence un reste alkyle en C₄-C₁₁ aliphatique linéaire, saturé, en particulier un reste alkyle en C₅-C₁₁, est/sont caractérisé(s) par les propriétés (1) à (5) suivantes dans leur combinaison et/ou présente/présentent celles-ci et/ou satisfait/satisfont à la spécification de produit suivante:
(1) Indice d'acidité (AV), en particulier déterminé selon Ph. Eur. 2.5.1 (Pharmacopée européenne 10.0): ≤ 0,5 mg KOH / g, en particulier ≤ 0,2 mg KOH / g, de préférence ≤ 0,1 mg KOH / g; et
(2) Indice d'hydroxyle (OHV), en particulier déterminé selon Ph. Eur. 2.5.3 (Pharmacopée européenne 10.0): ≤ 0,5 mg KOH / g, en particulier ≤ 0,2 mg KOH / g, de préférence ≤ 0,1 mg KOH / g; et
(3) Teneur en triglycérides, en particulier déterminée selon Ph. Eur. 2.2.28 (Pharmacopée européenne 10.0): ≥ 99 %, en particulier ≥ 99,2 %, de préférence ≥ 99,5 %, à chaque fois par rapport au produit de réaction; et
(4) Teneur totale en esters d'acides gras de MCPD, en particulier déterminée selon la méthode DGF C-VI 18 (10) (Einheitsmethode der Deutschen Gesellschaft für Fettwissenschaft [méthode unitaire de la Société allemande de la science des graisses]): ≤ 0,5 ppm, en particulier ≤ 0,2 ppm, de préférence ≤ 0,1 ppm, à chaque fois par rapport au produit de réaction; et
(5) Teneur totale en esters de glycidol d'acides gras, en particulier déterminée selon la méthode DGF C-VI 18 (10) (Einheitsmethode der Deutschen Gesellschaft für Fettwissenschaft [méthode unitaire de la Société allemande de la science des graisses]): ≤ 0,5 ppm, en particulier ≤ 0,2 ppm, de préférence ≤ 0,1 ppm, à chaque fois par rapport au produit de réaction; et/ou
où le procédé est réalisé de telle manière et/ou la propriété et/ou la mesure (i), facultativement en combinaison supplémentaire avec la propriété et/ou la mesure (ii) et/ou la propriété et/ou la mesure (iii) et/ou la propriété et/ou la mesure (iv), sont appliquées et/ou réalisées, à condition que le produit de réaction, en particulier le ou les triglycérides d'acides gras en C₅-C₁₂, en particulier le ou les triglycérides d'acides gras en C₆-C₁₂, de formule générale (II)
CH₂ [O - C(O)R¹ ] - CH [O - C(O)R² ] - CH₂ [O - C(O)R³ ] (II)
où, dans la formule générale (II), les restes R¹, R² et R³ représentent, chacun indépendamment les uns des autres, un reste alkyle en C₄-C₁₁ aliphatique linéaire ou ramifié, saturé ou insaturé, en particulier un reste alkyle en C₅-C₁₁, de préférence un reste alkyle en C₄-C₁₁ aliphatique linéaire, saturé, en particulier un reste alkyle en C₅-C₁₁, est/sont caractérisé(s) par les propriétés (1) à (5) suivantes dans leur combinaison et/ou présente/présentent celles-ci et/ou satisfait/satisfont à la spécification de produit suivante:
(1) Indice d'acidité (AV), en particulier déterminé selon Ph. Eur. 2.5.1 (Pharmacopée européenne 10.0): ≤ 0,5 mg KOH / g, en particulier ≤ 0,2 mg KOH / g, de préférence ≤ 0,1 mg KOH / g; et
(2) Indice d'hydroxyle (OHV), en particulier déterminé selon Ph. Eur. 2.5.3 (Pharmacopée européenne 10.0): ≤ 0,5 mg KOH / g, en particulier ≤ 0,2 mg KOH / g, de préférence ≤ 0,1 mg KOH / g; et
(3) Teneur en triglycérides, en particulier déterminée selon Ph. Eur. 2.2.28 (Pharmacopée européenne 10.0): ≥ 99 %, en particulier ≥ 99,2 %, de préférence ≥ 99,5 %, à chaque fois par rapport au produit de réaction; et
(4) Teneur totale en esters d'acides gras de MCPD, en particulier déterminée selon la méthode DGF C-VI 18 (10) (Einheitsmethode der Deutschen Gesellschaft für Fettwissenschaft [méthode unitaire de la Société allemande de la science des graisses]): ≤ 0,5 ppm, en particulier ≤ 0,2 ppm, de préférence ≤ 0,1 ppm, à chaque fois par rapport au produit de réaction; et
(5) Teneur totale en esters de glycidol d'acides gras, en particulier déterminée selon la méthode DGF C-VI 18 (10) (Einheitsmethode der Deutschen Gesellschaft für Fettwissenschaft [méthode unitaire de la Société allemande de la science des graisses]): ≤ 0,5 ppm, en particulier ≤ 0,2 ppm, de préférence ≤ 0,1 ppm, à chaque fois par rapport au produit de réaction.

3. Procédé pour la production de triglycérides d'acides gras, en particulier à haut degré de pureté, selon la revendication 1 ou la revendication 2,
où 1,2,3-propanetriol de formule (I)
CH₂(OH) - CH(OH) - CH₂(OH) (I)
ou sa forme protégée ou son précurseur, de préférence le solcétal, est utilisé comme réactif et transformé et/ou converti en les triglycérides d'acides gras correspondants,
pour obtenir comme produit de réaction un ou plusieurs triglycérides d'acides gras en C₅-C₁₂, en particulier un ou plusieurs triglycérides d'acides gras en C₆-C₁₂, de formule générale (II)
CH₂[O - C(O)R¹] - CH [O - C(O)R²] - CH₂[O - C(O)R³] (II)
où, dans la formule générale (II), les restes R¹, R² et R³ représentent, chacun indépendamment les uns des autres, un reste alkyle en C₄-C₁₁ aliphatique linéaire ou ramifié, saturé ou insaturé, en particulier un reste alkyle en C₅-C₁₁, de préférence un reste alkyle en C₄-C₁₁ aliphatique linéaire, saturé, en particulier un reste alkyle en C₅-C₁₁,
où le procédé comprend au moins une étape d'estérification, en particulier une étape d'estérification utilisant des acides gras en C₅-C₁₂, en particulier des acides gras en C₆-C₁₂, et/ou leurs anhydrides et/ou esters;
où le procédé est **caractérisé par** la propriété et/ou la mesure (i), facultativement en combinaison supplémentaire avec la propriété et/ou la mesure (ii) et/ou la propriété et/ou la mesure (iii) et/ou la propriété et/ou la mesure (iv):
(i) une dérivatisation de préférence sélective d'esters d'acides gras de MCPD et d'esters de glycidol d'acides gras présents dans le produit de réaction résultant est effectuée et/ou le produit de réaction résultant est traité et/ou mis en contact avec au moins un agent de traitement nucléophile, choisi dans le groupe constitué par les hydrogénosulfites (HSO₃⁻), les sulfites (SO₃²⁻), les hydrogénothiosulfates (HS₂O₃⁻), les thiosulfates (S₂O₃²⁻), les hydrogénophosphites (H₂PO₃⁻), les phosphites (HPO₃²⁻) et les phosphines ainsi que leurs combinaisons, de préférence avec l'hydrogénosulfite (HSO₃⁻), en particulier pour la dérivatisation de préférence sélective d'esters d'acides gras de MCPD et d'esters de glycidol d'acides gras;
(ii) l'au moins une étape d'estérification a lieu et/ou est effectuée à des températures ne dépassant pas 180 °C, en particulier ne dépassant pas 150 °C, de préférence ne dépassant pas 120 °C, de préférence ne dépassant pas 100 °C, de manière particulièrement préférée ne dépassant pas 80 °C, de manière encore plus préférée ne dépassant pas 50 °C;
(iii) le 1,2,3-propanetriol de formule (I) utilisé comme réactif ou sa forme protégée, de préférence le solcétal, est exempt de toute espèce chlorée et présente une teneur totale en espèce chlorée d'au plus 1 ppm, en particulier d'au plus 0,5 ppm, de préférence d'au plus 0,1 ppm, par rapport au 1,2,3-propanetriol de formule (I) ou à sa forme protégée, de préférence le solcétal;
(iv) l'au moins une étape d'estérification a lieu et/ou est effectuée en l'absence de tout catalyseur à base de métal,
où le procédé est réalisé de telle manière et/ou la propriété et/ou la mesure (i), facultativement en combinaison supplémentaire avec la propriété et/ou la mesure (ii) et/ou la propriété et/ou la mesure (iii) et/ou la propriété et/ou la mesure (iv), sont appliquées et/ou réalisées, à condition que le produit de réaction, en particulier le ou les triglycérides d'acides gras en C₅-C₁₂, en particulier le ou les triglycérides d'acides gras en C₆-C₁₂, de formule générale (II) susmentionnée est/sont caractérisé(s) par les propriétés (1) à (5) suivantes dans leur combinaison et/ou présente/présentent celles-ci et/ou satisfait/satisfont à la spécification de produit suivante:
(1) Indice d'acidité (AV), en particulier déterminé selon Ph. Eur. 2.5.1 (Pharmacopée européenne 10.0): ≤ 0,5 mg KOH / g, en particulier ≤ 0,2 mg KOH / g, de préférence ≤ 0,1 mg KOH / g; et
(2) Indice d'hydroxyle (OHV), en particulier déterminé selon Ph. Eur. 2.5.3 (Pharmacopée européenne 10.0): ≤ 0,5 mg KOH / g, en particulier ≤ 0,2 mg KOH / g, de préférence ≤ 0,1 mg KOH / g; et
(3) Teneur en triglycérides, en particulier déterminée selon Ph. Eur. 2.2.28 (Pharmacopée européenne 10.0): ≥ 99 %, en particulier ≥ 99,2 %, de préférence ≥ 99,5 %, à chaque fois par rapport au produit de réaction; et
(4) Teneur totale en esters d'acides gras de MCPD, en particulier déterminée selon la méthode DGF C-VI 18 (10) (Einheitsmethode der Deutschen Gesellschaft für Fettwissenschaft [méthode unitaire de la Société allemande de la science des graisses]): ≤ 0,5 ppm, en particulier ≤ 0,2 ppm, de préférence ≤ 0,1 ppm, à chaque fois par rapport au produit de réaction; et
(5) Teneur totale en esters de glycidol d'acides gras, en particulier déterminée selon la méthode DGF C-VI 18 (10) (Einheitsmethode der Deutschen Gesellschaft für Fettwissenschaft [méthode unitaire de la Société allemande de la science des graisses]): ≤ 0,5 ppm, en particulier ≤ 0,2 ppm, de préférence ≤ 0,1 ppm, à chaque fois par rapport au produit de réaction.

4. Procédé selon l'une quelconque des revendications précédentes,
où le procédé est réalisé de telle manière et/ou la propriété et/ou la mesure (i), facultativement en combinaison supplémentaire avec la propriété et/ou la mesure (ii) et/ou la propriété et/ou la mesure (iii) et/ou la propriété et/ou la mesure (iv), sont appliquées et/ou réalisées, à condition que le produit de réaction, en particulier le ou les triglycérides d'acides gras en C₅-C₁₂, en particulier le ou les triglycérides d'acides gras en C₆-C₁₂, de formule générale (II) susmentionnée est/sont caractérisé(s) par les propriétés (1) à (5) suivantes dans leur combinaison et/ou présente/présentent celles-ci et/ou satisfait/satisfont à la spécification de produit suivante:
(1) Indice d'acidité (AV), en particulier déterminé selon Ph. Eur. 2.5.1 (Pharmacopée européenne 10.0): ≤ 0,1 mg KOH / g; et
(2) Indice d'hydroxyle (OHV), en particulier déterminé selon Ph. Eur. 2.5.3 (Pharmacopée européenne 10.0): ≤ 0,1 mg KOH / g; et
(3) Teneur en triglycérides, en particulier déterminée selon Ph. Eur. 2.2.28 (Pharmacopée européenne 10.0): ≥ 99,5 %, en particulier ≥ 99,6 %, de préférence ≥ 99,8 %, à chaque fois par rapport au produit de réaction; et
(4) Teneur totale en esters d'acides gras de MCPD, en particulier déterminée selon la méthode DGF C-VI 18 (10) (Einheitsmethode der Deutschen Gesellschaft für Fettwissenschaft [méthode unitaire de la Société allemande de la science des graisses]): ≤ 0,1 ppm, par rapport au produit de réaction; et
(5) Teneur totale en esters de glycidol d'acides gras, en particulier déterminée selon la méthode DGF C-VI 18 (10) (Einheitsmethode der Deutschen Gesellschaft für Fettwissenschaft [méthode unitaire de la Société allemande de la science des graisses]): ≤ 0,1 ppm, par rapport au produit de réaction.

5. Procédé selon l'une quelconque des revendications précédentes,
où les acides gras en C₅-C₁₂, en particulier les acides gras en C₆-C₁₂, et/ou leurs anhydrides et/ou esters utilisés dans l'au moins une étape d'estérification sont représentés par la formule générale (III)
R⁴ - C(O)OH (III)
où, dans la formule générale (III), le reste R⁴ représente un reste alkyle en C₄-C₁₁ aliphatique linéaire ou ramifié, saturé ou insaturé, en particulier un reste alkyle en C₅-C₁₁, de préférence un reste alkyle en C₄-C₁₁ aliphatique linéaire, saturé, en particulier un reste alkyle en C₅-C₁₁, y compris leurs anhydrides et esters, ainsi que des combinaisons de ceux-ci; et/ou
où les acides gras en C₅-C₁₂, en particulier les acides gras en C₆-C₁₂, et/ou leurs anhydrides et/ou esters utilisés dans l'au moins une estérification sont des acides gras en C₅-C₁₂ saturés linéaires, en particulier des acides gras en C₆-C₁₂ saturés linéaires, et/ou leurs anhydrides et/ou esters; et/ou
où les acides gras en C₅-C₁₂, en particulier les acides gras en C₆-C₁₂, et/ou leurs anhydrides et/ou esters utilisés dans l'au moins une étape d'estérification sont choisis dans le groupe constitué par l'acide valérique, l'acide caproïque, l'acide énanthique, l'acide caprylique, l'acide pélargonique, l'acide caprique, l'acide undécylique, l'acide laurique et/ou leurs anhydrides et/ou esters, en particulier l'acide caproïque, l'acide énanthique, l'acide caprylique, l'acide pélargonique, l'acide caprique, l'acide undécylique, l'acide laurique et/ou leurs anhydrides et/ou esters, de préférence l'acide caproïque, l'acide énanthique, l'acide caprylique, l'acide caprique, l'acide laurique et/ou leurs anhydrides et/ou esters, plus préférablement l'acide caproïque, l'acide énanthique, l'acide caprylique, l'acide caprique et/ou leurs anhydrides et/ou esters.

6. Procédé selon l'une quelconque des revendications 1 à 5,
où le produit de réaction est un ou plusieurs triglycérides d'acides gras en C₅-C₁₂, en particulier un ou plusieurs triglycérides d'acides gras en C₆-C₁₂, de formule générale (II) définie ci-dessus, mais à condition que tous les restes R¹, R² et R³ soient identiques.

7. Procédé selon l'une quelconque des revendications 1 à 6,
où le produit de réaction est la triheptanoïne; et/ou
où, dans la formule générale (II) susmentionnée, les restes R¹, R² et R³ représentent chacun un reste alkyle en C₆ aliphatique linéaire, saturé; et/ou
où, dans l'au moins une étape d'estérification, l'acide heptanoïque et/ou son anhydride et/ou ester est utilisé.

8. Procédé selon l'une quelconque des revendications 1 à 6,
où le produit de réaction est la tricapryline; et/ou
où, dans la formule générale (II) susmentionnée, les restes R¹, R² et R³ représentent chacun un reste alkyle en C₇ aliphatique linéaire, saturé; et/ou
où, dans l'au moins une étape d'estérification, l'acide octanoïque et/ou son anhydride et/ou ester est utilisé.

9. Procédé selon l'une quelconque des revendications 1 à 7,
où le produit de réaction n'est pas la tricapryline.

10. Procédé selon l'une quelconque des revendications 1 à 5,
où le produit de réaction est un ou plusieurs triglycérides d'acides gras en C₅-C₁₂, en particulier un ou plusieurs triglycérides d'acides gras en C₆-C₁₂, de formule générale (II) définie ci-dessus, mais à condition qu'au moins deux des restes R¹, R² et R³ soient différents l'un de l'autre.

11. Procédé selon l'une quelconque des revendications 1 à 5 et 10,
où le produit de réaction est un ou plusieurs triglycérides d'acides gras en C₅-C₁₂, en particulier un ou plusieurs triglycérides d'acides gras en C₆-C₁₂, de formule générale (II) définie ci-dessus, mais à condition que tous les restes R¹, R² et R³ soient différents l'un de l'autre.

12. Procédé selon l'une quelconque des revendications précédentes,
où le procédé est **caractérisé par** une combinaison de la propriété et/ou de la mesure (i) avec au moins deux des propriétés et/ou mesures (ii) à (iv); et/ou
où le procédé est **caractérisé par** une combinaison de toutes les propriétés et/ou mesures (i) à (iv).

13. Procédé selon l'une quelconque des revendications précédentes,
où le procédé complet et/ou l'ensemble du procédé, en particulier également en incluant d'éventuelles étapes de prétraitement et/ou de post-traitement facultatives, a lieu et/ou est effectué à des températures ne dépassant pas 180 °C, en particulier ne dépassant pas 160 °C, de préférence ne dépassant pas 150 °C, de préférence ne dépassant pas 120 °C; et/ou
où le procédé est **caractérisé par** une autre propriété et/ou une autre mesure (v):
(v) le procédé complet et/ou l'ensemble du procédé, en particulier également en incluant d'éventuelles étapes de prétraitement et/ou de post-traitement facultatives, a lieu et/ou est effectué à des températures ne dépassant pas 180 °C, en particulier ne dépassant pas 160 °C, de préférence ne dépassant pas 150 °C, de préférence ne dépassant pas 120 °C.

14. Procédé selon l'une quelconque des revendications précédentes,
où le procédé complet et/ou l'ensemble du procédé, en particulier également en incluant d'éventuelles étapes de prétraitement et/ou de post-traitement facultatives, a lieu et/ou est effectué en l'absence de tout catalyseur à base de métal; et/ou
où le procédé est **caractérisé par** l'autre propriété et/ou l'autre mesure (vi):
(vi) le procédé complet et/ou l'ensemble du procédé, en particulier également en incluant d'éventuelles étapes de prétraitement et/ou de post-traitement facultatives, a lieu et/ou est effectué en l'absence de tout catalyseur à base de métal.

15. Procédé selon l'une quelconque des revendications précédentes,
où l'au moins une étape d'estérification a lieu et/ou est effectuée en tant qu'estérification en une étape ou en variante en tant qu'estérification en deux étapes ou en plusieurs étapes.

16. Procédé selon l'une quelconque des revendications précédentes,
où l'au moins une étape d'estérification a lieu et/ou est effectuée en tant qu'estérification en une étape utilisant des anhydrides d'acides gras en C₅-C₁₂ pour réagir avec le 1,2,3-propanetriol de formule (I) ou sa forme protégée ou son précurseur, de préférence le solcétal, ou où, en variante, l'au moins une étape d'estérification a lieu et/ou est effectuée en tant qu'estérification en deux étapes ou en plusieurs étapes utilisant des acides gras en C₅-C₁₂ et/ou leurs esters pour réagir avec le 1,2,3-propanetriol de formule (I) ou sa forme protégée ou son précurseur, de préférence le solcétal, dans une première étape, suivie d'une autre estérification, en particulier une autre estérification de groupes OH libres et/ou restants, dans une étape suivante, de préférence en utilisant des anhydrides d'acides gras en C₅-C₁₂.

17. Procédé selon l'une quelconque des revendications précédentes,
où l'au moins une étape d'estérification a lieu et/ou est effectuée en l'absence de solvants et/ou sans aucun solvant.

18. Procédé selon l'une quelconque des revendications précédentes,
où l'au moins une étape d'estérification a lieu et/ou est effectuée à des températures dans l'intervalle de 5 °C à 95 °C, en particulier dans l'intervalle de 10 °C à 85 °C, de préférence dans l'intervalle de 15 °C à 80 °C, de manière plus préférée dans l'intervalle de 20 °C à 75 °C, de manière encore plus préférée dans l'intervalle de 25 °C à 70 °C; et/ou
où l'au moins une étape d'estérification a lieu et/ou est effectuée à une pression dans l'intervalle de 0,0001 bar à 10 bar, en particulier dans l'intervalle de 0,001 bar à 5 bar, de préférence dans l'intervalle de 0,01 bar à 2 bar, de manière plus préférée dans l'intervalle de 0,05 bar à 1 bar, de manière encore plus préférée à 1 bar.

19. Procédé selon l'une quelconque des revendications précédentes,
où l'au moins une étape d'estérification a lieu et/ou est effectuée en l'absence de tout catalyseur, ou où, en variante, l'au moins une étape d'estérification a lieu et/ou est effectuée au moins partiellement en présence d'une enzyme en tant que catalyseur et/ou au moins partiellement en présence d'un catalyseur à base d'enzyme;
en particulier où le catalyseur est recyclé après l'au moins une étape d'estérification.

20. Procédé selon l'une quelconque des revendications précédentes,
où l'au moins une étape d'estérification a lieu et/ou est effectuée en l'absence de tout catalyseur en utilisant des anhydrides d'acides gras en C₅-C₁₂ pour réagir avec le 1,2,3-propanetriol de formule (I) ou sa forme protégée ou son précurseur, de préférence le solcétal, ou où, en variante, l'au moins une étape d'estérification a lieu et/ou est effectuée au moins partiellement en présence d'une enzyme en tant que catalyseur et/ou au moins partiellement en présence d'un catalyseur à base d'enzyme en utilisant des acides gras en C₅-C₁₂ et/ou leurs esters pour réagir avec le 1,2,3-propanetriol de formule (I) ou sa forme protégée ou son précurseur, de préférence le solcétal;
en particulier où le catalyseur est recyclé après l'au moins une étape d'estérification.

21. Procédé selon l'une quelconque des revendications précédentes,
où l'au moins une étape d'estérification a lieu et/ou est effectuée au moins partiellement en présence d'une enzyme en tant que catalyseur et/ou au moins partiellement en présence d'un catalyseur à base d'enzyme;
en particulier où l'enzyme est choisie parmi les synthétases, les catalases, les estérases, les lipases et leurs combinaisons; et/ou
en particulier où l'enzyme provient de Candida antarctica, Mucor miehei, Thermomyces lanuginosus, Candida rugosa, Aspergillus oryzae, Pseudomonas cepacia, Pseudomonas fluorescens, Rhizopus delemar et Pseudomonas sp. ainsi que leurs combinaisons, de préférence de Candida antarctica, Mucor miehei et Thermomyces lanuginosus, de manière plus préférée de Candida antarctica; et/ou
en particulier où l'enzyme est utilisée sous forme immobilisée, en particulier immobilisée sur un support, de préférence sur un support polymère, de préférence sur un support organique polymère, de manière plus préférée avec des propriétés hydrophobes, de manière encore plus préférée sur un support à base d'une résine poly(méth)acrylique; et/ou
en particulier où l'enzyme est recyclée après l'au moins une étape d'estérification; et/ou en particulier où l'au moins une étape d'estérification a lieu et/ou est effectuée à des températures dans l'intervalle de 5 °C à 95 °C, en particulier dans l'intervalle de 10 °C à 85 °C, de préférence dans l'intervalle de 15 °C à 80 °C, de manière plus préférée dans l'intervalle de 20 °C à 75 °C, de manière encore plus préférée dans l'intervalle de 25 °C à 70 °C; et/ou
en particulier où l'enzyme est utilisée en quantités, par rapport à la quantité totale de tous les composés de départ, dans l'intervalle de 0,0001 à 25 % en poids, en particulier dans l'intervalle de 0,001 à 20 % en poids, de préférence dans l'intervalle de 0,01 à 15 % en poids, de préférence dans l'intervalle de 0,05 à 10 % en poids; et/ou
en particulier où l'enzyme est utilisée en quantités, par rapport à la quantité totale de tous les composés de départ, dans l'intervalle de 1 • 10⁻⁶ à 5 % en moles, en particulier dans l'intervalle de 1 • 10⁻⁵ à 2,5 % en moles, de préférence dans l'intervalle de 1 • 10⁻⁴ à 1,5 % en moles, de préférence dans l'intervalle de 1 • 10⁻³ à 1 % en moles; et/ou
en particulier où l'au moins une étape d'estérification a lieu et/ou est effectuée à une pression dans l'intervalle de 0,0001 bar à 10 bar, en particulier dans l'intervalle de 0,001 bar à 5 bar, de préférence dans l'intervalle de 0,01 bar à 2 bar, de manière plus préférée dans l'intervalle de 0,05 bar à 1 bar, de manière encore plus préférée à 1 bar.

22. Procédé selon l'une quelconque des revendications précédentes,
où le produit de réaction est préparé par l'une des routes de synthèse (A) à (F) suivantes:
(A) où, selon une route de synthèse (A), 1,2,3-propanetriol de formule (I)
CH₂(OH) - CH(OH) - CH₂(OH) (I)
de préférence en présence d'une enzyme en tant que catalyseur et/ou en présence d'un catalyseur à base d'enzyme, est d'abord mis à réagir avec au moins un acide gras en C₅-C₁₂ aliphatique linéaire ou ramifié, saturé ou insaturé ou son ester, en particulier avec au moins un acide gras en C₆-C₁₂ aliphatique linéaire ou ramifié, saturé ou insaturé ou son ester, de préférence avec au moins un acide gras en C₅-C₁₂ aliphatique linéaire, saturé ou son ester, en particulier avec au moins un acide gras en C₆-C₁₂ aliphatique linéaire, saturé ou son ester, de préférence avec formation d'un diglycéride, de préférence d'un 1,3-diglycéride,
suivie d'une réaction subséquente, de préférence en l'absence de tout catalyseur, avec au moins un anhydride d'acide gras en C₅-C₁₂ aliphatique linéaire ou ramifié, saturé ou insaturé, en particulier avec au moins un anhydride d'acide gras en C₆-C₁₂ aliphatique linéaire ou ramifié, saturé ou insaturé, de préférence avec au moins un anhydride d'acide gras en C₅-C₁₂ aliphatique linéaire, saturé, en particulier avec au moins un anhydride d'acide gras en C₆-C₁₂ aliphatique linéaire, saturé,
pour former le produit de réaction de formule générale (II) tel que défini ci-dessus; ou bien
(B) où, selon une route de synthèse (B), 1,2,3-propanetriol de formule (I)
CH₂(OH) - CH(OH) - CH₂(OH) (I)
de préférence en présence d'une enzyme en tant que catalyseur et/ou en présence d'un catalyseur à base d'enzyme, est d'abord mis à réagir avec au moins un acide gras en C₅-C₁₂ aliphatique linéaire ou ramifié, saturé ou insaturé ou son ester, en particulier avec au moins un acide gras en C₆-C₁₂ aliphatique linéaire ou ramifié, saturé ou insaturé ou son ester, de préférence avec au moins un acide gras en C₅-C₁₂ aliphatique linéaire, saturé ou son ester, en particulier avec au moins un acide gras en C₆-C₁₂ aliphatique linéaire, saturé ou son ester, de préférence avec formation d'un diglycéride, de préférence d'un 1,3-diglycéride,
suivie d'une réaction subséquente, de préférence en présence d'une enzyme en tant que catalyseur et/ou en présence d'un catalyseur à base d'enzyme, avec au moins un acide gras en C₅-C₁₂ aliphatique linéaire ou ramifié, saturé ou insaturé ou son ester, en particulier avec au moins un acide gras en C₆-C₁₂ aliphatique linéaire ou ramifié, saturé ou insaturé ou son ester, de préférence avec au moins un acide gras en C₅-C₁₂ aliphatique linéaire, saturé ou son ester, en particulier avec au moins un acide gras en C₆-C₁₂ aliphatique linéaire, saturé ou son ester,
pour obtenir le produit de réaction de formule générale (II) tel que défini ci-dessus; ou bien
(C) où, selon une route de synthèse (C), 1,2,3-propanetriol de formule (I)
CH₂(OH) - CH(OH) - CH₂(OH) (I)
de préférence en l'absence de tout catalyseur, est d'abord mis à réagir avec au moins un anhydride d'acide gras en C₅-C₁₂ aliphatique linéaire ou ramifié, saturé ou insaturé, en particulier avec au moins un anhydride d'acide gras en C₆-C₁₂ aliphatique linéaire ou ramifié, saturé ou insaturé, de préférence avec au moins un anhydride d'acide gras en C₅-C₁₂ aliphatique linéaire, saturé, en particulier avec au moins un anhydride d'acide gras en C₆-C₁₂ aliphatique linéaire, saturé,
pour obtenir le produit de réaction de formule générale (II) tel que défini ci-dessus; ou bien
(D) où, selon une route de synthèse (D), une forme protégée ou un précurseur de 1,2,3-propanetriol de formule (I) susmentionnée, de préférence le solcétal, est mis à réagir, de préférence en l'absence de tout catalyseur, avec au moins un anhydride d'acide gras en C₅-C₁₂ aliphatique linéaire ou ramifié, saturé ou insaturé, en particulier avec au moins un anhydride d'acide gras en C₆-C₁₂ aliphatique linéaire ou ramifié, saturé ou insaturé, de préférence avec au moins un anhydride d'acide gras en C₅-C₁₂ aliphatique linéaire, saturé, en particulier avec au moins un anhydride d'acide gras en C₆-C₁₂ aliphatique linéaire, saturé,
en particulier où, après la mono-estérification du groupe OH libre de la forme protégée ou du précurseur de 1,2,3-propanetriol de formule (I) susmentionnée, une réaction de déprotection, en particulier une ouverture de cycle et éventuellement une élimination de l'acétone, est effectuée, suivie d'une autre estérification avec l'au moins un anhydride d'acide gras en C₅-C₁₂ aliphatique linéaire ou ramifié, saturé ou insaturé, en particulier avec l'au moins un anhydride d'acide gras en C₆-C₁₂ aliphatique linéaire ou ramifié, saturé ou insaturé, de préférence avec l'au moins un anhydride d'acide gras en C₅-C₁₂ aliphatique linéaire, saturé, en particulier avec l'au moins un anhydride d'acide gras en C₆-C₁₂ aliphatique linéaire, saturé,
pour obtenir le produit de réaction de formule générale (II) tel que défini ci-dessus; ou bien
(E) où, selon une route de synthèse (E), une forme protégée ou un précurseur de 1,2,3-propanetriol de formule (I) susmentionnée, de préférence le solcétal, est mis à réagir, de préférence en présence d'une enzyme en tant que catalyseur et/ou en présence d'un catalyseur à base d'enzyme, avec au moins un acide gras en C₅-C₁₂ aliphatique linéaire ou ramifié, saturé ou insaturé ou son ester, en particulier avec au moins un acide gras en C₆-C₁₂ aliphatique linéaire ou ramifié, saturé ou insaturé ou son ester, de préférence avec au moins un acide gras en C₅-C₁₂ aliphatique linéaire, saturé ou son ester, en particulier avec au moins un acide gras en C₆-C₁₂ aliphatique linéaire, saturé ou son ester, suivie d'une réaction de déprotection, en particulier une ouverture de cycle et une élimination de l'acétone, de préférence avec formation d'un monoglycéride, de préférence d'un monoglycéride terminal et/ou d'un 1-monoglycéride,
suivie d'une réaction subséquente, de préférence en l'absence de tout catalyseur, avec au moins un anhydride d'acide gras en C₅-C₁₂ aliphatique linéaire ou ramifié, saturé ou insaturé, en particulier avec au moins un anhydride d'acide gras en C₆-C₁₂ aliphatique linéaire ou ramifié, saturé ou insaturé, de préférence avec au moins un anhydride d'acide gras en C₅-C₁₂ aliphatique linéaire, saturé, en particulier avec au moins un anhydride d'acide gras en C₆-C₁₂ aliphatique linéaire, saturé,
pour obtenir le produit de réaction de formule générale (II) tel que défini ci-dessus; ou bien
(F) où, selon une route de synthèse (F), 1,2,3-propanetriol de formule (I)
CH₂(OH) - CH(OH) - CH₂(OH) (I)
facultativement en présence d'une enzyme en tant que catalyseur et/ou en présence d'un catalyseur à base d'enzyme, est mis à réagir avec au moins un acide gras en C₅-C₁₂ aliphatique linéaire ou ramifié, mono- ou polyinsaturé et/ou son ester et/ou anhydride, en particulier avec au moins un acide gras en C₆-C₁₂ aliphatique linéaire ou ramifié, mono- ou polyinsaturé et/ou son ester et/ou anhydride, de préférence avec au moins un acide gras en C₅-C₁₂ aliphatique linéaire, mono- ou polyinsaturé et/ou son ester et/ou anhydride, en particulier avec au moins un acide gras en C₆-C₁₂ aliphatique linéaire, mono- ou polyinsaturé et/ou son ester et/ou anhydride, de préférence avec formation de triglycérides d'acides gras en C₅-C₁₂ aliphatiques mono- ou polyinsaturés,
suivie d'une réaction d'hydrogénation subséquente, de préférence en présence d'au moins un catalyseur d'hydrogénation, de préférence avec hydrogénation des doubles liaisons des restes d'acides gras en C₅-C₁₂ aliphatiques mono- ou polyinsaturés des triglycérides,
pour former le produit de réaction de formule générale (II) tel que défini ci-dessus.
